(19) **Europäisches Patentamt / European Patent Office / Office européen des brevets**

(11) **EP 4 438 055 A1**

(12) **EUROPEAN PATENT APPLICATION**
published in accordance with Art. 153(4) EPC

(43) Date of publication:
**02.10.2024 Bulletin 2024/40**

(21) Application number: **22897899.5**

(22) Date of filing: **24.11.2022**

(51) International Patent Classification (IPC):
**A61K 39/00** (2006.01)   **C07K 16/28** (2006.01)

(52) Cooperative Patent Classification (CPC):
**A61K 39/00; C07K 16/28**

(86) International application number:
**PCT/CN2022/134161**

(87) International publication number:
**WO 2023/093816 (01.06.2023 Gazette 2023/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.11.2021 CN 202111411306**

(71) Applicant: **Chia Tai Tianqing Pharmaceutical Group Co., Ltd.**
**Lianyungang City, Jiangsu 222062 (CN)**

(72) Inventors:
- **LV, Qiang**
  **Suzhou, Jiangsu 215000 (CN)**
- **JIANG, Xuefeng**
  **Suzhou, Jiangsu 215000 (CN)**
- **SHAO, Na**
  **Suzhou, Jiangsu 215000 (CN)**
- **XU, Hui**
  **Suzhou, Jiangsu 215000 (CN)**
- **DENG, Changjing**
  **Suzhou, Jiangsu 215000 (CN)**
- **GUAN, Guangkuo**
  **Suzhou, Jiangsu 215000 (CN)**
- **ZHOU, Liang**
  **Suzhou, Jiangsu 215000 (CN)**
- **ZHANG, Zhengping**
  **Lianyungang, Jiangsu 222062 (CN)**
- **LI, Tiantian**
  **Lianyungang, Jiangsu 222062 (CN)**
- **ZHOU, Yunyan**
  **Lianyungang, Jiangsu 222062 (CN)**

(74) Representative: **Grünecker Patent- und Rechtsanwälte**
**PartG mbB**
**Leopoldstraße 4**
**80802 München (DE)**

Remarks:
The complete document including Reference Table(s) and the Sequence Listing(s) can be downloaded from the EPO website

(54) **ANTI-SIGLEC-15 ANTIBODY AND USE THEREOF**

(57)     Provided are an anti-Siglec-15 antibody and a use thereof. Specifically, provided are an isolated anti-Siglec-15 antibody or antigen-binding fragment thereof, an expression vector and host cell for expressing the antibody or antigen-binding fragment thereof, and a use of the antibody or antigen-binding fragment. Further provided are a pharmaceutical composition containing the antibody or antigen-binding fragment thereof, and a use of the pharmaceutical composition.

FIG. 6

EP 4 438 055 A1

## Description

### TECHNICAL FIELD

The present disclosure belongs to the field of biomedicine and specifically relates to an anti-Siglec-15 antibody and use thereof. More specifically, the present disclosure relates to a human Siglec-15 monoclonal antibody and use thereof.

### BACKGROUND

Immunotherapy has become the fourth major cancer treatment modality following such conventional treatments as surgery, radiotherapy, and chemotherapy. Immunotherapy is considered to be the most promising treatment modality due to its low side effects, high antitumor activity, and broad indications. Monoclonal antibodies (mAbs) against the inhibitory receptor cytotoxic T lymphocyte-associated antigen 4 (CTLA-4) and programmed cell death 1 (PD-1) show clinical efficacy and sustained treatment response in over 15 human malignancies. Anti-PD-1/PD-L1 treatment is currently the most famous and clinically effective cancer immunotherapy, but it is only effective against 20%-30% of human solid tumors, with most cancer patients showing primary or acquired resistance. In addition, a large number of patients receiving ICB (immune checkpoint blockade) therapy develop treatment-related toxicity called "immune-related adverse event" (irAE), which sometimes even leads to death. Therefore, it is highly desirable to develop new immune checkpoint inhibitors that exhibit high antitumor efficacy in a variety of malignancies and are safe in targeting.

Siglec-15 is a member of the Siglec family and is first reported due to its role in osteoclast differentiation and bone remodeling (Hiruma Y et. al. 2011, Biochemical and Biophysical Research Communications, 409(3): 424-429). Meanwhile, the detailed function and mechanism of Siglec-15 in tumor regulation are revealed by researchers, and they find that Siglec-15 may be an effective immunosuppressive molecule that will contribute to normalized cancer therapy in the future. Siglec-15 mRNA is rarely expressed in most normal human tissues and various immune cell subsets. Siglec-15 is expressed predominantly on cancer cells, macrophages, and bone marrow cells. Siglec-15 is widely expressed in a variety of tumors including colon cancer, thyroid cancer, endometrioid cancer, kidney cancer, bladder cancer, lung cancer, and liver cancer. Mouse experiments demonstrated that Siglec-15 on macrophages can inhibit T cell responses *in vivo,* which does not affect T cell apoptosis, but rather inhibit T cell specific responses by regulating cell growth. The knockout of the Siglec-15 gene does not cause autoimmune diseases or other symptoms in mice. After the tumor cells were inoculated into the Siglec-15 gene knocked-out mice, the mice were found to exhibit slow tumor growth, prolonged survival, and correspondingly increased infiltration of CD8$^+$ T cells and NK cells. The anti-Siglec-15 monoclonal antibody α-S15 was used in various mouse tumor models, and the α-S15 was found to successfully block the immunosuppressive effect caused by Siglec-15, so that the *in vivo* antitumor immune response was improved. In addition, expression of Siglec-15 and that of PD-L1 do not overlap in tumors, suggesting that using Siglec-15 as a therapeutic target may be a new treatment option for patients who do not respond to the PD-1/PD-L1 therapy. In conclusion, Siglec15 is a safe and effective target with great potential.

Currently, several clinical trials led by NextCure corporation are underway to test the efficacy of NC318 (an anti-Siglec-15 monoclonal antibody) in solid tumors (such as non-small cell lung cancer (NSCLC), ovarian cancer, head and neck squamous cell carcinoma (HNSCC), and triple negative breast cancer (TNBC)).

Considering the extremely limited anti-Siglec-15 monoclonal antibodies at present, it is of great significance to develop anti-Siglec-15 antibodies targeting human Siglec-15 with higher safety and better therapeutic effect.

1. WO 2018/057735A1;
2. Wang, J., Sun, J., Liu, L.N. et al. Siglec-15 as an immune suppressor and potential target for normalization cancer immunotherapy. Nat Med 25, 656-666 (2019).

### SUMMARY

The present disclosure provides an anti-Siglec-15 antibody with high affinity, high specificity, and high bioactivity suitable for use in treating Siglec-15-associated diseases.

The present disclosure provides an isolated antibody, e.g., a mouse, chimeric, humanized, or fully human monoclonal antibody or an antigen-binding fragment thereof that binds to Siglec-15 (e.g., human Siglec-15 and monkey Siglec-15).

The antibody or the antigen-binding fragment thereof disclosed herein has a variety of uses, including detecting Siglec-15 protein and treating and preventing Siglec-15-associated diseases and disorders, including cancers, infections, autoimmune diseases, abnormal bone metabolism, or a combination thereof.

In one aspect, the present disclosure provides an isolated antibody or an antigen-binding fragment thereof, which comprises heavy chain CDRs comprising a heavy chain CDR1, a heavy chain CDR2, and a heavy chain CDR3, wherein: (1) the heavy chain CDR1 comprises the amino acid sequence set forth in SEQ ID NO: 5 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 5, the heavy chain CDR2 comprises the amino acid sequence set forth in SEQ ID NO: 7 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 7, and the heavy chain CDR3 comprises the amino acid sequence set forth in SEQ ID NO: 9 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 9; (2) the heavy chain CDR1 comprises the amino acid sequence set forth in SEQ ID NO: 23 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 23, the heavy chain CDR2 comprises the amino acid sequence set forth in SEQ ID NO: 25 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 25, and the heavy chain CDR3 comprises the amino acid sequence set forth in SEQ ID NO: 27 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 27; (3) the heavy chain CDR1 comprises the amino acid sequence set forth in SEQ ID NO: 41 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 41, the heavy chain CDR2 comprises the amino acid sequence set forth in SEQ ID NO: 43 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 43, and the heavy chain CDR3 comprises the amino acid sequence set forth in SEQ ID NO: 45 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 45; (4) the heavy chain CDR1 comprises the amino acid sequence set forth in SEQ ID NO: 59 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 59, the heavy chain CDR2 comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 61, and the heavy chain CDR3 comprises the amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 63; (5) the heavy chain CDR1 comprises the amino acid sequence set forth in SEQ ID NO: 77 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 77, the heavy chain CDR2 comprises the amino acid sequence set forth in SEQ ID NO: 79 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 79, and the heavy chain CDR3 comprises the amino acid sequence set forth in SEQ ID NO: 81 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 81; (6) the heavy chain CDR1 comprises the amino acid sequence set forth in SEQ ID NO: 95 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 95, the heavy chain CDR2 comprises the amino acid sequence set forth in SEQ ID NO: 97 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 97, and the heavy chain CDR3 comprises the amino acid sequence set forth in SEQ ID NO: 99 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 99; (7) the heavy chain CDR1 comprises the amino acid sequence set forth in SEQ ID NO: 131 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 131, the heavy chain CDR2 comprises the amino acid sequence set forth in SEQ ID NO: 133 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 133, and the heavy chain CDR3 comprises the amino acid sequence set forth in SEQ ID NO: 135 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 135; (8) the heavy chain CDR1 comprises the amino acid sequence set forth in SEQ ID NO: 95 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 95, the heavy chain CDR2 comprises the amino acid sequence set forth in SEQ ID NO: 112 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 112, and the heavy chain CDR3 comprises the amino acid sequence set forth in SEQ ID NO: 99 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 99; (9) the heavy chain CDR1 comprises the amino acid sequence set forth in SEQ ID NO: 95 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 95, the heavy chain CDR2 comprises the amino acid sequence set forth in SEQ ID NO: 115 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 115, and the heavy chain CDR3 comprises the amino acid sequence set forth in SEQ ID NO: 99 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 99; (10) the heavy chain CDR1 comprises the amino acid sequence set forth in SEQ ID NO: 95 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 95, the heavy chain CDR2 comprises the amino acid sequence set forth in SEQ ID NO: 118 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 118, and the heavy chain CDR3 comprises the amino acid sequence set forth in SEQ ID NO: 99 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 99; or (11) the heavy chain CDR1 comprises the amino acid sequence set forth in SEQ ID NO: 131 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 131, the heavy chain CDR2 comprises the amino acid sequence set forth in SEQ ID NO: 133 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 133, and the heavy chain CDR3 comprises the amino acid sequence set forth in SEQ ID NO: 151 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 151. In some specific embodiments, the isolated antibody or the antigen-binding fragment thereof binds to Siglec-15.

In some embodiments, the isolated antibody or the antigen-binding fragment thereof comprises light chain CDRs comprising a light chain CDR1, a light chain CDR2, and a light chain CDR3, wherein: (1) the light chain CDR1 comprises the amino acid sequence set forth in SEQ ID NO: 14 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 14, the light chain CDR2 comprises the amino acid sequence set forth in SEQ ID NO: 16 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 16, and the light chain CDR3 comprises the amino acid sequence set forth in SEQ ID NO: 18 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 18; (2) the light chain CDR1 comprises the amino acid sequence set forth in SEQ ID NO: 32 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 32, the light chain CDR2 comprises the amino acid sequence set forth in SEQ ID NO: 34 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 34, and the light chain CDR3 comprises the amino acid sequence set forth in SEQ ID NO: 36 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 36; (3) the light chain CDR1 comprises the amino acid sequence set forth in SEQ ID NO: 50 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 50, the light chain CDR2 comprises the amino acid sequence set forth in SEQ ID NO: 52 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 52, and the light chain CDR3 comprises the amino acid sequence set forth in SEQ ID NO: 54 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 54; (4) the light chain CDR1 comprises the amino acid sequence set forth in SEQ ID NO: 68 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 68, the light chain CDR2 comprises the amino acid sequence set forth in SEQ ID NO: 70 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 70, and the light chain CDR3 comprises the amino acid sequence set forth in SEQ ID NO: 72 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 72; (5) the light chain CDR1 comprises the amino acid sequence set forth in SEQ ID NO: 86 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 86, the light chain CDR2 comprises the amino acid sequence set forth in SEQ ID NO: 88 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 88, and the light chain CDR3 comprises the amino acid sequence set forth in SEQ ID NO: 90 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 90; (6) the light chain CDR1 comprises the amino acid sequence set forth in SEQ ID NO: 104 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 104, the light chain CDR2 comprises the amino acid sequence set forth in SEQ ID NO: 106 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 106, and the light chain CDR3 comprises the amino acid sequence set forth in SEQ ID NO: 108 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 108; (7) the light chain CDR1 comprises the amino acid sequence set forth in SEQ ID NO: 140 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 140, the light chain CDR2 comprises the amino acid sequence set forth in SEQ ID NO: 142 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 142, and the light chain CDR3 comprises the amino acid sequence set forth in SEQ ID NO: 144 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 144; (8) the light chain CDR1 comprises the amino acid sequence set forth in SEQ ID NO: 121 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 121, the light chain CDR2 comprises the amino acid sequence set forth in SEQ ID NO: 106 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 106, and the light chain CDR3 comprises the amino acid sequence set forth in SEQ ID NO: 108 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 108; (9) the light chain CDR1 comprises the amino acid sequence set forth in SEQ ID NO: 124 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 124, the light chain CDR2 comprises the amino acid sequence set forth in SEQ ID NO: 106 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 106, and the light chain CDR3 comprises the amino acid sequence set forth in SEQ ID NO: 108 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 108; or (10) the light chain CDR1 comprises the amino acid sequence set forth in SEQ ID NO: 127 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 127, the light chain CDR2 comprises the amino acid sequence set forth in SEQ ID NO: 106 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 106, and the light chain CDR3 comprises the amino acid sequence set forth in SEQ ID NO: 108 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 108. In some specific embodiments, the isolated antibody or the antigen-binding fragment thereof binds to Siglec-15.

In some embodiments, the isolated antibody or the antigen-binding fragment thereof comprises a heavy chain CDR1, a heavy chain CDR2, a heavy chain CDR3, a light chain CDR1, a light chain CDR2, and a light chain CDR3, wherein: (1) the heavy chain CDR1 comprises the amino acid sequence set forth in SEQ ID NO: 5, 23, 41, 59, 77, 95 or 131 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 5, 23, 41, 59, 77, 95 or 131; (2) the heavy chain CDR2 comprises the amino acid sequence set forth in SEQ ID NO: 7, 25, 43, 61, 79, 97, 133, 112, 115 or 118 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 7, 25, 43, 61, 79, 97, 133, 112, 115 or 118; (3) the heavy chain CDR3 comprises the amino acid sequence set forth in SEQ ID NO: 9, 27, 45, 63, 81, 99, 135 or 151 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 9, 27, 45, 63, 81, 99, 135, or 151; (4) the light chain CDR1 comprises the amino acid sequence set forth in SEQ ID NO: 14, 32, 50, 68, 86, 104, 140, 121, 124 or 127 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 14, 32, 50, 68, 86, 104, 140, 121, 124 or 127; (5) the light chain CDR2 comprises the amino acid sequence set forth in SEQ ID NO: 16, 34, 52, 70, 88, 106 or 142 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to SEQ ID NO: 16, 34, 52, 70, 88, 106 or 142; and (6) the light chain CDR3 comprises the amino acid sequence set forth in SEQ ID NO: 18, 36, 54, 72, 90, 108 or 144 or an amino acid sequence having at least 80%, 81%, 82%, 83%, 84%, 85%, 86%, 87%, 88%, 89%, 90%, 91%, 92%, 93%, 94%, 95%, 96%, 97%, 98% or 99% identity to SEQ ID NO: 18, 36, 54, 72, 90, 108 or 144. In some specific embodiments, the isolated antibody or the antigen-binding fragment thereof binds to Siglec-15.

In some embodiments, the isolated antibody or the antigen-binding fragment thereof comprises a heavy chain CDR1, a heavy chain CDR2, a heavy chain CDR3, a light chain CDR1, a light chain CDR2, and a light chain CDR3, wherein: (1) the heavy chain CDR1 comprises the amino acid sequence set forth in SEQ ID NO: 5 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 5, the heavy chain CDR2 comprises the amino acid sequence set forth in SEQ ID NO: 7 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 7, the heavy chain CDR3 comprises the amino acid sequence set forth in SEQ ID NO: 9 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 9, the light chain CDR1 comprises the amino acid sequence set forth in SEQ ID NO: 14 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 14, the light chain CDR2 comprises the amino acid sequence set forth in SEQ ID NO: 16 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 16, and the light chain CDR3 comprises the amino acid sequence set forth in SEQ ID NO: 18 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 18; (2) the heavy chain CDR1 comprises the amino acid sequence set forth in SEQ ID NO: 23 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 23, the heavy chain CDR2 comprises the amino acid sequence set forth in SEQ ID NO: 25 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 25, the heavy chain CDR3 comprises the amino acid sequence set forth in SEQ ID NO: 27 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 27, the light chain CDR1 comprises the amino acid sequence set forth in SEQ ID NO: 32 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 32, the light chain CDR2 comprises the amino acid sequence set forth in SEQ ID NO: 34 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 34, and the light chain CDR3 comprises the amino acid sequence set forth in SEQ ID NO: 36 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 36; (3) the heavy chain CDR1 comprises the amino acid sequence set forth in SEQ ID NO: 41 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 41, the heavy chain CDR2 comprises the amino acid sequence set forth in SEQ ID NO: 43 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 43, the heavy chain CDR3 comprises the amino acid sequence set forth in SEQ ID NO: 45 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 45, the light chain CDR1 comprises the amino acid sequence set forth in SEQ ID NO: 50 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 50, the light chain CDR2 comprises the amino acid sequence set forth in SEQ ID NO: 52 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 52, and the light chain CDR3 comprises the amino acid sequence set forth in SEQ ID NO: 54 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 54; (4) the heavy chain CDR1 comprises the amino acid sequence set forth in SEQ ID NO: 59 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 59, the heavy chain CDR2 comprises the amino acid sequence set forth in SEQ ID NO: 61 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 61, the heavy chain CDR3 comprises the amino acid sequence set forth in SEQ ID NO: 63 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 63, the light chain CDR1 comprises the amino acid sequence set forth in SEQ ID NO: 68 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 68, the light chain CDR2 comprises the amino acid sequence set forth in SEQ ID NO: 70 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 70, and the light chain CDR3 comprises the amino acid sequence set forth in SEQ ID NO: 72 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 72; (5) the heavy chain CDR1 comprises the amino acid sequence set forth in SEQ ID NO: 77 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 77, the heavy chain CDR2 comprises the amino acid sequence set forth in SEQ ID NO: 79 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 79, the heavy chain CDR3 comprises the amino acid sequence set forth in SEQ ID NO: 81 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 81, the light chain CDR1 comprises the amino acid sequence set forth in SEQ ID NO: 86 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 86, the light chain CDR2 comprises the amino acid sequence set forth in SEQ ID NO: 88 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 88, and the light chain CDR3 comprises the amino acid sequence set forth in SEQ ID NO: 90 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 90; (6) the heavy chain CDR1 comprises the amino acid sequence set forth in SEQ ID NO: 95 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 95, the heavy chain CDR2 comprises the amino acid sequence set forth in SEQ ID NO: 97 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 97, the heavy chain CDR3 comprises the amino acid sequence set forth in SEQ ID NO: 99 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 99, the light chain CDR1 comprises the amino acid sequence set forth in SEQ ID NO: 104 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 104, the light chain CDR2 comprises the amino acid sequence set forth in SEQ ID NO: 106 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 106, and the light chain CDR3 comprises the amino acid sequence set forth in SEQ ID NO: 108 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 108; or (7) the heavy chain CDR1 comprises the amino acid sequence set forth in SEQ ID NO: 131 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 131, the heavy chain CDR2 comprises the amino acid sequence set forth in SEQ ID NO: 133 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 133, the heavy chain CDR3 comprises the amino acid sequence set forth in SEQ ID NO: 135 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 135, the light chain CDR1 comprises the amino acid sequence set forth in SEQ ID NO: 140 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 140, the light chain CDR2 comprises the amino acid sequence set forth in SEQ ID NO: 142 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 142, and the light chain CDR3 comprises the amino acid sequence set forth in SEQ ID NO: 144 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 144. In some specific embodiments, the isolated antibody or the antigen-binding fragment thereof binds to Siglec-15.

In some embodiments, the isolated antibody or the antigen-binding fragment thereof comprises a heavy chain CDR1, a heavy chain CDR2, a heavy chain CDR3, a light chain CDR1, a light chain CDR2, and a light chain CDR3, wherein: (1) the heavy chain CDR1 comprises the amino acid sequence set forth in SEQ ID NO: 95 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 95, the heavy chain CDR2 comprises the amino acid sequence set forth in SEQ ID NO: 112 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 112, the heavy chain CDR3 comprises the amino acid sequence set forth in SEQ ID NO: 99 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 99, the light chain CDR1 comprises the amino acid sequence set forth in SEQ ID NO: 104 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 104, the light chain CDR2 comprises the amino acid sequence set forth in SEQ ID NO: 106 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 106, and the light chain CDR3 comprises the amino acid sequence set forth in SEQ ID NO: 108 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 108; (2) the heavy chain CDR1 comprises the amino acid sequence set forth in SEQ ID NO: 95 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 95, the heavy chain CDR2 comprises the amino acid sequence set forth in SEQ ID NO: 115 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 115, the heavy chain CDR3 comprises the amino acid sequence set forth in SEQ ID NO: 99 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 99, the light chain CDR1 comprises the amino acid sequence set forth in SEQ ID NO: 104 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 104, the light chain CDR2 comprises the amino acid sequence set forth in SEQ ID NO: 106 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 106, and the light chain CDR3 comprises the amino acid sequence set forth in SEQ ID NO: 108 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 108; (3) the heavy chain CDR1 comprises the amino acid sequence set forth in SEQ ID NO: 95 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 95, the heavy chain CDR2 comprises the amino acid sequence set forth in SEQ ID NO: 118 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 118, the heavy chain CDR3 comprises the amino acid sequence set forth in SEQ ID NO: 99 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 99, the light chain CDR1 comprises the amino acid sequence set forth in SEQ ID NO: 121 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 121, the light chain CDR2 comprises the amino acid sequence set forth in SEQ ID NO: 106 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 106, and the light chain CDR3 comprises the amino acid sequence set forth in SEQ ID NO: 108 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 108; (4) the heavy chain CDR1 comprises the amino acid sequence set forth in SEQ ID NO: 95 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 95, the heavy chain CDR2 comprises the amino acid sequence set forth in SEQ ID NO: 118 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 118, the heavy chain CDR3 comprises the amino acid sequence set forth in SEQ ID NO: 99 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 99, the light chain CDR1 comprises the amino acid sequence set forth in SEQ ID NO: 124 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 124, the light chain CDR2 comprises the amino acid sequence set forth in SEQ ID NO: 106 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 106, and the light chain CDR3 comprises the amino acid sequence set forth in SEQ ID NO: 108 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 108; (5) the heavy chain CDR1 comprises the amino acid sequence set forth in SEQ ID NO: 95 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 95, the heavy chain CDR2 comprises the amino acid sequence set forth in SEQ ID NO: 118 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 118, the heavy chain CDR3 comprises the amino acid sequence set forth in SEQ ID NO: 99 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 99, the light chain CDR1 comprises the amino acid sequence set forth in SEQ ID NO: 127 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 127, the light chain CDR2 comprises the amino acid sequence set forth in SEQ ID NO: 106 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 106, and the light chain CDR3 comprises the amino acid sequence set forth in SEQ ID NO: 108 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 108; (6) the heavy chain CDR1 comprises the amino acid sequence set forth in SEQ ID NO: 131 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 131, the heavy chain CDR2 comprises the amino acid sequence set forth in SEQ ID NO: 133 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 133, the heavy chain CDR3 comprises the amino acid sequence set forth in SEQ ID NO: 135 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 135, the light chain CDR1 comprises the amino acid sequence set forth in SEQ ID NO: 140 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 140, the light chain CDR2 comprises the amino acid sequence set forth in SEQ ID NO: 142 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 142, and the light chain CDR3 comprises the amino acid sequence set forth in SEQ ID NO: 144 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 144; or (7) the heavy chain CDR1 comprises the amino acid sequence set forth in SEQ ID NO: 131 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 131, the heavy chain CDR2 comprises the amino acid sequence set forth in SEQ ID NO: 133 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 133, the heavy chain CDR3 comprises the amino acid sequence set forth in SEQ ID NO: 151 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 151, the light chain CDR1 comprises the amino acid sequence set forth in SEQ ID NO: 140 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 140, the light chain CDR2 comprises the amino acid sequence set forth in SEQ ID NO: 142 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 142, and the light chain CDR3 comprises the amino acid sequence set forth in SEQ ID NO: 144 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 144. In some specific embodiments, the isolated antibody or the antigen-binding fragment thereof binds to Siglec-15.

In some embodiments, the isolated antibody or the antigen-binding fragment thereof comprises a heavy chain variable region comprising the amino acid sequence set forth in SEQ ID NO: 3, 21, 39, 57, 75, 93, 111, 114, 117, 129, 147 or 149 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 3, 21, 39, 57, 75, 93, 111, 114, 117, 129, 147 or 149. In some specific embodiments, the isolated antibody or the antigen-binding fragment thereof binds to Siglec-15.

In some embodiments, the isolated antibody or the antigen-binding fragment thereof comprises a light chain variable region comprising the amino acid sequence set forth in SEQ ID NO: 12, 30, 48, 66, 84, 102, 120, 123, 126 or 138 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 12, 30, 48, 66, 84, 102, 120, 123, 126 or 138. In some specific embodiments, the isolated antibody or the antigen-binding fragment thereof binds to Siglec-15.

In some embodiments, the isolated antibody or the antigen-binding fragment thereof comprises a heavy chain variable region and a light chain variable region, wherein: (1) the heavy chain variable region comprises the amino acid sequence set forth in SEQ ID NO: 3 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 3, and the light chain variable region comprises the amino acid sequence set forth in SEQ ID NO: 12 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 12; (2) the heavy chain variable region comprises the amino acid sequence set forth in SEQ ID NO: 21 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 21, and the light chain variable region comprises the amino acid sequence set forth in SEQ ID NO: 30 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 30; (3) the heavy chain variable region comprises the amino acid sequence set forth in SEQ ID NO: 39 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 39, and the light chain variable region comprises the amino acid sequence set forth in SEQ ID NO: 48 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 48; (4) the heavy chain variable region comprises the amino acid sequence set forth in SEQ ID NO: 57 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 57, and the light chain variable region comprises the amino acid sequence set forth in SEQ ID NO: 66 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 66; (5) the heavy chain variable region comprises the amino acid sequence set forth in SEQ ID NO: 75 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 75, and the light chain variable region comprises the amino acid sequence set forth in SEQ ID NO: 84 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 84; (6) the heavy chain variable region comprises the amino acid sequence set forth in SEQ ID NO: 93 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 93, and the light chain variable region comprises the amino acid sequence set forth in SEQ ID NO: 102 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 102; (7) the heavy chain variable region comprises the amino acid sequence set forth in SEQ ID NO: 129 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 129, and the light chain variable region comprises the amino acid sequence set forth in SEQ ID NO: 138 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 138; (8) the heavy chain variable region comprises the amino acid sequence set forth in SEQ ID NO: 111 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 111, and the light chain variable region comprises the amino acid sequence set forth in SEQ ID NO: 102 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 102; (9) the heavy chain variable region comprises the amino acid sequence set forth in SEQ ID NO: 114 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 114, and the light chain variable region comprises the amino acid sequence set forth in SEQ ID NO: 102 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 102; (10) the heavy chain variable region comprises the amino acid sequence set forth in SEQ ID NO: 117 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 117, and the light chain variable region comprises the amino acid sequence set forth in SEQ ID NO: 120 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 120; (11) the heavy chain variable region comprises the amino acid sequence set forth in SEQ ID NO: 117 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 117, and the light chain variable region comprises the amino acid sequence set forth in SEQ ID NO: 123 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 123; (12) the heavy chain variable region comprises the amino acid sequence set forth in SEQ ID NO: 117 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 117, and the light chain variable region comprises the amino acid sequence set forth in SEQ ID NO: 126 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 126; (13) the heavy chain variable region comprises the amino acid sequence set forth in SEQ ID NO: 147 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 147, and the light chain variable region comprises the amino acid sequence set forth in SEQ ID NO: 138 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 138; or (14) the heavy chain variable region comprises the amino acid sequence set forth in SEQ ID NO: 149 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 149, and the light chain variable region comprises the amino acid sequence set forth in SEQ ID NO: 138 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 138. In some specific embodiments, the isolated antibody or the antigen-binding fragment thereof binds to Siglec-15.

In some embodiments, the isolated antibody or the antigen-binding fragment thereof comprises a heavy chain comprising a heavy chain variable region and a heavy chain constant region and a light chain comprising a light chain variable region and a light chain constant region, wherein the heavy chain variable region and the light chain variable region comprise the amino acid sequences described above; the heavy chain constant region comprises a human IgG1, IgG2, or IgG4 constant region or a variant thereof, preferably a human IgG1 constant region or a variant thereof; the light chain constant region comprises a human κ constant region or a human λ constant region or a variant thereof, wherein the antibody or the antigen-binding fragment thereof binds to Siglec-15. The variant of a constant region refers to an amino acid sequence having 1, 2, 3, 4, or 5 amino acid substitutions, deletions, and additions relative to the constant region. In some embodiments, the substitution may be a conservative amino acid substitution.

In some embodiments, the isolated antibody or the antigen-binding fragment thereof comprises a heavy chain and a light chain, wherein the heavy chain comprises the amino acid sequence set forth in SEQ ID NO: 2, 20, 38, 56, 74, 92, 110, 113, 116, 128, 146 or 148 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 2, 20, 38, 56, 74, 92, 110, 113, 116, 128, 146 or 148; and/or the light chain comprises the amino acid sequence set forth in SEQ ID NO: 11, 29, 47, 65, 83, 101, 119, 122, 125 or 137 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 11, 29, 47, 65, 83, 101, 119, 122, 125 or 137. In some specific embodiments, the isolated antibody or the antigen-binding fragment thereof binds to Siglec-15.

In some embodiments, the isolated antibody or the antigen-binding fragment thereof comprises a heavy chain and a light chain, wherein: (1) the heavy chain comprises the amino acid sequence set forth in SEQ ID NO: 2 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 2, and the light chain comprises the amino acid sequence set forth in SEQ ID NO: 11 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 11; (2) the heavy chain comprises the amino acid sequence set forth in SEQ ID NO: 20 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 20, and the light chain comprises the amino acid sequence set forth in SEQ ID NO: 29 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 29; (3) the heavy chain comprises the amino acid sequence set forth in SEQ ID NO: 38 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 38, and the light chain comprises the amino acid sequence set forth in SEQ ID NO: 47 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 47; (4) the heavy chain comprises the amino acid sequence set forth in SEQ ID NO: 56 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 56, and the light chain comprises the amino acid sequence set forth in SEQ ID NO: 65 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 65; (5) the heavy chain comprises the amino acid sequence set forth in SEQ ID NO: 74 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 74, and the light chain comprises the amino acid sequence set forth in SEQ ID NO: 83 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 83; (6) the heavy chain comprises the amino acid sequence set forth in SEQ ID NO: 92 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 92, and the light chain comprises the amino acid sequence set forth in SEQ ID NO: 101 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 101; (7) the heavy chain comprises the amino acid sequence set forth in SEQ ID NO: 128 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 128, and the light chain comprises the amino acid sequence set forth in SEQ ID NO: 137 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 137; (8) the heavy chain comprises the amino acid sequence set forth in SEQ ID NO: 110 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 110, and the light chain comprises the amino acid sequence set forth in SEQ ID NO: 101 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 101; (9) the heavy chain comprises the amino acid sequence set forth in SEQ ID NO: 113 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 113, and the light chain comprises the amino acid sequence set forth in SEQ ID NO: 101 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 101; (10) the heavy chain comprises the amino acid sequence set forth in SEQ ID NO: 116 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:

116, and the light chain comprises the amino acid sequence set forth in SEQ ID NO: 119 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 119; (11) the heavy chain comprises the amino acid sequence set forth in SEQ ID NO: 116 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 116, and the light chain comprises the amino acid sequence set forth in SEQ ID NO: 122 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 122; (12) the heavy chain comprises the amino acid sequence set forth in SEQ ID NO: 116 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 116, and the light chain comprises the amino acid sequence set forth in SEQ ID NO: 125 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 125; (13) the heavy chain comprises the amino acid sequence set forth in SEQ ID NO: 146 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 146, and the light chain comprises the amino acid sequence set forth in SEQ ID NO: 137 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 137; or (14) the heavy chain comprises the amino acid sequence set forth in SEQ ID NO: 148 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 148, and the light chain comprises the amino acid sequence set forth in SEQ ID NO: 137 or an amino acid sequence having at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 137. In some specific embodiments, the isolated antibody or the antigen-binding fragment thereof binds to Siglec-15.

In some embodiments, the antibody disclosed herein comprises or consists of two heavy chains and two light chains, the heavy chains and light chains being linked to each other by disulfide bonds, wherein each heavy chain comprises the heavy chain constant region, the heavy chain variable region, or CDR sequences described above, and each light chain comprises the light chain constant region, the light chain variable region, or CDR sequences described above, wherein the C-terminus of the heavy chain variable region is linked to the N-terminus of the heavy chain constant region, and the C-terminus of the light chain variable region is linked to the N-terminus of the light chain constant region.

The antibody disclosed herein may be, e.g., a full-length antibody of the IgG1, IgG2 or IgG4 subclass. In some embodiments, the anti-Siglec-15 antibody or the antigen-binding fragment thereof disclosed herein is an IgG1 subclass antibody. In another embodiment, the antibody disclosed herein may be a single-chain antibody (scFv) or an antibody fragment, such as a Fab fragment, a F(ab')$_2$ fragment, an Fd fragment, an Fv fragment, a dAb, or an isolated CDR region.

In one aspect, the present disclosure provides an antibody or an antigen-binding fragment thereof that binds to the same epitope on human Siglec-15 as any of the exemplary anti-Siglec-15 antibodies or the antigen-binding fragments thereof disclosed herein. In some embodiments, the present disclosure provides an antibody or an antigen-binding fragment thereof that competes for binding to human Siglec-15 with any of the exemplary anti-Siglec-15 antibodies or the antigen-binding fragments thereof disclosed herein.

In one aspect, the present disclosure provides an isolated nucleic acid molecule encoding the antibody or the antigen-binding fragment thereof disclosed herein.

In one aspect, the present disclosure provides an expression vector comprising the aforementioned nucleic acid molecule.

In one aspect, the present disclosure provides a host cell comprising the aforementioned nucleic acid molecule or the aforementioned expression vector.

In one aspect, the present disclosure provides a polypeptide fusion or multispecific antibody comprising the aforementioned antibody or the antigen-binding fragment thereof.

In one aspect, the present disclosure provides a viral vector comprising a nucleic acid molecule encoding the antibody or the antigen-binding fragment thereof disclosed herein or the aforementioned nucleic acid molecule.

In one aspect, the present disclosure provides a pharmaceutical composition comprising the antibody or the antigen-binding fragment thereof disclosed herein and one or more pharmaceutically acceptable excipients, diluents, or carriers, wherein the antibody or the antigen-binding fragment thereof reduces or blocks binding of Siglec-15 to its ligand and/or reduces or blocks Siglec-15-mediated signal transduction.

In one aspect, the present disclosure provides a method for detecting an expression amount of Siglec-15 in a sample derived from a subject, which comprises the step of contacting the sample with the antibody or the antigen-binding fragment thereof disclosed herein under conditions that the antibody or the antigen-binding fragment thereof is capable of forming a complex with Siglec-15 and the step of detecting the formation of the complex or determining the amount of the complex.

In one aspect, the present disclosure provides a method for treating or ameliorating Siglec-15-associated diseases and disorders in a subject in need thereof, which comprises administering to the subject a therapeutically effective amount of the antibody or the antigen-binding fragment thereof disclosed herein, the aforementioned nucleic acid molecule, or the aforementioned pharmaceutical composition.

In some specific embodiments, the Siglec-15-associated diseases or disorders comprise cancers, infections, autoimmune diseases, abnormal bone metabolism, or a combination thereof.

In one aspect, the present disclosure provides a method for regulating an immune response in a subject in need thereof, which comprises administering to the subject a therapeutically effective amount of the antibody or the antigen-binding fragment thereof disclosed herein, the aforementioned nucleic acid molecule, or the aforementioned pharmaceutical composition.

In some specific embodiments, the subject is suffering from a cancer, an infection, an autoimmune disease, abnormal bone metabolism, or a combination thereof.

In one aspect, the present disclosure provides a method for reducing T cell suppression in a subject in need thereof, which comprises administering to the subject a therapeutically effective amount of the antibody or the antigen-binding fragment thereof disclosed herein, the aforementioned nucleic acid molecule, or the aforementioned pharmaceutical composition.

In some specific embodiments, the subject is suffering from a cancer, an infection, an autoimmune disease, abnormal bone metabolism, or a combination thereof.

In one aspect, the present disclosure provides a method for detecting or diagnosing Siglec-15-associated diseases or disorders, which comprises: (a) determining Siglec-15 expression in a cell or a tissue sample of a subject using the antibody or the antigen-binding fragment thereof disclosed herein, and (b) comparing the level of Siglec-15 with a control level, wherein an increase in the determined level of Siglec-15 as compared with the control level indicates the presence of the disease or disorder.

In one aspect, the present disclosure provides a method for monitoring the progression of a disease or disorder, which comprises: (a) determining Siglec-15 expression in a cell or a tissue sample of a subject obtained at a first time point and at a subsequent time point using the antibody or the antigen-binding fragment thereof disclosed herein; and (b) comparing the levels of Siglec-15 expression in the cell or the tissue sample of the subject at the first time point and at the subsequent time point, wherein an increase in the level of Siglec-15 determined at the subsequent time point as compared with the first time point indicates the progression of the disease or disorder.

In one aspect, the present disclosure provides a method for monitoring a response to treatment, which comprises: (a) determining Siglec-15 expression in a cell or a tissue sample of a subject before and after the treatment using the antibody or the antigen-binding fragment thereof disclosed herein; and (b) comparing the levels of Siglec-15 over time, wherein a decrease in the level of Siglec-15 determined after the treatment as compared with the level of Siglec-15 before the treatment indicates good response to the treatment.

Effects of the present invention

In a specific embodiment, the present disclosure uses the single B cell cloning technology (SBC) to select positive clones, which, compared with conventional monoclonal antibody screening technologies, can greatly shorten the development cycle and save a lot of materials and manpower and thereby greatly improve the efficiency of antibody drug discovery.

In a specific embodiment, the anti-Siglec-15 antibody prepared by immunizing a human-derived antibody transgenic mouse has an amino acid sequence of a fully human-derived antibody, so that a lengthy antibody humanization process is omitted, sufficient flexibility and diversity are provided for selecting candidate drug molecules, and thereby the CMC for drug development and drug properties in clinical development are improved. In a specific embodiment, the anti-Siglec-15 antibody disclosed herein has a high affinity for human Siglec-15 protein, with a dissociation constant typically less than $1 \times 10^{-10}$ M.

In a specific embodiment, the anti-Siglec-15 antibody disclosed herein substantially does not bind to Siglec-2, Siglec-3, Siglec-4a, Siglec-10, or PD-L1, as detected by an enzyme-linked immunosorbent assay.

In a specific embodiment, the anti-Siglec-15 antibody disclosed herein can significantly increase the expression level of IFN-$\gamma$ in a T cell experiment.

In a specific embodiment, the anti-Siglec-15 antibody disclosed herein can significantly inhibit myeloid cell survival and TNF-$\alpha$ release in a human myeloid cell experiment.

In a specific embodiment, the anti-Siglec-15 antibody disclosed herein has similar binding ability for human Siglec-15 protein and monkey Siglec-15 protein, e.g., cynomolgus monkey Siglec-15 protein.

**BRIEF DESCRIPTION OF THE DRAWINGS**

FIGs. 1A-1D show the results of the experiment on the binding of recombinant antibodies to human Siglec-15 protein;

FIGs. 2A-2B show the results of the experiment on the binding of recombinant antibodies to cynomolgus monkey Siglec-15 protein;

FIGs. 3A-3B show the results of the experiment on the binding of recombinant antibodies to mouse Siglec-15 protein;

FIGs. 4A-4C show the results of the experiment on the binding of recombinant antibodies to CHO-K1/human Siglec-15;

FIGs. 5A-5B show the results of the experiment on the binding of recombinant antibodies to CHO-K1/cynomolgus monkey Siglec-15;

FIG. 6 shows the results of the experiment on the cross-reaction of recombinant antibodies with other proteins of the same class;

FIGs. 7A-7D show the results of the T cell function assay of recombinant antibodies-secretion of cytokine IFN-$\gamma$;

FIGs. 8A-8D show the results of the myeloid cell function assay of recombinant antibodies-cell proliferation;

FIG. 9 shows the results of the myeloid cell function assay of recombinant antibodies-secretion of cytokine TNF-$\alpha$.

## DETAILED DESCRIPTION

Definitions

In order to better understand the present disclosure, certain technical and scientific terms are specifically defined below. Unless otherwise specifically defined herein, all other technical and scientific terms used herein have the same meaning as commonly understood by those of ordinary skill in the art to which the present disclosure belongs.

In the claims and/or the specification of the present disclosure, unless otherwise stated in the context, an indication such as "a/an" or "said" or "the" is intended to support both the singular and/or plural cases.

As used in the claims and specification, the word "comprise", "have", "include", or "contain" is intended to be inclusive or open-ended and does not exclude additional, unreferenced elements or method steps.

As used herein, the term "about" means that a numerical value includes the standard deviation of error for the device or method used to determine the numerical value. Exemplarily, the aforementioned standard deviation is typically within a range of 20-30% of the original value. In this context, unless otherwise stated, the values of the parameters or conditions in a step are all modified by "about" by default.

Although the disclosure supports the definition of the term "or" as merely an alternative and "and/or", the term "or" in the claims means "and/or" unless it is explicitly stated to be only an alternative or mutually exclusive between alternatives.

As used herein, the term "sequence identity" or "percentage identity" in the comparison of two nucleic acids or polypeptides means that, when compared and aligned for maximum correspondence as measured by the sequence alignment algorithm for nucleotides or amino acid residues or visual inspection, they have a specific percentage of identical sequence. That is, the identity of nucleotide or amino acid sequences can be defined to be such a ratio that is the number of identical nucleotides or amino acids to the total number of nucleotides or amino acids for alignment in the two or more nucleotide or amino acid sequences aligned to have the maximum number of identical nucleotides or amino acids, with gaps introduced as needed.

As used herein, the sequence identity between two or more polynucleotides or polypeptides may be determined by the following: the nucleotide or amino acid sequences of the polynucleotides or polypeptides are aligned and the number of positions in the aligned polynucleotides or polypeptides containing the same nucleotide or amino acid residue is scored and compared with the number of positions in the aligned polynucleotides or polypeptides containing different nucleotides or amino acid residues. Polynucleotides may differ at one position, for example, by containing different nucleotides or missing nucleotides. Polypeptides may differ at one position, for example, by containing different amino acids or missing amino acids. Sequence identity can be calculated by dividing the total number of amino acid residues in the polynucleotide or polypeptide by the number of positions containing the same nucleotide or amino acid residue. For example, percentage identity can be calculated by dividing the total number of nucleotides or amino acid residues in the polynucleotide or polypeptide by the number of positions containing the same nucleotide or amino acid residue and multiplying by 100.

Exemplarily, in the present disclosure, two or more sequences or subsequences have "sequence identity" or "percentage identity" of at least 40%, 50%, 60%, 70%, 80%, 85%, 90%, 91%, 92%, 93%, 94%, 95%, 96%, 97%, 98%, or 99% nucleotides or amino acid residues when compared and aligned for maximum correspondence as measured by the sequence comparison algorithm or visual inspection. The determination/calculation of "sequence identity" or "percentage identity" may be based on any suitable region of the sequence. For example, a region of at least about 10 residues in length, a region of at least about 20 residues in length, a region of at least about 50 residues, a region of at least about 100 residues, a region of at least about 200 residues, or a region of at least about 400 residues. In certain embodiments, the sequence is substantially the same over the entire length of any one or two compared biopolymers (i.e., nucleic acids or polypeptides).

As used herein, the correspondence between the numbering of nucleotides or amino acids in different sequences is based on the numbering of the target nucleotide or target amino acid compared with the reference nucleotide or reference amino acid when the sequences are compared and aligned for maximum correspondence as measured by the nucleotide or amino acid residue sequence comparison algorithm or visual inspection to thereby determine "sequence identity" or "percentage identity". Exemplarily, sequence comparison and percentage identity determination between two sequences can be performed using the BLASTN/BLASTP algorithm on the National Center For Biotechnology Institute with default settings.

As used herein, the term "amino acid mutation" or "nucleotide mutation" includes "substitution, repetition, deletion, or addition of one or more amino acids or nucleotides". In the present disclosure, the term "mutation" refers to a change in a nucleotide sequence or an amino acid sequence. In a specific embodiment, the term "mutation" refers to "substitution".

In an embodiment, a "mutation" of the present disclosure may be selected from the group consisting of "conservative mutations". In the present disclosure, the term "conservative mutation" refers to a mutation that can normally maintain the function of a protein. A representative example of conservative mutation is conservative replacement/substitution. For example, certain amino acids may substitute for other amino acids in the sequence without causing significant loss of activity. Because the interaction capacity and properties of a polypeptide define its biological functional activity, certain amino acid sequence substitutions may be made in the polypeptide sequence and a polypeptide with similar properties still can be obtained.

As used herein, the term "conservative mutation" relates to the replacement of an amino acid residue with an amino acid residue having a similar side chain. Families of amino acid residues having similar side chains have been defined in the art and include amino acids having basic side chains (e.g., lysine, arginine, and histidine), acidic side chains (e.g., aspartic acid and glutamic acid), uncharged polar side chains (e.g., glycine, asparagine, glutamine, serine, threonine, tyrosine, and cysteine), nonpolar side chains (e.g., alanine, valine, leucine, isoleucine, proline, phenylalanine, methionine, and tryptophan), β-branched chains (e.g., threonine, valine, and isoleucine), and aromatic side chains (e.g., tyrosine, phenylalanine, tryptophan, and histidine).

As used herein, the "conservative mutation" typically refers to the substitution of one type of amino acid at one or more sites in a protein. Such substitutions may be conservative. The substitution regarded as a conservative mutation may specifically include a substitution of Ala with Ser or Thr, a substitution of Arg with Gln, His, or Lys, a substitution of Asn with Glu, Gln, Lys, His, or Asp, a substitution of Asp with Asn, Glu, or Gln, a substitution of Cys with Ser or Ala, a substitution of Gin with Asn, Glu, Lys, His, Asp, or Arg, a substitution of Glu with Gly, Asn, Gln, Lys, or Asp, a substitution of Gly with Pro, a substitution of His with Asn, Lys, Gln, Arg, or Tyr, a substitution of Ile with Leu, Met, Val, or Phe, a substitution of Leu with Ile, Met, Val, or Phe, a substitution of Lys with Asn, Glu, Gln, His, or Arg, a substitution of Met with Ile, Leu, Val, or Phe, a substitution of Phe with Trp, Tyr, Met, Ile, or Leu, a substitution of Ser with Thr or Ala, a substitution of Thr with Ser or Ala, a substitution of Trp with Phe or Tyr, a substitution of Tyr with His, Phe, or Trp, and a substitution of Val with Met, Ile, or Leu. In addition, the conservative mutations also include naturally occurring mutations caused by individual differences, differences in strain and species, and the like from which genes are derived.

As used herein, the term "polynucleotide" refers to a polymer composed of nucleotides. A polynucleotide may be in the form of an individual fragment or may be a component of a larger nucleotide sequence structure. It is derived from a nucleotide sequence isolated at least once in quantity or concentration and can be recognized, manipulated, and restored to the sequence and its component nucleotide sequences by standard molecular biology methods (e.g., using cloning vectors). When a nucleotide sequence is represented by a DNA sequence (i.e., A, T, G, C), this also includes an RNA sequence (i.e., A, U, G, C) in which "U" substitutes for "T". In other words, a "polynucleotide" refers to a polymer of nucleotides removed from other nucleotides (either an individual fragment or an entire fragment) or may be a constituent part or component of a larger nucleotide structure, such as an expression vector or a polycistronic sequence. Polynucleotides include DNA, RNA, and cDNA sequences. A "recombinant polynucleotide" is one of the "polynucleotides".

As used herein, the term "recombinant polynucleotide" refers to a polynucleotide having sequences that are not linked together in nature. The recombinant polynucleotide may be included in a suitable vector, and the vector may be used to transform into a suitable host cell. A host cell containing a recombinant polynucleotide is called a "recombinant host cell". The polynucleotide is then expressed in the recombinant host cell to produce, for example, a "recombinant polypeptide".

As used herein, the term "Siglec-15", also known as "sialic acid-binding immunoglobulin-like lectin-15", is a member of the Siglec family, with high expression in multiple immune cells in the tumor microenvironment. In the present disclosure, the amino acid sequence encoding the human Siglec-15 precursor is set forth in SEQ ID NO: 1. In the sequence set forth in SEQ ID NO: 1, amino acids 1-19 are signal peptide amino acids.

The "antibody" in the present disclosure refers to a binding protein having at least one antigen (e.g., Siglec-15) binding domain. The antibody or the antigen-binding fragment thereof in the present disclosure may be an intact antibody (e.g., a full-length antibody) or any fragment thereof, including a monoclonal antibody or a fragment thereof and an antibody variant or a fragment thereof. Examples of the antibody or the antigen-binding fragment thereof include a monospecific antibody or a multispecific antibody, a Fab fragment, a F(ab')$_2$ fragment, an Fv fragment, an isolated CDR region, a single-chain Fv (scFv), and any antibody fragment known in the art. The anti-Siglec-15 antibody or the antigen-binding fragment thereof disclosed herein may be of the IgG1, IgG2, IgG3, or IgG4 subclass. The term "subclass" refers to the class of antibodies encoded by the heavy chain constant region gene. In some embodiments, the anti-Siglec-15 antibody or the antigen-binding fragment thereof disclosed herein is of IgG1 subclass. The anti-Siglec-15 antibody or the antigen-binding fragment thereof disclosed herein may be derived from any species including, but not limited to, mice, rats, rabbits, primates, llamas, and humans. The anti-Siglec-15 antibody or the antigen-binding fragment thereof disclosed herein may be a murine antibody, a chimeric antibody, a humanized antibody, or a fully human-derived antibody. Unless otherwise stated, the "antibody" in the present disclosure includes a full-length antibody and any antigen-binding fragment (i.e., "antigen-binding portion") or single chain thereof.

A conventional "full-length antibody" is a glycoprotein comprising two heavy (H) chains and two light (L) chains, wherein the heavy chains and the light chains are linked by disulfide bonds. Each heavy chain consists of a heavy chain variable region (VH) and a heavy chain constant region (CH). The heavy chain constant region consists of three domains: CH1, CH2, and CH3. Each light chain consists of a light chain variable region (VL) and a light chain constant region (CL). The light chain constant region consists of one domain CL. The VH and VL regions can further be divided into hypervariable regions, i.e., complementarity determining regions (CDRs), and framework regions (FRs) with relatively conservative sequences. Each VH and VL consists of three CDRs and four FRs arranged from the amino-terminus to the carboxy-terminus as follows: FR1, CDR1, FR2, CDR2, FR3, CDR3, FR4. The variable regions of the heavy chains and light chains comprise binding domains that interact with antigens. The constant regions of the antibody can mediate the binding of immunoglobulins to host tissues or factors, including various cells of the immune system (e.g., effector cells) and the first component of the classical complement system (C1q). Meanwhile, as is understood by those skilled in the art, a particular "full-length antibody", e.g., a nanobody, has only heavy (H) chains and no light (L) chains.

In the present disclosure, the "antigen-binding fragment" or "antibody-binding portion" of an antibody refers to one or more fragments of the antibody that retain the function of specifically binding to an antigen (e.g., Siglec-15). It has been demonstrated that the antigen-binding function of an antibody can be implemented by fragments of a full-length antibody. Examples encompassed within the term "antigen-binding portion/fragment" of the antibody include: (i) Fab fragments: monovalent fragments consisting of the $V_L$, $V_H$, CL, and CH1 domains; (ii) F(ab')$_2$ fragments, bivalent fragments comprising two Fab fragments linked by a disulfide bridge in the hinge region; (iii) Fd fragments consisting of the VH and CH1 domains; (iv) Fv fragments consisting of the VL and VH domains of a single arm of the antibody; (v) dAb fragments consisting of the VH domain (see Ward et al., Nature. 341:544-546 (1989)); (vi) isolated complementarity determining regions (CDRs); and (vii) nanobodies, antibodies comprising a single heavy chain variable domain and two constant domains. Furthermore, although the two domains VL and VH of the Fv fragment are encoded by different genes, they can be recombinantly linked by a synthetic linker to form a single protein chain in which VL pairs with VH to form a monovalent molecule (referred to as single-chain Fv (scFv), see, e.g., Bird et al., Science. 242:423-426 (1988); Huston et al., Proc. Natl. Acad. Sci. 85:5879-5883 (1988)). These single-chain antibodies are also encompassed within the term antigen-binding portion/fragment. Furthermore, recombinant polypeptides, fusion proteins, immunoconjugates, and multispecific antibodies comprising such antigen-binding portions/fragments are also encompassed within the term antigen-binding portion/fragment. These antibody fragments can be obtained by those skilled in the art using known conventional techniques, and the fragments can be subjected to functional screening using the same method as that for full-length antibodies.

In the present disclosure, an "isolated antibody" refers to an antibody that is substantially free of other antibodies with different antigenic specificities (e.g., an isolated antibody that specifically binds to Siglec-15 is substantially free of antibodies that specifically bind to antigens other than Siglec-15). However, an isolated antibody that specifically binds to human Siglec-15 may cross bind to other antigens (e.g., Siglec-15 from other species). Furthermore, the isolated antibody is substantially free of other cellular components and/or chemical substances.

In the present disclosure, the "mouse antibody" or "murine antibody" refers to an antibody in which the framework regions and CDRs in the variable region are derived from mouse germline immunoglobulin sequences. Furthermore, if the antibody comprises a constant region, the constant region is also derived from mouse germline immunoglobulin sequences. The mouse antibody disclosed herein may comprise amino acid residues not encoded by mouse germline immunoglobulin sequences (e.g., mutations introduced by *in vitro* random mutation or point mutation or by *in vivo* somatic mutation), but "mouse antibody" does not include antibodies in which CDR sequences derived from other mammalian species are inserted into the mouse framework sequence.

In the present disclosure, a "chimeric antibody" generally refers to an antibody in which a portion of the heavy or light chain amino acid sequence is homologous to or belongs to the same class as that of a corresponding amino acid sequence in an antibody from a particular species, while the other portion of the chain is homologous to a corresponding sequence in another species. For example, variable regions of both the light and heavy chains may be derived from the variable regions of an antibody from one animal species (e.g., mouse, rat, etc.) while the constant portions are homologous to antibody sequences from another species (e.g., human). For example, to obtain a chimeric antibody, B cells or hybridoma cells of non-human origin may be used to produce the variable regions, while the constant regions in combination therewith are derived from a human. Because the constant region of a chimeric antibody may be of human origin, the chimeric antibody may be less likely to elicit an immune response upon injection than antibodies using constant regions of non-human origin.

In the present disclosure, a "humanized antibody" refers to an antibody from a non-human species whose protein sequence has been modified to increase its similarity to a naturally occurring human antibody. It contains fewer sequences from non-human immunoglobulins, thereby reducing the immunogenicity of a heteroantibody when introduced into humans.

In the present disclosure, a "fully human-derived antibody" generally refers to a fully human antibody, i.e., the constant and variable regions of the antibody are all of human origin. The fully human-derived antibody can be obtained by a phage antibody library technology, a technology for preparing a human antibody by a transgenic mouse, a ribosome display technology, an EBV-transformed B cell cloning technology, a single B cell cloning technology, and the like.

Fragments of antibodies against Siglec-15 used herein specifically bind to Siglec-15.

In the present disclosure, an "antibody that specifically binds to human Siglec-15" refers to an antibody that binds to human Siglec-15 (and possibly also Siglec-15 of other non-human species) but substantially does not bind to non-Siglec-15. Preferably, the antibody binds to human Siglec-15 with "high affinity", i.e., a KD of $5.0 \times 10^{-8}$ M or less, $1.0 \times 10^{-8}$ M or less, preferably $5.0 \times 10^{-9}$ M or less, $1.0 \times 10^{-9}$ M or less, and more preferably $5.0 \times 10^{-10}$ M or less, or $1.0 \times 10^{-10}$ M or less.

The term "substantially does not bind to" a protein or cell refers to not binding to the protein or cell, or not binding to it with high affinity, i.e., binding to the protein or cell with a KD of $1.0 \times 10^{-6}$ M or higher, preferably $1.0 \times 10^{-5}$ M or higher, $1.0 \times 10^{-4}$ M or higher, and more preferably $1.0 \times 10^{-3}$ M or higher, or $1.0 \times 10^{-2}$ M or higher.

For IgG, the term "high affinity" refers to a KD of $5.0 \times 10^{-8}$ M or less, $1.0 \times 10^{-8}$ M or less, preferably $5.0 \times 10^{-9}$ M or less, $1.0 \times 10^{-9}$ M or less, and more preferably $5.0 \times 10^{-10}$ M or less. However, for other antibody isotypes, the "high-affinity" binding may be different. For example, the "high-affinity" binding of IgM isotype refers to a KD of $1.0 \times 10^{-6}$ M or less, preferably $1.0 \times 10^{-7}$ M or less, more preferably $1.0 \times 10^{-8}$ M or less.

In the present disclosure, the term "$EC_{50}$", also known as half maximal effective concentration, refers to the concentration of an antibody that can cause 50% of the maximum effect after a specified exposure time.

In the present disclosure, the term "$IC_{50}$", also known as half maximal inhibitory concentration, refers to the concentration of an antibody that inhibits a specific biological or biochemical function by 50% relative to the case where the antibody is absent.

In the present disclosure, the term "subject" includes any human or non-human animal. The term "non-human animal" includes all vertebrates, e.g. mammals and non-mammals, preferably mammals, e.g., non-human primates, sheep, dogs, cats, cows, and horses.

In the present disclosure, the term "therapeutically effective amount" refers to an amount sufficient to achieve, or at least partially achieve, a desired effect. The therapeutically "effective amount" or "effective dose" of a drug or therapeutic agent refers to an amount sufficient to prevent or ameliorate symptoms associated with a disease or disorder and/or alleviate the severity of the disease or disorder, preferably an amount that results in a reduction in the severity of the symptoms of the disease or an increase in the frequency and duration of asymptomatic phases or prevents damage or inability caused by the disease, when used alone or in combination with another therapeutic agent. The therapeutically effective amount is related to the disease to be treated, wherein the actual effective amount can be readily determined by those skilled in the art.

It is well known to those skilled in the art that the CDRs of an antibody can be defined in the art using a variety of methods, such as the Kabat definition scheme based on sequence variability (see Kabat et al., Sequences of Proteins of Immunological Interest, Fifth Edition, National Institutes of Health (U.S.), Bethesda, Maryland (1991)), the Chothia definition scheme based on the location of the structural loop regions (see J Mol Biol 273:927-48, 1997), the ImMuno-GenTics (IMGT) numbering scheme (Lefranc M.P., Immunologist, 7, 132-136 (1999); Lefranc, M.P. et al., Dev. Comp. Immunol., 27, 55-77 (2003)), the AbM numbering scheme (defined by Oxford Molecular's AbM antibody modeling software), the Contact numbering scheme (based on analysis of available complex crystal structures), and combinations of two or more of the Kabat, Chothia, IMGT, AbM, and Contact definition schemes.

In the technical solution of the present disclosure, the Combined definition scheme comprising the Kabat definition and the Chothia definition can be used to determine the amino acid residues in a variable domain sequence. The Combined definition scheme combines the Kabat definition with the Chothia definition to obtain a larger range. See Table 1 below for details. It will be appreciated by those skilled in the art that, unless otherwise specified, the term "CDR" or "complementarity determining region" of a given antibody or a region thereof (e.g., a variable region) will be understood to encompass complementarity determining regions defined by any one of the known schemes. Although the protection scope claimed in the present disclosure is the sequences shown based on the Combined definition scheme, the amino acid sequences corresponding to other schemes for defining CDRs (e.g., Kabat, Chothia, IMGT, AbM, Contact, or other definition schemes) shall also fall within the protection scope of the present disclosure.

Table 1. Schematic table of schemes for defining CDRs (Combined definition scheme) of antibodies disclosed herein

|  | Kabat | Chothia | Combined |
|---|---|---|---|
| LCDR1 | L24--L34 | L24--L34 | L24-L34 |
| LCDR2 | L50--L56 | L50--L56 | L50-L56 |
| LCDR3 | L89--L97 | L89--L97 | L89-L97 |
| HCDR1 | H31--H35 | H26--H32 | H26-H35 |
| HCDR2 | H50--H65 | H52--H56 | H50-H65 |
| HCDR3 | H95--H102 | H95--H102 | H95-H102 |

Aspects of the present disclosure are described in more detail below.

Anti-Siglec-15 antibody specifically binding to Siglec-15 and other beneficial functional features

The anti-Siglec-15 antibody or the antigen-binding fragment thereof disclosed herein specifically binds to Siglec-15 (e.g., human Siglec-15, monkey Siglec-15, and murine Siglec-15). The anti-Siglec-15 antibody or the antigen-binding fragment thereof disclosed herein specifically binds to human Siglec-15 with high affinity. The anti-Siglec-15 antibody or the antigen-binding fragment thereof disclosed herein substantially does not bind to other receptors in the Siglec family, e.g., Siglec-2, Siglec-3, Siglec-4a, or Siglec-10. The anti-Siglec-15 antibody or the antigen-binding fragment thereof disclosed herein substantially does not bind to PD-L1. Preferably, the anti-Siglec-15 antibody disclosed herein is a monoclonal antibody. Furthermore, the antibody may be, for example, a mouse (murine), chimeric, humanized, or fully human monoclonal antibody.

Siglec-15 monoclonal antibody

Preferably, the anti-Siglec-15 antibody or the antigen-binding fragment thereof disclosed herein is an antibody having structural and chemical properties described below, and the amino acid sequence numbers corresponding to exemplary antibodies or antigen-binding fragments thereof are provided in Table 2 and Table 5 below. Some antibodies have identical CDRs, and some antibodies have identical VHs or VLs. The heavy chain constant region of the antibody may be a human IgG1, IgG2, or IgG4 constant region or a variant thereof, preferably a human IgG1 heavy chain constant region or a variant thereof, and the light chain constant region of the antibody may be a human κ constant region, a human λ constant region, or a variant thereof, preferably a human κ light chain constant region or a variant thereof. These antibodies may also comprise a mouse IgG1 heavy chain constant region and/or a mouse κ light chain constant region or a variant thereof.

The heavy chain CDR region and the light chain CDR region of the anti-Siglec-15 antibodies described herein are defined by the Combined numbering scheme. However, as is well known in the art, the CDRs may also be determined by other numbering schemes, including but not limited to the Kabat, Chothia, IMGT, AbM, or Contact numbering scheme/method, based on the heavy/light chain variable region sequence.

The VH and/or VL sequences (or CDR sequences) of other anti-Siglec-15 antibodies that bind to human Siglec-15 can be "mixed and paired" with the VH and/or VL sequences (or CDR sequences) of the antibody disclosed herein. Preferably, when VH and VL chains (or CDRs thereof) are mixed and paired, the VH sequence in a particular VH/VL pair can be substituted with a structurally similar VH sequence. Likewise, it is preferred to replace the VL sequence in a particular VH/VL pair with a structurally similar VL sequence.

Thus, in one embodiment, the antibody or the antigen-binding fragment thereof disclosed herein comprises:

(a) a heavy chain variable region comprising an amino acid sequence listed in Table 2 or Table 5; and
(b) a light chain variable region comprising an amino acid sequence listed in Table 2 or Table 5 or the VL of another anti-Siglec-15 antibody, wherein the antibody or the antigen-binding fragment thereof specifically binds to human Siglec-15.

In another embodiment, the antibody or the antigen-binding fragment thereof disclosed herein comprises:

(a) heavy chain CDR1, CDR2, and CDR3 listed in Table 2 or Table 5; and

(b) light chain CDR1, CDR2, and CDR3 listed in Table 2 or Table 5 or the CDRs of another anti-Siglec-15 antibody, wherein the antibody or the antigen-binding fragment thereof specifically binds to human Siglec-15.

In another embodiment, the antibody or the antigen-binding fragment thereof disclosed herein comprises the heavy chain CDR2 of the anti-Siglec-15 antibody disclosed herein and the CDRs of other antibodies that bind to human Siglec-15, e.g., the heavy chain CDR1 and/or CDR3 and/or the light chain CDR1, CDR2, and/or CDR3 of another anti-Siglec-15 antibody.

Furthermore, it is well known in the art that the CDR3 domain is independent of the CDR1 and/or CDR2 domains and can independently determine the antibody's binding specificity for the same antigen, and it can be predicted that multiple antibodies with the same binding specificity can be produced based on the CDR3 sequence. See, e.g., Klimka et al., British J. of Cancer. 83(2):252-260 (2000); Beiboer et al., J. Mol. Biol. 296:833-849 (2000); Rader et al., Proc. Natl. Acad. Sci. U.S.A. 95:8910-8915 (1998); Barbas et al., J. Am. Chem. Soc. 116:2161-2162 (1994); Barbas et al., Proc. Natl. Acad. Sci. U.S.A. 92:2529-2533 (1995); Ditzel et al., J. Immunol. 157:739-749 (1996); Berezov et al., BLAjournal 8: Scientific Review 8 (2001); Igarashi et al., J. Biochem (Tokyo). 117:452-7 (1995); Bourgeois et al., J. Virol. 72:807-10 (1998); Levi et al., Proc. Natl. Acad. Sci. U.S.A. 90:4374-8 (1993); Polymenis and Stoller, J. Immunol. 152:5218-5329 (1994); and Xu and Davis, Immunity. 13:37-45 (2000); and U.S. Pat. Nos. 6,951,646; 6,914,128; 6,090,382; 6,818,216; 6,156,313; 6,827,925; 5,833,943; 5,762,905, and 5,760,185. These references are all incorporated herein by reference in their entirety.

In another embodiment, the antibody or the antigen-binding fragment thereof disclosed herein comprises the heavy chain CDR2 of the anti-Siglec-15 antibody disclosed herein and at least the heavy and/or light chain CDR3 of the anti-Siglec-15 antibody disclosed herein, and a CDR of another anti-Siglec-15 antibody, wherein the antibody or antigen-binding fragment thereof specifically binds to human Siglec-15. Preferably, these antibodies or antigen-binding fragments thereof and the anti-Siglec-15 antibody disclosed herein (a) compete for binding to Siglec-15; (b) retain functional characteristics; (c) bind to the same epitope; and/or (d) have similar binding affinity.

In another embodiment, the antibody or the antigen-binding fragment thereof disclosed herein further comprises the light chain CDR2 of the anti-Siglec-15 antibody disclosed herein and the light chain CDR of another anti-Siglec-15 antibody, wherein the antibody or the antigen-binding fragment thereof specifically binds to human Siglec-15. In another embodiment, the antibody or the antigen-binding fragment thereof disclosed herein further comprises the heavy and/or light chain CDR1 of the anti-Siglec-15 antibody disclosed herein and a CDR of another anti-Siglec-15 antibody, wherein the antibody or the antigen-binding fragment thereof specifically binds to human Siglec-15.

Conservative mutation

In another embodiment, the antibody or the antigen-binding fragment thereof disclosed herein comprises the CDR1, CDR2, and CDR3 sequences of a heavy chain variable region and/or a light chain variable region comprising one or more conservative mutations relative to the anti-Siglec-15 antibody disclosed herein. It will be understood in the art that some conservative sequence mutations do not eliminate the antigen-binding ability. See, e.g., Brummell et al., Biochem 32:1180-8 (1993); de Wildt et al., Prot. Eng. 10:835-41 (1997); Komissarov et al., J. Biol. Chem. 272:26864-26870 (1997); Hall et al., J. Immunol. 149:1605-12 (1992); Kelley and O'Connell Biochem. 32:6862-35 (1993); Adib-Conquy et al., Int. Immunol. 10: 341-6 (1998); and Beers et al., Clin. Can. Res. 6:2835-43 (2000).

Therefore, in an embodiment, the antibody comprises a heavy chain variable region and/or a light chain variable region each comprising CDR1, CDR2 and CDR3, wherein:

(a) The CDR1 sequence of the heavy chain variable region comprises the sequence listed in Table 2 or Table 5 and/or conservative mutations thereof; and/or
(b) The CDR2 sequence of the heavy chain variable region comprises the sequence listed in Table 2 or Table 5 and/or conservative mutations thereof; and/or
(c) The CDR3 sequence of the heavy chain variable region comprises the sequence listed in Table 2 or Table 5 and/or conservative mutations thereof; and/or
(d) the CDR1 and/or CDR2 and/or CDR3 sequences of the light chain variable region comprise the sequences listed in Table 2 or Table 5 and/or conservative mutations thereof; and
(e) the antibody specifically binds to human Siglec-15.

The antibody disclosed herein features one or more of the following functional properties, e.g., high affinity for human Siglec-15, blocking binding of Siglec-15 to its ligand, and/or reducing or blocking Siglec-15-mediated signal transduction.

In multiple embodiments, the antibody may be a mouse, chimeric, humanized, or fully human-derived antibody or an antigen-binding fragment thereof.

The term "conservative modification" or "conservative mutation" used herein refers to an amino acid modification that does not significantly affect or alter the binding properties of an antibody. Such conservative modifications or conservative mutations include amino acid substitutions, additions, and deletions. Modifications can be introduced into the antibody disclosed herein using standard techniques known in the art, e.g., point mutation and PCR-mediated mutation. Conservative amino acid substitutions are those in which an amino acid residue is replaced with an amino acid residue having a similar side chain. Families of amino acid residues having similar side chains are known in the art. Therefore, one or more amino acid residues in the CDRs of the antibody disclosed herein can be substituted with other amino acid residues of the same side chain family, and the resulting antibody can be tested for retained functions (i.e., the functions described above) using the functional tests described herein.

Engineered and modified antibody

The antibody disclosed herein can be engineered to produce modified antibodies using antibodies having one or more VH/VL sequences of the anti-Siglec-15 antibody disclosed herein as starting materials. One or more residues within one or both of the variable regions (i.e., VH and/or VL) (e.g., within one or more CDRs and/or one or more framework regions) of the antibody can be modified. Furthermore, or alternatively, residues in the constant region can be modified, for example, to alter the effector function of the antibody.

In certain embodiments, CDR grafting can be used to modify the variable regions of the antibody. The antibody interacts with the target antigen mainly through amino acid residues in the six complementarity determining regions (CDRs) of the heavy chain and the light chain. Therefore, the amino acid sequences within the CDRs of each antibody are more diverse than the sequences outside the CDRs. Since the CDR sequences are responsible for the major antibody-antigen interactions, expression vectors in which the CDR sequences of particular naturally occurring antibodies are grafted into the framework sequences of different antibodies with different properties can be constructed to express recombinant antibodies simulating the properties of the particular naturally occurring antibodies (Riechmann et al., Nature. 332:323-327 (1998); Jones et al., Nature. 321:522-525 (1986); Queen et al., Proc. Natl. Acad. U.S.A. 86:10029-10033 (1989); and U.S. Pat. Nos. 5,225,539; 5,530,101; 5,585,089; 5,693,762, and 6,180,370).

Accordingly, another embodiment of the present disclosure relates to an isolated monoclonal antibody or an antigen-binding fragment thereof, which comprises a heavy chain variable region comprising CDR1, CDR2 and CDR3 of the sequences described above in the present disclosure and/or a light chain variable region comprising CDR1, CDR2 and CDR3 of the sequences described above in the present disclosure. Although these antibodies comprise the CDR sequences of the VH and VL of the monoclonal antibody disclosed herein, they may comprise different framework sequences.

Such framework sequences can be found in public DNA databases or public references including germline antibody gene sequences. For example, germline DNA sequences for human heavy chain variable region and light chain variable region genes can be found in the Vbase human germline sequence database (www.mrc-cpe.cam.ac.uk/Vbase) and Kabat et al., (1991), supra; Tomlinson et al., J. Mol. Biol. 227:776-798 (1992); and Cox et al., Eur. J. Immunol. 24:827-836 (1994). In another embodiment, germline DNA sequences for human heavy chain variable region and light chain variable region genes can be found in the Genbank database.

Antibody protein sequences were compared with the protein sequence databases using one of the sequence similarity search methods of Gapped BLAST (Altschul et al, (1997), supra) that are well known to those skilled in the art.

The framework sequences of the antibodies disclosed herein are preferably those that are structurally similar to the framework sequences used for the antibodies disclosed herein. The VH CDR1, CDR2 and CDR3 sequences can be grafted into a framework region that comprises the same sequence as the germline immunoglobulin gene from which the framework sequences were derived, or the CDR sequences can be grafted into a framework region that comprises one or more mutations compared with the germline sequence. For example, in some cases, it may be beneficial to mutate residues in the framework regions; such mutations may maintain or enhance the antigen-binding ability of the antibody (see, e.g., U.S. Pat. Nos. 5,530,101; 5,585,089; 5,693,762, and 6,180,370). Another type of variable region modification is to mutate amino acid residues within CDR1, CDR2, and/or CDR3 of the VH and/or VL to improve one or more properties (e.g., affinity and physicochemical properties) of the target antibody. Mutations may be introduced by point mutation or PCR-mediated mutations, and the effect of the mutations on antibody binding or other functional properties may be assessed through *in vitro* or *in vivo* assays known in the art. Preferably, conservative modifications known in the art are introduced. The conservative modifications may be amino acid substitutions, additions, or deletions, preferably substitutions. Furthermore, typically no more than one, two, three, four, or five residues within each CDR are altered.

Furthermore, in another embodiment, the present disclosure provides an isolated anti-Siglec-15 monoclonal antibody or an antigen-binding fragment thereof, which comprises a heavy chain variable region and a light chain variable region. The heavy chain variable region comprises: (a) an HCDR1 comprising the HCDR1 sequence of the present disclosure or an amino acid sequence comprising 1, 2, 3, 4, or 5 amino acid substitutions, deletions, or additions; (b) an HCDR2 comprising the HCDR2 sequence of the present disclosure or an amino acid sequence comprising 1, 2, 3, 4, or 5 amino acid substitutions, deletions, or additions; and (c) an HCDR3 comprising the HCDR3 sequence of the present disclosure or an amino acid sequence comprising 1, 2, 3, 4, or 5 amino acid substitutions, deletions, or additions. The light chain variable region comprises: (a) an LCDR1 comprising the LCDR1 sequence of the present disclosure or an amino acid sequence comprising 1, 2, 3, 4, or 5 amino acid substitutions, deletions, or additions; (b) an LCDR2 comprising the LCDR2 sequence of the present disclosure or an amino acid sequence comprising 1, 2, 3, 4, or 5 amino acid substitutions, deletions, or additions; and (c) an LCDR3 comprising the LCDR3 sequence of the present disclosure or an amino acid sequence comprising 1, 2, 3, 4, or 5 amino acid substitutions, deletions, or additions.

In a specific embodiment, the aforementioned amino acid substitutions are conservative substitutions between different amino acids.

The engineered antibodies disclosed herein include those antibodies in which the framework region residues of the VH and/or VL are modified to improve the properties of the antibodies. In general, such framework region modifications may reduce the immunogenicity of the antibody. For example, one or more framework residues are "back mutated" into the corresponding germline sequence. More specifically, antibodies undergoing somatic mutation may contain framework region residues that differ from the resulting antibody germline sequence. These residues can be identified by comparing the antibody framework sequence to the germline sequence of the resulting antibody.

Another type of modification involves mutating one or more residues of the framework region or even one or more CDRs to remove T cell epitopes, thereby reducing the immunogenicity that an antibody may produce. This method is also known as "deimmunization" and is described in more detail in U.S. patent publication No. 20030153043.

Furthermore, in addition to the framework region or CDR modifications, another type of modification, e.g., modifications to the Fc region of the antibody disclosed herein by genetic engineering, is often used to alter one or more functional properties of the antibody, such as serum half-life, complement fixation, Fc receptor binding, and/or antigen-dependent cytotoxicity. Furthermore, the antibody disclosed herein may also be chemically modified (e.g., to be linked with one or more chemical functional groups), or modified to alter its glycosylation, to alter one or more functional properties of the antibody.

In one embodiment, the CH1-hinge region is modified, for example, by altering the number of cysteine residues in the hinge region. This method is described in detail in U.S. Pat. No. 5,677,425. Altering the number of cysteine residues in the CH1-hinge region can, for example, facilitate the assembly of the light chain and the heavy chain or change the stability of the antibody.

In another embodiment, the Fc-hinge region of the antibody is mutated to change the biological half-life of the antibody. More specifically, one or more amino acid mutations are introduced into the CH2-CH3 region of the Fc-hinge region such that the antibody has reduced staphylococcal protein A (SpA) binding compared with the antibody with the natural Fc-hinge domain. This method is described in more detail in U.S. Pat. No. 6,165,745.

In another embodiment, the glycosylation of the antibody is modified. For example, deglycosylated antibodies (i.e., antibodies that lack glycosylation) can be prepared. Such glycosylation modifications can be achieved, for example, by altering one or more glycosylation sites within the antibody sequence. For example, one or more amino acid substitutions can be made to eliminate glycosylation sites in one or more variable region frameworks and thus the glycosylation at those sites. Such deglycosylation can increase the affinity of the antibody for the antigen. See, e.g., U.S. Pat. Nos. 5,714,350 and 6,350,861.

Furthermore, antibodies with altered glycosylation, such as low-fucosylated antibodies with reduced fucose residues or antibodies with increased bisecting *N*-acetylglucosamine (GlcNac) structures, can be made. It has been demonstrated that altered glycosylation increases the ADCC activity of the antibody. Such glycosylation modifications can be achieved, for example, by expressing the antibody in a host cell in which the glycosylation system is altered, which is known in the art and can be used as a host cell for expressing the recombinant antibodies disclosed herein to produce antibodies with altered glycosylation. For example, because cell lines Ms704, Ms705, and Ms709 lack the fucosyltransferase gene FUT8 ($\alpha$(1,6)-fucosyltransferase), antibodies expressed in the Ms704, Ms705, and Ms709 cell lines lack fucose. The Ms704, Ms705, and Ms709 FUT8-/- cell lines are prepared by targeted disruption of the FUT8 gene in CHO/DG44 cells using two alternative vectors (see U.S. Pat. No. 20040110704 and Ohnuki et al., Biotechnol Bioeng. 87:614-22 (2004)). As another example, EP1,176,195 describes a cell line with disrupted FUT8 gene function. The gene encodes fucosyltransferase, such that antibodies expressed in such a cell line exhibit low fucosylation by reducing or eliminating $\alpha$-1,6 bond-related enzymes. EP1,176,195 also describes a cell line with low or absent enzymatic activity for adding fucose to *N*-acetylglucosamine bound to the Fc region of an antibody, e.g., the rat myeloma cell line YB2/0 (ATCC CRL 1662). WO03/035835 describes Lec13 cells, a CHO variant cell line, which has a reduced ability to add fucose to Asn(297)-related sugars, which results in low fucosylation in the antibodies expressed by the host cell (see Shields et al., J. Biol. Chem. 277:26733-26740 (2002)). Antibodies with altered glycosylation characteristics can also be prepared in eggs, as described in WO06/089231. Alternatively, antibodies with altered glycosylation characteristics can be prepared in plant cells such as Lemna. WO99/54342 discloses a cell line that is genetically engineered to express glycosyltransferase that modifies glycoproteins (e.g., P(1,4)-N-acetylglucosamine transferase III (GnTIII)) such that antibodies expressed in the cell line exhibit increased bisecting GlcNac structures and thus have enhanced ADCC activity (see also Umana et al., Nat. Biotech. 17:176-180 (1999)). Alternatively, fucosidase is used to cut off the fucose residues of the antibody; for example, $\alpha$-L-fucosidase removes the fucose residues from the antibody (Tarentino et al., Biochem. 14:5516-23 (1975)).

Another type of modification for the antibody disclosed herein is PEGylation. PEGylation of an antibody can, for example, increase the biological (e.g., serum) half-life of the antibody. To obtain a PEGylated antibody, an antibody or a fragment thereof is reacted with polyethylene glycol (PEG), e.g., a reactive ester or aldehyde derivative of PEG, under such conditions that one or more PEG groups are attached to the antibody or the antibody fragment. Preferably, the PEGylation is performed by acylation or alkylation with a reactive PEG molecule (or a similar reactive water-soluble polymer). The term "polyethylene glycol" as used herein includes any form of PEG for producing other protein derivatives, such as mono(C1-C10)alkoxy- or aryloxy-polyethylene glycol or polyethylene glycol-maleimide. In certain embodiments, the antibody to be PEGylated is a deglycosylated antibody. Methods of PEGylation are known in the art and can be applied to the antibody disclosed herein. See, e.g., EP0154316 and EP0401384.

Physical properties of the antibody

Each antibody has a unique isoelectric point (pI), typically within the pH range of 6-9.5. The pI of IgG1 antibodies is typically within the pH range of 7-9.5, while that of IgG4 antibodies is typically within the pH range of 6-8. It is speculated that antibodies with pI values outside the normal range may undergo some unfolding and be unstable under *in vivo* conditions. Therefore, an anti-Siglec-15 antibody with a pI value within the normal range is preferred. This can be achieved by selecting antibodies with pI within the normal range or by mutating surface residues.

Nucleic acid molecule encoding the antibody disclosed herein

In another aspect, the present disclosure provides a nucleic acid molecule encoding the heavy chain and/or light chain variable regions or CDRs of the antibody or the antigen-binding fragment thereof disclosed herein. The nucleic acid molecule may be present as intact cells, as cell lysates, or in partially purified or substantially pure form. The nucleic acid molecule is "isolated" or "substantially pure" when purified from other cellular components or other contaminants, e.g., from other cellular nucleic acids or proteins, by using standard techniques. The nucleic acid molecule disclosed herein may be single-stranded or double-stranded. The nucleic acid molecule disclosed herein may be, for example, DNA or RNA, and may or may not comprise intron sequences. In one preferred embodiment, the nucleic acid molecule is a cDNA molecule.

The nucleic acid molecule disclosed herein can be obtained using standard molecular biology techniques. For antibodies expressed by hybridomas (e.g., hybridomas prepared from transgenic mice carrying human immunoglobulin genes), light chain and heavy chain cDNAs encoding the antibodies prepared from the hybridomas can be obtained by standard PCR amplification or using cDNA cloning techniques. For antibodies obtained from immunoglobulin gene libraries (e.g., using phage display technology), nucleic acids encoding such antibodies can be recovered from the gene libraries.

Preferably, the nucleic acid molecule disclosed herein includes those encoding the VH and VL sequences or CDRs of the Siglec-15 monoclonal antibody disclosed herein. Once the DNA fragments encoding the VH and VL fragments are obtained, operations such as converting the variable region genes into full-length antibody chain genes, Fab fragment genes, or scFv genes can be further conducted using standard recombinant DNA techniques. In these operations, the DNA fragment encoding the VL or VH is operably linked to another DNA fragment encoding another protein, e.g., an antibody constant region or a flexible linker. The term "operably linked" used herein means that two DNA fragments are joined together such that the amino acid sequences encoded by the two DNA fragments are both within the reading frame.

Isolated DNA encoding the VH region can be converted into a full-length heavy chain gene by operably linking the DNA encoding the VH to another DNA molecule encoding the heavy chain constant region (CH1, CH2, and CH3). The sequences of the human heavy chain constant region genes are known in the art, and these DNA fragments can be obtained by standard PCR amplification. The heavy chain constant region may be an IgG1, IgG2, IgG3, IgG4, IgA, IgE, IgM, or IgD constant region, but is most preferably an IgG1 constant region. For the Fab fragment heavy chain gene, the DNA encoding the VH can be operably linked to another DNA molecule encoding only the heavy chain CH1 constant region.

Isolated DNA encoding the VL region can be converted into a full-length light chain gene (as well as a Fab light chain gene) by operably linking the DNA encoding the VL to another DNA molecule encoding the light chain constant region (CL). The sequences of the human light chain constant region genes are known in the art, and these DNA fragments can be obtained by standard PCR amplification. In preferred embodiments, the light chain constant region may be a $\kappa$ or $\lambda$ light chain constant region.

To prepare an scFv gene, a DNA fragment encoding the VH and VL is operably linked to another fragment encoding a flexible linker, for example, another fragment encoding the amino acid sequence $(Gly_4\text{-}Ser)_3$, such that the VH and VL sequences can be expressed as a continuous single-chain protein in which the VL and VH regions are linked by the flexible linker (see, e.g., Bird et al., Science 242:423-426 (1988); Huston et al., Proc Nat. Acad. Sci. USA 85:5879-5883 (1988); and McCafferty et al., Nature 348:552-554 (1990)).

Preparation of the monoclonal antibody disclosed herein

In a specific embodiment, the monoclonal antibody (mAb) disclosed herein can be prepared using the somatic hybridization (hybridoma) technique described in Kohler and Milstein, Nature. 256:495 (1975). Other embodiments for preparing monoclonal antibodies include viral or oncogenic transformation of B lymphocytes and phage display techniques. Chimeric or humanized antibodies are also well known in the art. See, e.g., U.S. Pat. Nos. 4,816,567; 5,225,539; 5,530,101; 5,585,089; 5,693,762; and 6,180,370.

In a specific embodiment, the present disclosure uses the single B cell cloning technology (SBC) to select positive clones, which, compared with conventional monoclonal antibody screening technologies, can greatly shorten the development cycle and save a lot of materials and manpower and thereby greatly improve the efficiency of antibody drug discovery.

In a specific embodiment, the anti-Siglec-15 antibody prepared in the present disclosure by immunizing a human-derived antibody transgenic mouse has an amino acid sequence of a fully human-derived antibody, so that a lengthy antibody humanization process is omitted, sufficient flexibility and diversity are provided for selecting candidate drug molecules, and thereby the CMC and drug properties in clinical development are improved.

The antibody disclosed herein can also be produced in host cell transfectomas using, for example, recombinant DNA techniques in combination with gene transfection methods (e.g., Morrison, S. Science 229:1202, 1985). In one embodiment, DNA encoding partial or full-length light and heavy chains obtained using standard molecular biology techniques is inserted into one or more expression vectors such that the gene is operably linked to transcriptional and translational regulatory sequences. In this case, the term "operably linked" refers to linking the antibody gene into the vector such that the transcriptional and translational regulatory sequences in the vectors perform their intended functions of regulating the transcription and translation of the antibody gene.

The term "regulatory sequence" includes promoters, enhancers, and other expression control elements (e.g., polyadenylation signals) that control the transcription or translation of the antibody gene. Such regulatory sequences are described in Goeddel (Gene Expression Technology. Methods in Enzymology 185, Academic Press, San Diego, CA (1990)). Preferably, regulatory sequences for expression in a mammalian host cell include viral elements that direct the high-level protein expression in mammalian cells, such as promoters and/or enhancers derived from cytomegalovirus (CMV), simian virus 40 (SV40), adenoviruses, e.g., the adenovirus major late promoter (AdMLP). Alternatively, non-viral regulatory sequences such as ubiquitin promoters or $\beta$-globin promoters are used. In addition, the regulatory elements consist of sequences of different origins, such as the SR$\alpha$ promoter system, which comprises the sequence from the SV40 early promoter and the sequence of the long terminal repeat of the human T-cell leukemia type I virus (Takebe et al., Mol Cell. Biol. 8:466-472 (1988)). The expression vector and expression regulatory sequences are compatible with the expression host cell used.

The antibody light chain gene and the antibody heavy chain gene can be inserted into the same expression vector or different expression vectors. In preferred embodiments, variable regions are inserted into an expression vector that has encoded the heavy chain constant region and the light chain constant region of the desired subtype to construct a full-length antibody gene, such that the VH is operably linked to the CH in the vector and the VL is operably linked to the CL in the vector. Alternatively, the recombinant expression vector can encode a signal peptide that facilitates the secretion of antibody chains from the host cell. The antibody chain gene can be cloned into a vector such that the signal peptide is linked to the amino terminus of the antibody chain gene in the reading frame. The signal peptide may be an immunoglobulin signal peptide or a heterologous signal peptide (i.e., a signal peptide from a non-immunoglobulin protein).

In addition to the antibody chain genes and regulatory sequences, the recombinant expression vector disclosed herein may carry other sequences, such as a sequence that regulates replication of the vector in the host cell (e.g., an origin of replication) and a selectable marker gene. The selectable marker gene can be used to select host cells into which the vector has been introduced (see, e.g., U.S. Pat. Nos. 4,399,216; 4,634,665; and 5,179,017). For example, the selectable marker gene typically imparts resistance to a drug such as G418, hygromycin, or methotrexate to the host cell into which the vector has been introduced. Preferred selectable marker genes include the dihydrofolate reductase (DHFR) gene (for methotrexate selection/amplification in dhfr host cells) and the neo gene (for G418 selection).

To express the light chain and the heavy chain, host cells are transfected with expression vectors encoding the heavy chain and the light chain using standard techniques. The term "transfect" encompasses a variety of techniques for introducing exogenous DNA into prokaryotic or eukaryotic host cells, such as electroporation, calcium phosphate precipitation, and DEAE-dextran transfection. Although expressing the antibody disclosed herein in prokaryotic or eukaryotic host cells is theoretically feasible, expressing the antibody in eukaryotic cells is preferred, and expressing the antibody in mammalian host cells is most preferred. This is because eukaryotic cells, particularly mammalian cells, are more likely to assemble and secrete properly folded and immunologically active antibodies than prokaryotic cells.

Preferred mammalian host cells for expressing the recombinant antibody disclosed herein include Chinese hamster ovary cells (CHO cells) (including dhfr-CHO cells administered with a DHFR selectable marker, as described in Urlaub and Chasin, Proc. Natl. Acad. Sci. USA 77:4216-4220 (1980); the DHFR selectable marker is described in RJ Kaufman and PA Sharp. J. Mol. Biol. 159:601-621 (1982)), NSO myeloma cells, COS cells, and SP2 cells. Another preferred expression system, particularly when NSO myeloma cells are used, is the GS gene expression system disclosed in WO87/04462, WO89/01036, and EP338,841. When a recombinant expression vector encoding an antibody gene is introduced into a mammalian host cell, the antibody is prepared by culturing the host cell for a period of time sufficient to allow expression of the antibody in the host cell, or preferably sufficient to allow secretion of the antibody into a medium in which the host cell is grown. Antibodies can be isolated from host cells or cell cultures thereof using protein purification methods.

Polypeptide fusion

In another aspect, the present disclosure provides a polypeptide fusion comprising one or more antibodies or antigen-binding fragments thereof disclosed herein, wherein the antibodies or the antigen-binding fragments thereof disclosed herein are linked to at least one other functional molecule, which may be a peptide or a protein or a non-protein.

In one embodiment, the polypeptide fusion disclosed herein includes immunoconjugates comprising one or more antibodies or antigen-binding fragments thereof disclosed herein and at least one other therapeutic agent (e.g., a cytotoxic agent, a radioisotope, an immunoadhesion molecule, or an imaging agent) linked to the antibody or the antigen-binding fragment thereof disclosed herein, such as an antibody-drug conjugate (ADC).

In one embodiment, the polypeptide fusion disclosed herein includes recombinant polypeptides or fusion proteins comprising one or more antibodies or antigen-binding fragments thereof disclosed herein and at least one other functional fragment (e.g., another peptide, protein, immunocytokine, or receptor ligand) linked to the antibodies or antigen-binding fragments thereof disclosed herein; the other functional fragment may be directed against B7-H1, CTLA4, or LAG3 to enhance immune regulation and bind to multiple mechanisms of action in one molecule, such as ligand blocking, immune cell activation, and direct tumor targeting.

In some embodiments, the linkage is a covalent linkage. In some embodiments, the linkage is a non-covalent linkage. In some embodiments, the linkage is a direct linkage. In some embodiments, the linkage is via a linker. A suitable linker includes, but is not limited to, an amino acid or a polypeptide linker. In other embodiments, the polypeptide fusion disclosed herein also includes other forms. Polypeptide fusions in the forms described above and other forms can be prepared by methods well known in the art, such as genetic engineering, chemical methods, and the like.

Multispecific Antibody

In another aspect, the present disclosure provides a multispecific antibody comprising one or more antibodies or antigen-binding fragments thereof disclosed herein. The multispecific antibody comprises an antibody or an antigen-binding fragment thereof disclosed herein and at least one antibody or antigen-binding fragment thereof having a different specificity than the antibody or antigen-binding fragment disclosed herein and is capable of binding to at least two different binding sites or targets. The "multispecific antibody" used herein encompasses antibodies with two types of specificity (i.e., a bispecific antibody), three types of specificity (i.e., a trispecific antibody), four types of specificity (i.e., a tetraspecific antibody), or more types of specificity. These multispecific antibodies can be prepared by methods well known in the art.

In one specific embodiment, the multispecific antibody disclosed herein may also have one or more other types of specificity in addition to the Fc binding specificity and the Siglec-15 binding specificity. Exemplarily, the other specificity may be for B7-H1, CTLA4, or LAG3.

In one specific embodiment, the multispecific antibody may be present in a variety of different forms and sizes. Exemplarily, in terms of the bispecific antibody, at one end of the size spectrum, the bispecific molecule remains in conventional antibody form except that it has two binding arms with different types of specificity rather than two binding arms with the same specificity. At the other end, the bispecific antibody consists of two single-chain antibody fragments (scFv) linked by a peptide chain, known as the Bs(scFv)$_2$ construct. Medium-sized bispecific antibodies comprise two different F(ab) fragments linked by a peptide linker. These and other forms of bispecific antibodies can be prepared by genetic engineering or somatic hybridization, or chemical methods. See, e.g., Kufer et al., cited supra; Cao and Suresh, Bioconjugate Chemistry, 9 (6), 635-644 (1998); and van Spriel et al., Immunology Today, 21 (8), 391-397 (2000).

Viral vector

In another aspect, the antibody or the antigen-binding fragment thereof disclosed herein can also be encoded or carried by a viral vector. In addition, the antibody or the antigen-binding fragment thereof disclosed herein may also be used with the viral vector, or the viral vector encoding or carrying the antibody or the antigen-binding fragment thereof disclosed herein is introduced into humans.

The present disclosure also provides a pharmaceutical composition comprising the polypeptide fusion, the multispecific antibody, or the viral vector disclosed herein and a pharmaceutically acceptable excipient, diluent, or carrier.

Pharmaceutical composition

In another aspect, the present disclosure provides a pharmaceutical composition comprising the antibody or the antigen-binding fragment thereof disclosed herein and a pharmaceutically acceptable excipient, diluent, or carrier. The pharmaceutical composition may optionally comprise one or more other pharmaceutically active ingredients, such as another antibody or drug, e.g. another anti-Siglec-15 antibody or another antibody against B7-H1, CTLA4, or LAG3.

Preferably, the pharmaceutical composition is suitable for intravenous, intramuscular, subcutaneous, parenteral, spinal, or epidermal administration (e.g., by injection or infusion). Depending on the route of administration, the active ingredient may be encapsulated in a material to protect it from acids and other natural conditions that may inactivate it. "Parenteral administration" refers to a mode that is different from enteral administration and topical administration and that is typically performed by injection, including but not limited to, intravenous, intramuscular, intraarterial, intramembranous, intracapsular, intraorbital, intracardiac, intradermal, intraperitoneal, transtracheal, subcutaneous, subcuticular, intraarticular, subcapsular, subarachnoid, intraspinal, epidural, and intrasternal injection and bolus injection. Alternatively, the pharmaceutical composition disclosed herein may be administered through a non-parenteral route, e.g., topical, epidermal, or mucosal administration, e.g., intranasal, oral, vaginal, rectal, sublingual, or topical administration.

The pharmaceutical compositions may be in the form of sterile aqueous solutions or dispersions. They may also be formulated in microemulsions, liposomes, or other ordered structures suitable for high concentrations of drugs. The administration regimen is adjusted to provide the best desired response (e.g., therapeutic response). For example, a single large dose may be administered, multiple split doses may be administered over time, or the dose may be reduced or increased in proportion to the criticality of the treatment situation. It is particularly advantageous to formulate parenteral compositions in dosage units featuring ease of administration and uniformity of dosage. Dosage unit refers to physically separate units that are suitable for single administration to a treated subject; each unit contains a predetermined amount of the active ingredient calculated to produce the desired therapeutic effect in association with the pharmaceutical carrier. Alternatively, the antibody may be administered in a sustained-release formulation, in which case the frequency of administration required is reduced.

For the administration of the composition, the dose may be about 0.0001 to 100 mg/kg of host body weight, more usually 0.01 to 5 mg/kg.

The pharmaceutical composition may be a sustained-release agent, including implants, transdermal patches, and microencapsulated delivery systems. Biodegradable and biocompatible polymers such as ethylene-vinyl acetate, polyanhydrides, polyglycolic acid, collagen, polyorthoesters, and polylactic acid may be used.

In certain embodiments, the antibody disclosed herein may be formulated to ensure proper *in vivo* distribution. For example, to ensure that the therapeutic antibody disclosed herein crosses the blood-brain barrier, the antibody may be formulated in liposomes, which may additionally contain targeting functional groups to enhance selective delivery to particular cells or organs.

Use and method of the present disclosure

The antibody or the antigen-binding fragment thereof, the nucleic acid molecule, the pharmaceutical composition, the polypeptide fusion, the multispecific antibody, or the viral vector disclosed herein has a variety of *in vitro* and *in vivo* applications relating to the diagnosis, treatment, and/or prevention of a Siglec-15-associated disease and disorder. The Siglec-15-associated diseases and disorders include, but are not limited to, cancers, infections, autoimmune diseases, or abnormal bone metabolism. The antibody or the antigen-binding fragment thereof, the nucleic acid molecule, the pharmaceutical composition, the polypeptide fusion, the multispecific antibody, or the viral vector disclosed herein can be administered to a human subject to prevent, alleviate, ameliorate, or treat the diseases or disorders.

The Siglec-15-associated diseases or disorders are well known to those skilled in the art and include, but are not limited to, cancers, infections, autoimmune diseases, or abnormal bone metabolism.

The autoimmune diseases that can be prevented and/or treated by the anti-Siglec-15 antibody or the antigen-binding fragment thereof, the nucleic acid molecule, the pharmaceutical composition, the polypeptide fusion, the multispecific antibody, or the viral vector disclosed herein include, but are not limited to, rheumatoid arthritis, systemic lupus erythematosus, psoriasis, asthma, atopic dermatitis, allergic rhinitis, and the like.

The "cancer" described herein refers to any malignant and/or invasive growth or tumor caused by abnormal cell growth, including solid tumor, blood cancer, bone marrow cancer, or lymphatic system cancer named after the type of cells forming them. The term "cancer" includes, but is not limited to, a primary cancer originating at a specific site in the body, a metastatic carcinoma that has spread from its initial location to other sites in the body, the recurrence of a primary cancer after remission, and a second primary cancer (a new primary cancer in a patient with a history of a cancer of different type).

The cancer that can be prevented and/or treated by the anti-Siglec-15 antibody or the antigen-binding fragment thereof, the nucleic acid molecule, the pharmaceutical composition, the polypeptide fusion, the multispecific antibody or the viral vector and the method disclosed herein is cancer associated with Siglec-15, e.g., cancer associated with expression of abnormally high levels of Siglec-15 or its ligands. The cancer includes, but is not limited to, skin cancer, stomach cancer, esophageal cancer, gastrointestinal cancer (including colon cancer and rectal cancer), bile duct cancer, uterine cancer (including endometrial cancer), kidney cancer, bladder cancer, prostate cancer, ovarian cancer, breast cancer, lung cancer (including non-small cell lung cancer and small cell lung cancer), liver cancer, pancreatic cancer, thyroid cancer, head and neck cancer (including head and neck squamous cell carcinoma), bone and connective tissue sarcomas (including osteosarcoma and soft tissue sarcoma), melanoma, leukemia (including acute leukemia, acute lymphocytic leukemia, acute myelocytic leukemia, and chronic leukemia), lymphoma (including Hodgkin's lymphoma and non-Hodgkin's lymphoma), multiple myeloma, and brain tumors (including neuroblastoma and glioma).

The "infection" described herein may be caused by bacteria, viruses, protozoa, helminths, or other microbial pathogens that enter the cell and are attacked by cytotoxic T lymphocytes.

The "infection" described herein may be acute or chronic. Acute infection is usually infection of short duration. During acute microbial infection, immune cells begin to express immunoregulatory receptors. Thus, in some embodiments, the uses and methods disclosed herein include enhancing an immune stimulatory response against an acute infection.

In some embodiments, the anti-Siglec-15 antibody or the antigen-binding fragment thereof, the nucleic acid molecule, the pharmaceutical composition, the polypeptide fusion, the multispecific antibody, or the viral vector disclosed herein is used to treat a chronic infection, e.g., an infection in which T cell exhaustion or T cell anergy has occurred and thereby the infection is present for a longer time along with the host. Because viral infection is primarily cleared by T cells, an increase in T cell activity can be therapeutically used to more rapidly or completely clear the infecting virus. Thus, the anti-Siglec-15 antibody or the antigen-binding fragment thereof, the nucleic acid molecule, the pharmaceutical composition, the polypeptide fusion, the multispecific antibody, or the viral vector disclosed herein can be administered for the treatment of a local or systemic viral infection. Abnormal bone metabolism that can be prevented and/or treated by the anti-Siglec-15 antibody or the antigen-binding fragment thereof, the nucleic acid molecule, the pharmaceutical composition, the polypeptide fusion, the multispecific antibody, or the viral vector disclosed herein includes, but is not limited to, osteoporosis, bone destruction associated with rheumatoid arthritis, cancerous hypercalcemia, bone destruction associated with multiple myeloma and bone metastasis of cancer, giant cell tumor, tooth loss due to periodontitis, osteolysis, bone destruction in chronic osteomyelitis, Paget's disease of bone, renal osteodystrophy, and osteogenesis imperfecta. In a diagnostic process, the antibody disclosed herein can be contacted with a sample of a subject under conditions that allow the antibody or the antigen-binding fragment thereof disclosed herein to bind to Siglec-15 to detect the amount of the Siglec-15 protein in the sample of the subject.

These and other methods of the present disclosure are discussed further below.

Combination therapy

The anti-Siglec-15 antibody or the antigen-binding fragment thereof, the nucleic acid molecule, the pharmaceutical composition, the polypeptide fusion, the multispecific antibody, or the viral vector provided herein may be administered alone or in combination with one or more other therapeutic agents capable of preventing, alleviating, ameliorating, or treating Siglec-15-associated diseases and disorders, including but not limited to cancers, infections, autoimmune diseases, or abnormal bone metabolism, in a subject.

Exemplary other therapeutic agents include, but are not limited to, cytokines, chemotherapeutic agents (including cytotoxic agents), radionuclides, other immunotherapeutic agents (including antibodies against B7-H1, PD-1, and CTLA4), enzymes, antibiotics, antiviral agents (particularly protease inhibitors, alone or in combination with nucleosides for the treatment of HIV or hepatitis B or hepatitis C), antiparasitic agents (helminths and protozoans), growth factors, growth inhibitors, hormones, hormone antagonists, antibodies and biologically active fragments thereof (including humanized antibodies, single-chain antibodies, and chimeric antibodies), antigen and vaccine formulations (including adjuvants), peptide drugs, anti-inflammatory agents, ligands that bind to Toll-like receptors (including but not limited to polyinosinic acid: polycytidylic acid (poly I:C) and CpG oligonucleotide) to activate the innate immune system, molecules that mobilize and optimize the adaptive immune system, other molecules that activate or up-regulate the actions of cytotoxic T lymphocytes, natural killer cells, and helper T cells, and other molecules that inactivate or down-regulate inhibitory factors or regulatory T cells.

In one embodiment, the present disclosure provides a method for treating a cancer in a subject, wherein the anti-Siglec-15 antibody or the antigen-binding fragment thereof disclosed herein is administered together with one or more other antibodies, e.g., an antibody against B7-H1, CTLA4, or LAG3. In one embodiment, the present disclosure provides a method for treating a cancer in a subject, wherein the anti-Siglec-15 antibody or the antigen-binding fragment thereof disclosed herein is administered together with one or more chemotherapeutic agents, e.g., alkylating agents, podophyllums, camptothecin analogs, taxoids, antimetabolites, and antibiotics. In certain embodiments, the subject is a human. Others that may be used in combination with the anti-Siglec-15 antibody or the antigen-binding fragment thereof disclosed herein to treat a cancer include radiation therapy, surgery, and the like.

The "treatment" refers to a method for alleviating the progression or severity of a symptom, a disorder, a condition, or a disease.

The combination of therapeutic agents described herein can be administered simultaneously as a single composition in a pharmaceutically acceptable carrier, or administered simultaneously as separate compositions, wherein each agent is in a pharmaceutically acceptable carrier. In another embodiment, the combination of therapeutic agents may be administered sequentially.

Furthermore, if the combination therapy is administered multiple times and the agents are administered sequentially, the sequence in the sequential administration at each time point can be reversed or maintained, and the sequential administration can be combined with simultaneous administration or any combination thereof. Unless otherwise indicated, the practice of the present disclosure will use conventional methods in protein chemistry, biochemistry, recombinant DNA technology, and pharmacology within the art.

## Examples

Other objects, features, and advantages of the present disclosure will become apparent from the following detailed description. However, it should be understood that the detailed description and specific examples, while indicating specific embodiments of the present disclosure, are provided for explanation only, since various changes and modifications made within the spirit and scope of the present disclosure will become apparent to those skilled in the art after reading this detailed description.

All reagents used in the examples are commercially available, unless otherwise stated.

As described above, the definition schemes involved below for CDR sequences of antibodies are all Combined definition scheme.

In the technical schemes of the present disclosure, the meanings represented by the numbers of the nucleotide and amino acid sequence listing in the specification are as follows:

SEQ ID NO: 1 shows the amino acid sequence of the human Siglec-15 precursor, which is as follows:

MEKSIWLLACLAWVLPTGSFVRTKIDTTENLLNTEVHSSPAQRWSMQVPPEVSAEAGDAAVLPCTFTHPH
RHYDGPLTAIWRAGEPYAGPQVFRCAAARGSELCQTALSLHGRFRLLGNPRRNDLSLRVERLALADDRR
YFCRVEFAGDVHDRYESRHGVRLHVTAAPRIVNISVLPSPAHAFRALCTAEGEPPPALAWSGPALGNSLAA
VRSPREGHGHLVTAELPALTHDGRYTCTAANSLGRSEASVYLFRFHGASGASTVALLLGALGFKALLLLG
VLAARAARRRPEHLDTPDTPPRSQAQESNYENLSQMNPRSPPATMCSP

wherein, the underlined part of the aforementioned sequence shows the signal peptide (amino acids at positions 1-19) of the human Siglec-15 precursor.

SEQ ID NO: 2 shows the amino acid sequence of the heavy chain (H) of the PR300526 antibody, which is as follows:

QVQLVQSGAEVKKPGASVKVSCKASGYTFTDYYMHWVRQAPGQGLEWMGWINPNSDGTNYAQRFQG

RVTMTRDTSISTAYMELSRLRSDDTAVYYCARGRLLRFFDWLDVMDAWGQGTSVTVSSASTKGPSVFPL APSSKSTSGGTAALGCLVKDYFPEPVTVSWNSGALTSGVHTFPAVLQSSGLYSLSSVVTVPSSSLGTQTYIC NVNHKPSNTKVDKKVEPKSCDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPIEKTISK AKGQPREPQVYTLPPSREEMTKNQVSLTCLVKGFYPSDIAVEWESNGQPENNYKTTPPVLDSDGSFFLYS KLTVDKSRWQQGNVFSCSVMHEALHNHYTQKSLSLSPGK

SEQ ID NO: 3 shows the amino acid sequence of the heavy chain variable region (VH) of the PR300526 antibody, which is as follows:

QVQLVQSGAEVKKPGASVKVSCKASGYTFTDYYMHWVRQAPGQGLEWMGWINPNSDGTNYAQRFQG RVTMTRDTSISTAYMELSRLRSDDTAVYYCARGRLLRFFDWLDVMDAWGQGTSVTVSS

SEQ ID NO: 4 shows the amino acid sequence of the first framework region of the heavy chain (HFR1) of the PR300526 antibody, which is as follows:
QVQLVQSGAEVKKPGASVKVSCKAS
SEQ ID NO: 5 shows the amino acid sequence of the first complementarity determining region of the heavy chain (HCDR1) of the PR300526 antibody, which is as follows:
GYTFTDYYMH
SEQ ID NO: 6 shows the amino acid sequence of the second framework region of the heavy chain (HFR2) of the PR300526 antibody, which is as follows:
WVRQAPGQGLEWMG
SEQ ID NO: 7 shows the amino acid sequence of the second complementarity determining region of the heavy chain (HCDR2) of the PR300526 antibody, which is as follows:
WINPNSDGTNYAQRFQG
SEQ ID NO: 8 shows the amino acid sequence of the third framework region of the heavy chain (HFR3) of the PR300526 antibody, which is as follows:
RVTMTRDTSISTAYMELSRLRSDDTAVYYCAR
SEQ ID NO: 9 shows the amino acid sequence of the third complementarity determining region of the heavy chain (HCDR3) of the PR300526 antibody, which is as follows:
GRLLRFFDWLDVMDA
SEQ ID NO: 10 shows the amino acid sequence of the fourth framework region of the heavy chain (HFR4) of the PR300526 antibody, which is as follows:
WGQGTSVTVSS
SEQ ID NO: 11 shows the amino acid sequence of the light chain (L) of the PR300526 antibody, which is as follows:

DIVMTQSPDSLAVSLGERATINCKSSRSVLYSSNNKNYLAWYQQKPGQPPKLLIYWASTRESGVPDRFSGS GSGTDFTLTISSLQAEDVAVYYCQQYYSIPYTFGQGTKLEIKRTVAAPSVFIFPPSDEQLKSGTASVVCLLN NFYPREAKVQWKVDNALQSGNSQESVTEQDSKDSTYSLSSTLTLSKADYEKHKVYACEVTHQGLSSPVT KSFNRGEC

SEQ ID NO: 12 shows the amino acid sequence of the light chain variable region (VL) of the PR300526 antibody, which is as follows:

DIVMTQSPDSLAVSLGERATINCKSSRSVLYSSNNKNYLAWYQQKPGQPPKLLIYWASTRESGVPDRFSGS GSGTDFTLTISSLQAEDVAVYYCQQYYSIPYTFGQGTKLEIK

SEQ ID NO: 13 shows the amino acid sequence of the first framework region of the light chain (LFR1) of the PR300526 antibody, which is as follows:
DIVMTQSPDSLAVSLGERATINC
SEQ ID NO: 14 shows the amino acid sequence of the first complementarity determining region of the light chain (LCDR1) of the PR300526 antibody, which is as follows:
KSSRSVLYSSNNKNYLA

SEQ ID NO: 15 shows the amino acid sequence of the second framework region of the light chain (LFR2) of the PR300526 antibody, which is as follows:

WYQQKPGQPPKLLIY

SEQ ID NO: 16 shows the amino acid sequence of the second complementarity determining region of the light chain (LCDR2) of the PR300526 antibody, which is as follows:

WASTRES

SEQ ID NO: 17 shows the amino acid sequence of the third framework region of the light chain (LFR3) of the PR300526 antibody, which is as follows:

GVPDRFSGSGSGTDFTLTISSLQAEDVAVVYYC

SEQ ID NO: 18 shows the amino acid sequence of the third complementarity determining region of the light chain (LCDR3) of the PR300526 antibody, which is as follows:

QQYYSIPYT

SEQ ID NO: 19 shows the amino acid sequence of the fourth framework region of the light chain (LFR4) of the PR300526 antibody, which is as follows:

FGQGTKLEIK

SEQ ID NO: 20 shows the amino acid sequence of the heavy chain (H) of the PR300659 antibody, which is as follows:

QVQLVESGGGVVQPGRSLRLSCAASGFIFSSYDIYWVRQAPGKGLEWVAVIWHDGSNKYYADSVKGRFT
ISRDNSKNTVYLQMNSLRAEDTAVYYCARDTHSYGSRIYYDYNYGMDVWGQGTTVTVSSASTKGPSVF
PLAPSSKSTSGGTAALGCLVKDYFPEPVTVSWNSGALTSGVHTFPAVLQSSGLYSLSSVVTVPSSSLGTQTY
ICNVNHKPSNTKVDKKVEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTTPPVLDSDGSFFLY
SKLTVDKSRWQQGNVFSCSVMHEALHNHYTQKSLSLSPGK

SEQ ID NO: 21 shows the amino acid sequence of the heavy chain variable region (VH) of the PR300659 antibody, which is as follows:

QVQLVESGGGVVQPGRSLRLSCAASGFIFSSYDIYWVRQAPGKGLEWVAVIWHDGSNKYYADSVKGRFT
ISRDNSKNTVYLQMNSLRAEDTAVYYCARDTHSYGSRIYYDYNYGMDVWGQGTTVTVSS

SEQ ID NO: 22 shows the amino acid sequence of the first framework region of the heavy chain (HFR1) of the PR300659 antibody, which is as follows:

QVQLVESGGGVVQPGRSLRLSCAAS

SEQ ID NO: 23 shows the amino acid sequence of the first complementarity determining region of the heavy chain (HCDR1) of the PR300659 antibody, which is as follows:

GFIFSSYDIY

SEQ ID NO: 24 shows the amino acid sequence of the second framework region of the heavy chain (HFR2) of the PR300659 antibody, which is as follows:

WVRQAPGKGLEWVA

SEQ ID NO: 25 shows the amino acid sequence of the second complementarity determining region of the heavy chain (HCDR2) of the PR300659 antibody, which is as follows:

VIWHDGSNKYYADSVKG

SEQ ID NO: 26 shows the amino acid sequence of the third framework region of the heavy chain (HFR3) of the PR300659 antibody, which is as follows:

RFTISRDNSKNTVYLQMNSLRAEDTAVYYCAR

SEQ ID NO: 27 shows the amino acid sequence of the third complementarity determining region of the heavy chain (HCDR3) of the PR300659 antibody, which is as follows:

DTHSYGSRIYYDYNYGMDV

SEQ ID NO: 28 shows the amino acid sequence of the fourth framework region of the heavy chain (HFR4) of the PR300659 antibody, which is as follows:

WGQGTTVTVSS

SEQ ID NO: 29 shows the amino acid sequence of the light chain (L) of the PR300659 antibody, which is as follows:

DIQMTQSPSSLSASVGDRVSITCRASQSISSYLNWYQQKPGKAPKLLIYAASSLQSGVPSRFSGSGSGADFT
LTISSLQPEDFATYYCQQTYSPPFTFGPGTKVDIKRTVAAPSVFIFPPSDEQLKSGTASVVCLLNNFYPREAK
VQWKVDNALQSGNSQESVTEQDSKDSTYSLSSTLTLSKADYEKHKVYACEVTHQGLSSPVTKSFNRGEC
SEQ ID NO: 30

shows the amino acid sequence of the light chain variable region (VL) of the PR300659 antibody, which is as follows:

DIQMTQSPSSLSASVGDRVSITCRASQSISSYLNWYQQKPGKAPKLLIYAASSLQSGVPSRFSGSGSGADFT
LTISSLQPEDFATYYCQQTYSPPFTFGPGTKVDIK

SEQ ID NO: 31 shows the amino acid sequence of the first framework region of the light chain (LFR1) of the PR300659 antibody, which is as follows:
DIQMTQSPSSLSASVGDRVSITC
SEQ ID NO: 32 shows the amino acid sequence of the first complementarity determining region of the light chain (LCDR1) of the PR300659 antibody, which is as follows:
RASQSISSYLN
SEQ ID NO: 33 shows the amino acid sequence of the second framework region of the light chain (LFR2) of the PR300659 antibody, which is as follows:
WYQQKPGKAPKLLIY
SEQ ID NO: 34 shows the amino acid sequence of the second complementarity determining region of the light chain (LCDR2) of the PR300659 antibody, which is as follows:
AASSLQS
SEQ ID NO: 35 shows the amino acid sequence of the third framework region of the light chain (LFR3) of the PR300659 antibody, which is as follows:
GVPSRFSGSGSGADFTLTISSLQPEDFATYYC
SEQ ID NO: 36 shows the amino acid sequence of the third complementarity determining region of the light chain (LCDR3) of the PR300659 antibody, which is as follows:
QQTYSPPFT
SEQ ID NO: 37 shows the amino acid sequence of the fourth framework region of the light chain (LFR4) of the PR300659 antibody, which is as follows:
FGPGTKVDIK
SEQ ID NO: 38 shows the amino acid sequence of the heavy chain (H) of the PR300670 antibody, which is as follows:

QVQLVESGGGVVQPGRSLRLSCVASGFTFSTYGIHWVRQAPGKGLDWVSVIWYDGSHKYYADSVQDRF
TISRDNSKNTLYLQMNSLRAEDTAVYYCAREGMITMVRGVIIDYQYHGMDVWGQGTTVTVSSASTKGPS
VFPLAPSSKSTSGGTAALGCLVKDYFPEPVTVSWNSGALTSGVHTFPAVLQSSGLYSLSSVVTVPSSSLGTQ
TYICNVNHKPSNTKVDKKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEYKCKVSNKALPAPIEKT
ISKAKGQPREPQVYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 39 shows the amino acid sequence of the heavy chain variable region (VH) of the PR300670 antibody, which is as follows:

QVQLVESGGGVVQPGRSLRLSCVASGFTFSTYGIHWVRQAPGKGLDWVSVIWYDGSHKYYADSVQDRF
TISRDNSKNTLYLQMNSLRAEDTAVYYCAREGMITMVRGVIIDYQYHGMDVWGQGTTVTVSS

SEQ ID NO: 40 shows the amino acid sequence of the first framework region of the heavy chain (HFR1) of the PR300670 antibody, which is as follows:
QVQLVESGGGVVQPGRSLRLSCVAS
SEQ ID NO: 41 shows the amino acid sequence of the first complementarity determining region of the heavy chain (HCDR1) of the PR300670 antibody, which is as follows:
GFTFSTYGIH
SEQ ID NO: 42 shows the amino acid sequence of the second framework region of the heavy chain (HFR2) of the PR300670 antibody, which is as follows:
WVRQAPGKGLDWVS
SEQ ID NO: 43 shows the amino acid sequence of the second complementarity determining region of the heavy chain (HCDR2) of the PR300670 antibody, which is as follows:
VIWYDGSHKYYADSVQD
SEQ ID NO: 44 shows the amino acid sequence of the third framework region of the heavy chain (HFR3) of the PR300670 antibody, which is as follows:
RFTISRDNSKNTLYLQMNSLRAEDTAVYYCAR
SEQ ID NO: 45 shows the amino acid sequence of the third complementarity determining region of the heavy chain (HCDR3) of the PR300670 antibody, which is as follows:
EGMITMVRGVIIDYQYHGMDV
SEQ ID NO: 46 shows the amino acid sequence of the fourth framework region of the heavy chain (HFR4) of the PR300670 antibody, which is as follows:
WGQGTTVTVSS
SEQ ID NO: 47 shows the amino acid sequence of the light chain (L) of the PR300670 antibody, which is as follows:

DIQMTQSPSTLSASVGDRVTITCRASQSIGRRLAWYQQKPGKAPKLLIYKASSLESGVPSRFSGSGSGTEFT
LTISSPQPDDFATYYCQQYHSYSYTFGQGTKLEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGE
C

SEQ ID NO: 48 shows the amino acid sequence of the light chain variable region (VL) of the PR300670 antibody, which is as follows:

DIQMTQSPSTLSASVGDRVTITCRASQSIGRRLAWYQQKPGKAPKLLIYKASSLESGVPSRFSGSGSGTEFT
LTISSPQPDDFATYYCQQYHSYSYTFGQGTKLEIK

SEQ ID NO: 49 shows the amino acid sequence of the first framework region of the light chain (LFR1) of the PR300670 antibody, which is as follows:
DIQMTQSPSTLSASVGDRVTITC
SEQ ID NO: 50 shows the amino acid sequence of the first complementarity determining region of the light chain (LCDR1) of the PR300670 antibody, which is as follows:
RASQSIGRRLA
SEQ ID NO: 51 shows the amino acid sequence of the second framework region of the light chain (LFR2) of the PR300670 antibody, which is as follows:
WYQQKPGKAPKLLIY
SEQ ID NO: 52 shows the amino acid sequence of the second complementarity determining region of the light chain (LCDR2) of the PR300670 antibody, which is as follows:
KASSLES
SEQ ID NO: 53 shows the amino acid sequence of the third framework region of the light chain (LFR3) of the PR300670 antibody, which is as follows:
GVPSRFSGSGSGTEFTLTISSPQPDDFATYYC
SEQ ID NO: 54 shows the amino acid sequence of the third complementarity determining region of the light chain (LCDR3) of the PR300670 antibody, which is as follows:
QQYHSYSYT
SEQ ID NO: 55 shows the amino acid sequence of the fourth framework region of the light chain (LFR4) of the PR300670 antibody, which is as follows:
FGQGTKLEIK
SEQ ID NO: 56 shows the amino acid sequence of the heavy chain (H) of the PR300679 antibody, which is as follows:

QVQLQESGPGLVKPSGTLSLTCAVSGGSISSSNWWSWVRQPPGKGLEWIGEIYHSGSTNYNPSLKSRVTIS
VDKSKNQFSLKLSSVTAADTAVYYCAREYSSSWLSVMDAWGQGASVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLDSDGSFFLYSKLTVDKSR
WQQGNVFSCSVMHEALHNHYTQKSLSLSPGK

SEQ ID NO: 57 shows the amino acid sequence of the heavy chain variable region (VH) of the PR300679 antibody, which is as follows:

QVQLQESGPGLVKPSGTLSLTCAVSGGSISSSNWWSWVRQPPGKGLEWIGEIYHSGSTNYNPSLKSRVTIS
VDKSKNQFSLKLSSVTAADTAVYYCAREYSSSWLSVMDAWGQGASVTVSS

SEQ ID NO: 58 shows the amino acid sequence of the first framework region of the heavy chain (HFR1) of the PR300679 antibody, which is as follows:
QVQLQESGPGLVKPSGTLSLTCAVS
SEQ ID NO: 59 shows the amino acid sequence of the first complementarity determining region of the heavy chain (HCDR1) of the PR300679 antibody, which is as follows:
GGSISSSNWWS
SEQ ID NO: 60 shows the amino acid sequence of the second framework region of the heavy chain (HFR2) of the PR300679 antibody, which is as follows:
WVRQPPGKGLEWIG
SEQ ID NO: 61 shows the amino acid sequence of the second complementarity determining region of the heavy chain (HCDR2) of the PR300679 antibody, which is as follows:
EIYHSGSTNYNPSLKS
SEQ ID NO: 62 shows the amino acid sequence of the third framework region of the heavy chain (HFR3) of the PR300679 antibody, which is as follows:
RVTISVDKSKNQFSLKLSSVTAADTAVYYCAR
SEQ ID NO: 63 shows the amino acid sequence of the third complementarity determining region of the heavy chain (HCDR3) of the PR300679 antibody, which is as follows:
EYSSSWLSVMDA
SEQ ID NO: 64 shows the amino acid sequence of the fourth framework region of the heavy chain (HFR4) of the PR300679 antibody, which is as follows:
WGQGASVTVSS
SEQ ID NO: 65 shows the amino acid sequence of the light chain (L) of the PR300679 antibody, which is as follows:

EIVLTQSPGTLSLSPGERATLSCRASQSVSSSYLAWYQQKPGQAPRLLIYGASSRATGIPDRFSGSGSGTDF
TLTISRLEPEDFAVYYCQQYGSSPRTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGE
C

SEQ ID NO: 66 shows the amino acid sequence of the light chain variable region (VL) of the PR300679 antibody, which is as follows:

EIVLTQSPGTLSLSPGERATLSCRASQSVSSSYLAWYQQKPGQAPRLLIYGASSRATGIPDRFSGSGSGTDF
TLTISRLEPEDFAVYYCQQYGSSPRTFGQGTKVEIK

SEQ ID NO: 67 shows the amino acid sequence of the first framework region of the light chain (LFR1) of the PR300679 antibody, which is as follows:
EIVLTQSPGTLSLSPGERATLSC
SEQ ID NO: 68 shows the amino acid sequence of the first complementarity determining region of the light chain (LCDR1) of the PR300679 antibody, which is as follows:

RASQSVSSSYLA

SEQ ID NO: 69 shows the amino acid sequence of the second framework region of the light chain (LFR2) of the PR300679 antibody, which is as follows:

WYQQKPGQAPRLLIY

SEQ ID NO: 70 shows the amino acid sequence of the second complementarity determining region of the light chain (LCDR2) of the PR300679 antibody, which is as follows:

GASSRAT

SEQ ID NO: 71 shows the amino acid sequence of the third framework region of the light chain (LFR3) of the PR300679 antibody, which is as follows:

GIPDRFSGSGSGTDFTLTISRLEPEDFAVYYC

SEQ ID NO: 72 shows the amino acid sequence of the third complementarity determining region of the light chain (LCDR3) of the PR300679 antibody, which is as follows:

QQYGSSPRT

SEQ ID NO: 73 shows the amino acid sequence of the fourth framework region of the light chain (LFR4) of the PR300679 antibody, which is as follows:

FGQGTKVEIK

SEQ ID NO: 74 shows the amino acid sequence of the heavy chain (H) of the PR301285 antibody, which is as follows:

QVQLQESGPGLVKPSETLSLTCTVSGGSMISYYWSWLRQPPGKGLEWIGYIYYSGNTNYNPSLKSRITIAV
DTSKNQFSLKLNSVTAADTAVYYCARERELHPYYFDYWGQGTLVTVSSASTKGPSVFPLAPSSKSTSGGT
AALGCLVKDYFPEPVTVSWNSGALTSGVHTFPAVLQSSGLYSLSSVVTVPSSSLGTQTYICNVNHKPSNTK
VDKKVEPKSCDKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSFFLYSKLTVDKSRWQQ
GNVFSCSVMHEALHNHYTQKSLSLSPGK

SEQ ID NO: 75 shows the amino acid sequence of the heavy chain variable region (VH) of the PR301285 antibody, which is as follows:

QVQLQESGPGLVKPSETLSLTCTVSGGSMISYYWSWLRQPPGKGLEWIGYIYYSGNTNYNPSLKSRITIAV
DTSKNQFSLKLNSVTAADTAVYYCARERELHPYYFDYWGQGTLVTVSS

SEQ ID NO: 76 shows the amino acid sequence of the first framework region of the heavy chain (HFR1) of the PR301285 antibody, which is as follows:

QVQLQESGPGLVKPSETLSLTCTVS

SEQ ID NO: 77 shows the amino acid sequence of the first complementarity determining region of the heavy chain (HCDR1) of the PR301285 antibody, which is as follows:

GGSMISYYWS

SEQ ID NO: 78 shows the amino acid sequence of the second framework region of the heavy chain (HFR2) of the PR301285 antibody, which is as follows:

WLRQPPGKGLEWIG

SEQ ID NO: 79 shows the amino acid sequence of the second complementarity determining region of the heavy chain (HCDR2) of the PR301285 antibody, which is as follows:

YIYYSGNTNYNPSLKS

SEQ ID NO: 80 shows the amino acid sequence of the third framework region of the heavy chain (HFR3) of the PR301285 antibody, which is as follows:

RITIAVDTSKNQFSLKLNSVTAADTAVYYCAR

SEQ ID NO: 81 shows the amino acid sequence of the third complementarity determining region of the heavy chain (HCDR3) of the PR301285 antibody, which is as follows:

ERELHPYYFDY

SEQ ID NO: 82 shows the amino acid sequence of the fourth framework region of the heavy chain (HFR4) of the PR301285 antibody, which is as follows:

WGQGTLVTVSS

SEQ ID NO: 83 shows the amino acid sequence of the light chain (L) of the PR301285 antibody, which is as follows:

DIQLTQSPSFLSASVGDRVTITCRASQGIDNYLAWYQQKPGKAPKVLIYAASTLQSGVPSRFSGSGSGTEFT
LTISSLQPEDFATYYCQQLNSYPLTFGGGTKVEIKRTVAAPSVFIFPPSDEQLKSGTASVVCLLNNFYPREAK
VQWKVDNALQSGNSQESVTEQDSKDSTYSLSSTLTLSKADYEKHKVYACEVTHQGLSSPVTKSFNRGEC

SEQ ID NO: 84 shows the amino acid sequence of the light chain variable region (VL) of the PR301285 antibody, which is as follows:

DIQLTQSPSFLSASVGDRVTITCRASQGIDNYLAWYQQKPGKAPKVLIYAASTLQSGVPSRFSGSGSGTEFT
LTISSLQPEDFATYYCQQLNSYPLTFGGGTKVEIK

SEQ ID NO: 85 shows the amino acid sequence of the first framework region of the light chain (LFR1) of the PR301285 antibody, which is as follows:
DIQLTQSPSFLSASVGDRVTITC
SEQ ID NO: 86 shows the amino acid sequence of the first complementarity determining region of the light chain (LCDR1) of the PR301285 antibody, which is as follows:
RASQGIDNYLA
SEQ ID NO: 87 shows the amino acid sequence of the second framework region of the light chain (LFR2) of the PR301285 antibody, which is as follows:
WYQQKPGKAPKVLIY
SEQ ID NO: 88 shows the amino acid sequence of the second complementarity determining region of the light chain (LCDR2) of the PR301285 antibody, which is as follows:
AASTLQS
SEQ ID NO: 89 shows the amino acid sequence of the third framework region of the light chain (LFR3) of the PR301285 antibody, which is as follows:
GVPSRFSGSGSGTEFTLTISSLQPEDFATYYC
SEQ ID NO: 90 shows the amino acid sequence of the third complementarity determining region of the light chain (LCDR3) of the PR301285 antibody, which is as follows:
QQLNSYPLT
SEQ ID NO: 91 shows the amino acid sequence of the fourth framework region of the light chain (LFR4) of the PR301285 antibody, which is as follows:
FGGGTKVEIK
SEQ ID NO: 92 shows the amino acid sequence of the heavy chain (H) of the PR301286 antibody, which is as follows:

QVQLVESGGGVVQPGKSLRLSCAASGFTFSSYGMHWVRQAPGKGLEWVAVIWIDGSNRYYAASVKGRF
TISRDNSKNTLYLQMNSLRAEDTAVYYCTRGYGDYAYVMDVWGQGASVTVSSASTKGPSVFPLAPSSKS
TSGGTAALGCLVKDYFPEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGSFFLYSKLTVDKS
RWQQGNVFSCSVMHEALHNHYTQKSLSLSPGK

SEQ ID NO: 93 shows the amino acid sequence of the heavy chain variable region (VH) of the PR301286 antibody, which is as follows:

QVQLVESGGGVVQPGKSLRLSCAASGFTFSSYGMHWVRQAPGKGLEWVAVIWIDGSNRYYAASVKGRF
TISRDNSKNTLYLQMNSLRAEDTAVYYCTRGYGDYAYVMDVWGQGASVTVSS

SEQ ID NO: 94 shows the amino acid sequence of the first framework region of the heavy chain (HFR1) of the PR301286 antibody, which is as follows:
QVQLVESGGGVVQPGKSLRLSCAAS
SEQ ID NO: 95 shows the amino acid sequence of the first complementarity determining region of the heavy chain (HCDR1) of the PR301286 antibody, which is as follows:
GFTFSSYGMH

SEQ ID NO: 96 shows the amino acid sequence of the second framework region of the heavy chain (HFR2) of the PR301286 antibody, which is as follows:

WVRQAPGKGLEWVA

SEQ ID NO: 97 shows the amino acid sequence of the second complementarity determining region of the heavy chain (HCDR2) of the PR301286 antibody, which is as follows:

VIWIDGSNRYYAASVKG

SEQ ID NO: 98 shows the amino acid sequence of the third framework region of the heavy chain (HFR3) of the PR301286 antibody, which is as follows:

RFTISRDNSKNTLYLQMNSLRAEDTAVYYCTR

SEQ ID NO: 99 shows the amino acid sequence of the third complementarity determining region of the heavy chain (HCDR3) of the PR301286 antibody, which is as follows:

GYGDYAYVMDV

SEQ ID NO: 100 shows the amino acid sequence of the fourth framework region of the heavy chain (HFR4) of the PR301286 antibody, which is as follows:

WGQGASVTVSS

SEQ ID NO: 101 shows the amino acid sequence of the light chain (L) of the PR301286 antibody, which is as follows:

DIVMTQSPLSLPVTPGEPASISCRSSQSLLPGNGYNYLDWYLQKPGQSPQLLIYLGSNRASGVPDRFSGSG
SGTDFTLKISRVEAEDVGIYYCMQALQSPLTFGGGTKVEIKRTVAAPSVFIFPPSDEQLKSGTASVVCLLNN
FYPREAKVQWKVDNALQSGNSQESVTEQDSKDSTYSLSSTLTLSKADYEKHKVYACEVTHQGLSSPVTK
SFNRGEC

SEQ ID NO: 102 shows the amino acid sequence of the light chain variable region (VL) of the PR301286 antibody, which is as follows:

DIVMTQSPLSLPVTPGEPASISCRSSQSLLPGNGYNYLDWYLQKPGQSPQLLIYLGSNRASGVPDRFSGSG
SGTDFTLKISRVEAEDVGIYYCMQALQSPLTFGGGTKVEIK

SEQ ID NO: 103 shows the amino acid sequence of the first framework region of the light chain (LFR1) of the PR301286 antibody, which is as follows:

DIVMTQSPLSLPVTPGEPASISC

SEQ ID NO: 104 shows the amino acid sequence of the first complementarity determining region of the light chain (LCDR1) of the PR301286 antibody, which is as follows:

RSSQSLLPGNGYNYLD

SEQ ID NO: 105 shows the amino acid sequence of the second framework region of the light chain (LFR2) of the PR301286 antibody, which is as follows:

WYLQKPGQSPQLLIY

SEQ ID NO: 106 shows the amino acid sequence of the second complementarity determining region of the light chain (LCDR2) of the PR301286 antibody, which is as follows:

LGSNRAS

SEQ ID NO: 107 shows the amino acid sequence of the third framework region of the light chain (LFR3) of the PR301286 antibody, which is as follows:

GVPDRFSGSGSGTDFTLKISRVEAEDVGIYYC

SEQ ID NO: 108 shows the amino acid sequence of the third complementarity determining region of the light chain (LCDR3) of the PR301286 antibody, which is as follows:

MQALQSPLT

SEQ ID NO: 109 shows the amino acid sequence of the fourth framework region of the light chain (LFR4) of the PR301286 antibody, which is as follows:

FGGGTKVEIK

SEQ ID NO: 110 shows the amino acid sequence of the heavy chain (H) of the PR301286-1 antibody, which is as follows (the underlined amino acids are amino acids mutated relative to the PR301286 antibody):

QVQLVESGGGVVQPGKSLRLSCAASGFTFSSYGMHWVRQAPGKGLEWVAVIWID<u>A</u>SNRYYAASVKGRF
TISRDNSKNTLYLQMNSLRAEDTAVYYCTRGYGDYAYVMDVWGQGASVTVSSASTKGPSVFPLAPSSKS
TSGGTAALGCLVKDYFPEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGSFFLYSKLTVDKS
RWQQGNVFSCSVMHEALHNHYTQKSLSLSPGK

SEQ ID NO: 111 shows the amino acid sequence of the heavy chain variable region (VH) of the PR301286-1 antibody, which is as follows (the underlined amino acids are amino acids mutated relative to the PR301286 antibody):

QVQLVESGGGVVQPGKSLRLSCAASGFTFSSYGMHWVRQAPGKGLEWVAVIWID<u>A</u>SNRYYAASVKGRF
TISRDNSKNTLYLQMNSLRAEDTAVYYCTRGYGDYAYVMDVWGQGASVTVSS

SEQ ID NO: 112 shows the amino acid sequence of the second complementarity determining region of the heavy chain (HCDR2) of the PR301286-1 antibody, which is as follows (the underlined amino acids are amino acids mutated relative to the PR301286 antibody):
VIWIDASNRYYAASVKG
SEQ ID NO: 113 shows the amino acid sequence of the heavy chain (H) of the PR301286-3 antibody, which is as follows (the underlined amino acids are amino acids mutated relative to the PR301286 antibody):

QVQLVESGGGVVQPGKSLRLSCAASGFTFSSYGMHWVRQAPGKGLEWVAVIWID<u>V</u>SNRYYAASVKGRF
TISRDNSKNTLYLQMNSLRAEDTAVYYCTRGYGDYAYVMDVWGQGASVTVSSASTKGPSVFPLAPSSKS
TSGGTAALGCLVKDYFPEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGSFFLYSKLTVDKS
RWQQGNVFSCSVMHEALHNHYTQKSLSLSPGK

SEQ ID NO: 114 shows the amino acid sequence of the heavy chain variable region (VH) of the PR301286-3 antibody, which is as follows (the underlined amino acids are amino acids mutated relative to the PR301286 antibody):

QVQLVESGGGVVQPGKSLRLSCAASGFTFSSYGMHWVRQAPGKGLEWVAVIWID<u>V</u>SNRYYAASVKGRF
TISRDNSKNTLYLQMNSLRAEDTAVYYCTRGYGDYAYVMDVWGQGASVTVSS

SEQ ID NO: 115 shows the amino acid sequence of the second complementarity determining region of the heavy chain (HCDR2) of the PR301286-3 antibody, which is as follows (the underlined amino acids are amino acids mutated relative to the PR301286 antibody):
VIWIDVSNRYYAASVKG
SEQ ID NO: 116 shows the amino acid sequence of the heavy chain (H) of the PR301286-5, PR301286-6, and PR301286-7 antibodies, which is as follows (the underlined amino acids are amino acids mutated relative to the PR301286 antibody):

QVQLVESGGGVVQPGKSLRLSCAASGFTFSSYGMHWVRQAPGKGLEWVAVIWID<u>A</u>SNRYYAASVKGRF
TISRDNSKNTLYLQMNSLRAEDTAVYYCTRGYGDYAYVMDVWGQGASVTVSSASTKGPSVFPLAPSSKS
TSGGTAALGCLVKDYFPEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGSFFLYSKLTVDKS
RWQQGNVFSCSVMHEALHNHYTQKSLSLSPGK

SEQ ID NO: 117 shows the amino acid sequence of the heavy chain cariable region (VH) of the PR301286-5, PR301286-6, and PR301286-7 antibodies, which is as follows (the underlined amino acids are amino acids mutated relative to the PR301286 antibody);

QVQLVESGGGVVQPGKSLRLSCAASGFTFSSYGMHWVRQAPGKGLEWVAVIWID<u>A</u>SNRYYAASVKGRF TISRDNSKNTLYLQMNSLRAEDTAVYYCTRGYGDYAYVMDVWGQGASVTVSS

SEQ ID NO: 118 shows the amino acid sequence of the second complementarity determining region of the heavy chain (HCDR2) of the PR301286-5, PR301286-6, and PR301286-7 antibodies, which is as follows (the underlined amino acids are amino acids mutated relative to the PR301286 antibody):
VIWIDASNRYYAASVKG
SEQ ID NO: 119 shows the amino acid sequence of the light chain (L) of the PR301286-5 antibody, which is as follows (the underlined amino acids are amino acids mutated relative to the PR301286 antibody):

DIVMTQSPLSLPVTPGEPASISCRSSQSLLPG<u>S</u>GYNYLDWYLQKPGQSPQLLIYLGSNRASGVPDRFSGSGS GTDFTLKISRVEAEDVGIYYCMQALQSPLTFGGGTKVEIKRTVAAPSVFIFPPSDEQLKSGTASVVCLLNNF YPREAKVQWKVDNALQSGNSQESVTEQDSKDSTYSLSSTLTLSKADYEKHKVYACEVTHQGLSSPVTKS FNRGEC

SEQ ID NO: 120 shows the amino acid sequence of the light chain variable region (VL) of the PR301286-5 antibody, which is as follows (the underlined amino acids are amino acids mutated relative to the PR301286 antibody):

DIVMTQSPLSLPVTPGEPASISCRSSQSLLPG<u>S</u>GYNYLDWYLQKPGQSPQLLIYLGSNRASGVPDRFSGSGS

GTDFTLKISRVEAEDVGIYYCMQALQSPLTFGGGTKVEIK

SEQ ID NO: 121 shows the amino acid sequence of the first complementarity determining region of the light chain (LCDR1) of the PR301286-5 antibody, which is as follows (the underlined amino acids are amino acids mutated relative to the PR301286 antibody):
RSSQSLLPGSGYNYLD
SEQ ID NO: 122 shows the amino acid sequence of the light chain (L) of the PR301286-6 antibody, which is as follows (the underlined amino acids are amino acids mutated relative to the PR301286 antibody):

DIVMTQSPLSLPVTPGEPASISCRSSQSLLPGN<u>A</u>YNYLDWYLQKPGQSPQLLIYLGSNRASGVPDRFSGSGS GTDFTLKISRVEAEDVGIYYCMQALQSPLTFGGGTKVEIKRTVAAPSVFIFPPSDEQLKSGTASVVCLLNNF YPREAKVQWKVDNALQSGNSQESVTEQDSKDSTYSLSSTLTLSKADYEKHKVYACEVTHQGLSSPVTKS FNRGEC

SEQ ID NO: 123 shows the amino acid sequence of the light chain variable region (VL) of the PR301286-6 antibody, which is as follows (the underlined amino acids are amino acids mutated relative to the PR301286 antibody):

DIVMTQSPLSLPVTPGEPASISCRSSQSLLPGN<u>A</u>YNYLDWYLQKPGQSPQLLIYLGSNRASGVPDRFSGSGS GTDFTLKISRVEAEDVGIYYCMQALQSPLTFGGGTKVEIK

SEQ ID NO: 124 shows the amino acid sequence of the first complementarity determining region of the light chain (LCDR1) of the PR301286-6 antibody, which is as follows (the underlined amino acids are amino acids mutated relative to the PR301286 antibody):
RSSQSLLPGNAYNYLD
SEQ ID NO: 125 shows the amino acid sequence of the light chain (L) of the PR301286-7 antibody, which is as follows (the underlined amino acids are amino acids mutated relative to the PR301286 antibody):

DIVMTQSPLSLPVTPGEPASISCRSSQSLLPGNTYNYLDWYLQKPGQSPQLLIYLGSNRASGVPDRFSGSGS
GTDFTLKISRVEAEDVGIYYCMQALQSPLTFGGGTKVEIKRTVAAPSVFIFPPSDEQLKSGTASVVCLLNNF
YPREAKVQWKVDNALQSGNSQESVTEQDSKDSTYSLSSTLTLSKADYEKHKVYACEVTHQGLSSPVTKS
FNRGEC

SEQ ID NO: 126 shows the amino acid sequence of the light chain variable region (VL) of the PR301286-7 antibody, which is as follows (the underlined amino acids are amino acids mutated relative to the PR301286 antibody):

DIVMTQSPLSLPVTPGEPASISCRSSQSLLPGNTYNYLDWYLQKPGQSPQLLIYLGSNRASGVPDRFSGSGS
GTDFTLKISRVEAEDVGIYYCMQALQSPLTFGGGTKVEIK

SEQ ID NO: 127 shows the amino acid sequence of the first complementarity determining region of the light chain (LCDR1) of the PR301286-7 antibody, which is as follows (the underlined amino acids are amino acids mutated relative to the PR301286 antibody):
RSSQSLLPGNTYNYLD
SEQ ID NO: 128 shows the amino acid sequence of the heavy chain (H) of the PR301289 antibody, which is as follows:

QVQLVQSGAEVKKPGASVKVSCRASGYTFTSYDINWVRQATGQGLEWMGWMNPHSGDTGYAQNFQGR
VTMTWNTSISTAYMELSSLRSEDTAVYYCARRRVGVYYYGMDVWGQGTTVTVSSASTKGPSVFPLAPSS
KSTSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDSDGSFFLYSKLTVD
KSRWQQGNVFSCSVMHEALHNHYTQKSLSLSPGK

SEQ ID NO: 129 shows the amino acid sequence of the heavy chain variable region (VH) of the PR301289 antibody, which is as follows:

QVQLVQSGAEVKKPGASVKVSCRASGYTFTSYDINWVRQATGQGLEWMGWMNPHSGDTGYAQNFQGR
VTMTWNTSISTAYMELSSLRSEDTAVYYCARRRVGVYYYGMDVWGQGTTVTVSS

SEQ ID NO: 130 shows the amino acid sequence of the first framework region of the heavy chain (HFR1) of the PR301289 antibody, which is as follows:
QVQLVQSGAEVKKPGASVKVSCRAS
SEQ ID NO: 131 shows the amino acid sequence of the first complementarity determining region of the heavy chain (HCDR1) of the PR301289 antibody, which is as follows:
GYTFTSYDIN
SEQ ID NO: 132 shows the amino acid sequence of the second framework region of the heavy chain (HFR2) of the PR301289 antibody, which is as follows:
WVRQATGQGLEWMG
SEQ ID NO: 133 shows the amino acid sequence of the second complementarity determining region of the heavy chain (HCDR2) of the PR301289 antibody, which is as follows:
WMNPHSGDTGYAQNFQG
SEQ ID NO: 134 shows the amino acid sequence of the third framework region of the heavy chain (HFR3) of the PR301289 antibody, which is as follows:
RVTMTWNTSISTAYMELSSLRSEDTAVYYCAR
SEQ ID NO: 135 shows the amino acid sequence of the third complementarity determining region of the heavy chain (HCDR3) of the PR301289 antibody, which is as follows:
RRVGVYYYGMDV
SEQ ID NO: 136 shows the amino acid sequence of the fourth framework region of the heavy chain (HFR4) of the PR301289 antibody, which is as follows:
WGQGTTVTVSS
SEQ ID NO: 137 shows the amino acid sequence of the light chain (L) of the PR301289 antibody, which is as follows:

ENVLTQSPGTLSLSPGERATLSCRASQSVSSSYFGWYQQKPGQAPRLLIYGASSRATGIPDRFSGSGSGTDF
TLTISRLEPEDFAVYYCQQYGSSPITFGQGTRLEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGE
C

SEQ ID NO: 138 shows the amino acid sequence of the light chain variable region (VL) of the PR301289 antibody, which is as follows:

ENVLTQSPGTLSLSPGERATLSCRASQSVSSSYFGWYQQKPGQAPRLLIYGASSRATGIPDRFSGSGSGTDF
TLTISRLEPEDFAVYYCQQYGSSPITFGQGTRLEIK

SEQ ID NO: 139 shows the amino acid sequence of the first framework region of the light chain (LFR1) of the PR301289 antibody, which is as follows:
ENVLTQSPGTLSLSPGERATLSC
SEQ ID NO: 140 shows the amino acid sequence of the first complementarity determining region of the light chain (LCDR1) of the PR301289 antibody, which is as follows:
RASQSVSSSYFG
SEQ ID NO: 141 shows the amino acid sequence of the second framework region of the light chain (LFR2) of the PR301289 antibody, which is as follows:
WYQQKPGQAPRLLIY
SEQ ID NO: 142 shows the amino acid sequence of the second complementarity determining region of the light chain (LCDR2) of the PR301289 antibody, which is as follows:
GASSRAT
SEQ ID NO: 143 shows the amino acid sequence of the third framework region of the light chain (LFR3) of the PR301289 antibody, which is as follows:
GIPDRFSGSGSGTDFTLTISRLEPEDFAVYYC
SEQ ID NO: 144 shows the amino acid sequence of the third complementarity determining region of the light chain (LCDR3) of the PR301289 antibody, which is as follows:
QQYGSSPIT
SEQ ID NO: 145 shows the amino acid sequence of the fourth framework region of the light chain (LFR4) of the PR301289 antibody, which is as follows:
FGQGTRLEIK
SEQ ID NO: 146 shows the amino acid sequence of the heavy chain (H) of the PR301289-1, which is as follows the underlined amino acids are amino acids mutated relative to the PR301289 antibody):

QVQLVQSGAEVKKPGASVKVSCRASGYTFTSYDINWVRQATGQGLEWMGWMNPHSGDTGYAQNFQGR
VTMTWQTSISTAYMELSSLRSEDTAVYYCARRRVGVYYYGMDVWGQGTTVTVSSASTKGPSVFPLAPSS
KSTSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDSDGSFFLYSKLTVD
KSRWQQGNVFSCSVMHEALHNHYTQKSLSLSPGK

SEQ ID NO: 147 shows the amino acid sequence of the heavy chain variable region (VH) of the PR301289-1 antibody, which is as follows (the underlined amino acids are amino acids mutated relative to the PR301289 antibody):

QVQLVQSGAEVKKPGASVKVSCRASGYTFTSYDINWVRQATGQGLEWMGWMNPHSGDTGYAQNFQGR
VTMTWQTSISTAYMELSSLRSEDTAVYYCARRRVGVYYYGMDVWGQGTTVTVSS

SEQ ID NO: 148 shows the amino acid sequence of the heavy chain (H) of the PR301289-2, which is as follows (the underlined amino acids are amino acids mutated relative to the PR301289 antibody):

QVQLVQSGAEVKKPGASVKVSCRASGYTFTSYDINWVRQATGQGLEWMGWMNPHSGDTGYAQNFQGR
VTMTWQTSISTAYMELSSLRSEDTAVYYCARRRVGVYYHGMDVWGQGTTVTVSSASTKGPSVFPLAPSS
KSTSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAPIEKTISKAKGQ
PREPQVYTLPPSREEMTKNQVSLTCLVKGFYPSDIAVEWESNGQPENNYKTTPPVLDSDGSFFLYSKLTVD
KSRWQQGNVFSCSVMHEALHNHYTQKSLSLSPGK

SEQ ID NO: 149 shows the amino acid sequence of the heavy chain variable region (VH) of the PR301289-2 antibody, which is as follows (the underlined amino acids are amino acids mutated relative to the PR301289 antibody):

QVQLVQSGAEVKKPGASVKVSCRASGYTFTSYDINWVRQATGQGLEWMGWMNPHSGDTGYAQNFQGR
VTMTWQTSISTAYMELSSLRSEDTAVYYCARRRVGVYYHGMDVWGQGTTVTVSS

SEQ ID NO: 150 shows the amino acid sequence of the third framework region of the heavy chain (HFR3) of the PR301289-1 and PR301289-2 antibodies, which is as follows (the underlined amino acids are amino acids mutated relative to the PR301289 antibody):
RVTMTWQTSISTAYMELSSLRSEDTAVYYCAR
SEQ ID NO: 151 shows the amino acid sequence of the third complementarity determining region of the heavy chain (HCDR3) of the PR301289-2 antibody, which is as follows (the underlined amino acids are amino acids mutated relative to the PR301289 antibody):
RRVGVYYHGMDV
SEQ ID NO: 152 shows the amino acid sequence of the heavy chain (H) of the PR300066 (Tab) antibody, which is as follows:

QVQLQQPGAELVKPGASVKMSCKASGYTFTSYWITWVIQRPGQGLEWIGDIYCGSDTMHYNEKFKNKA
TLTVDTSSSTAYMQLSSLTSEDSAVYYCARWWDYGSSYDYFDYWGQGTTLTVSSASTKGPSVFPLAPSSK
STSGGTAALGCLVKDYFPEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLDSDGSFFLYSKLTVDK
SRWQQGNVFSCSVMHEALHNHYTQKSLSLSPGK

SEQ ID NO: 153 shows the amino acid sequence of the light chain (L) of the PR300066 (Tab) antibody, which is as follows:

DIKMTQSPSSMYASLGERVTITCKASQDINSYLSWFQQKPGKSPKTLIYRANRLVDGVPSRFSGSGSGQDY
SLTISSLEYEDMGIYYCLQYDEFPYTFGGGTKLEIKRTVAAPSVFIFPPSDEQLKSGTASVVCLLNNFYPRE
AKVQWKVDNALQSGNSQESVTEQDSKDSTYSLSSTLTLSKADYEKHKVYACEVTHQGLSSPVTKSFNRG
EC

SEQ ID NO: 154 shows the amino acid sequence of the recombinant protein human Siglec-15 ECD-hFc, which is as follows:

FVRTKIDTTENLLNTEVHSSPAQRWSMQVPPEVSAEAGDAAVLPCTFTHPHRHYDGPLTAIWRAGEPYAG
PQVFRCAAARGSELCQTALSLHGRFRLLGNPRRNDLSLRVERLALADDRRYFCRVEFAGDVHDRYESRH
GVRLHVTAAPRIVNISVLPSPAHAFRALCTAEGEPPPALAWSGPALGNSLAAVRSPREGHGHLVTAELPALT
HDGRYTCTAANSLGRSEASVYLFRFHGASGASTIEGRMDEPKSCDKTHTCPPCPAPELLGGPSVFLFPPKP
KDTLMISRTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVKGFYPSDIAVEWESNGQ
PENNYKTTPPVLDSDGSFFLYSKLTVDKSRWQQGNVFSCSVMHEALHNHYTQKSLSLSPGK

SEQ ID NO: 155 shows the amino acid sequence of cynomolgus monkey Siglec-15, which is as follows:

MESSIRLLACLACVLPTGSFVRTKIDTTENLLNTEVHSSPAQRWSMQVPAEVSAAAGDAAVLPCTFTHPH
RHYDGPLTAIWRAGEPYAGPQVFRCAAARGSELCQTALSLHGRFRLLGNPRRNDLSLRVERLALADDRR
YFCRVEFAGDVHDRYESRHGVRLHVTAAPRIINISVLPGPAHAFRALCTAEGEPPPALAWSGPALGNGSAA
VPSSGQGHGHLVTAELPALNHDGRYTCTAANSLGRSEASVYLFRFHGASGASTVALLLGALGLKALLLLG
VLAAGVARRRPEHLNTPDTPPRSQAQESNYENLSQMNPRSPPAAMCSP

SEQ ID NO: 156 shows the amino acid sequence of OS8, which is as follows:

QVQLQQSGAELARPGASVKMSCKASGYTFTRYTMHWVKQRPGQGLEWIGYINPSRGYTNYNQKFKDK
ATLTTDKSSSTAYMQLSSLTSEDSAVYYCARYYDDHYCLDYWGQGTTLTVSSGGGGSGGGGSGGGGSQI
VLTQSPAIMSASPGEKVTMTCSASSSVSYMNWYQQKSGTSPKRWIYDTSKLASGVPAHFRGSGSGTSYSL
TISGMEAEDAATYYCQQWSSNPFTFGSGTKLEIN

Example 1. Immunization of Fully Human-Derived H2L2 Transgenic Mice and Screening and Identification of Fully Human-Derived Antibodies

1.1 Immunization of fully human-derived H2L2 transgenic mice

Three immunogens were used, i.e., human Siglec-15 ECD-hFc protein, 293T/Siglec-15 cell line, and Siglec-15 DNA. The three immunogens described above were used to immunize HARBOUR H2L2 transgenic mice (see WO 2010/070263A1, which is incorporated herein by reference) individually to generate anti-human Siglec-15 ECD antibodies.

(1) Immunization of H2L2 mice with human Siglec-15 ECD-hFc protein: the recombinant human Siglec-15 ECD-hFc protein (SEQ ID NO: 154) was used to immunize HARBOUR H2L2 transgenic mice aged 6-8 weeks, and the mice were raised in a specific pathogen free (SPF) environment. For the primary immunization, 50 μg of the immunoprotein was mixed with complete Freund's adjuvant (CFA, Sigma, Cat#: F5881), and the mixture was injected into the abdominal cavity and the axillary lymph node and the inguinal lymph node. To boost the immune response, two weeks after the primary immunization, 25 μg of the immunogenic protein was mixed with the Ribi (Sigma adjuvant system, Sigma, Cat#: S6322) adjuvant, and the mixture was injected into the abdominal cavity and subcutaneous lymph nodes of each mouse, followed by the injection of 25 μg of the immunogen every two weeks (the adjuvant was also Ribi for each time) for a total of 6 times including the primary immunization. One week after each booster immunization, mouse blood was collected and centrifuged to obtain the plasma, and the plasma was diluted to 6 concentrations (1:100, 1:1000, 1:3000, 1:9000, 1:27000, and 1:81000). The titer of the anti-human Siglec-15 antibody in the mouse blood was determined by ELISA on an ELISA plate coated with human Siglec-15 ECD-his protein (Acrobiosystems, Cat#: SG5-H52H3), and the specific reactivity of the mouse plasma at 2 concentrations (1:100 and 1:1000) for CHO-K1/human Siglec-15 cells (KYinno, Beijing) and CHO-K1 blast cells with high expression of Siglec-15 (the amino acid sequence of which is set forth in SEQ ID NO: 1) was assayed by flow cytometry. Plasma of mice before immunization was used as a blank control group (PB).

(2) Immunization of H2L2 mice with 293T/Siglec-15 cell line: cDNAs encoding human Siglec-15 (SEQ ID NO: 1) were obtained by gene synthesis and cloned into the expression vector plenti6.3, and then the expression vector was transfected into 293T cells to construct the stable cell line 293T/Siglec-15 with high expression of human Siglec-15. The immunogen, the 293T/Siglec-15 cell line, was used to immunize HARBOUR H2L2 transgenic mice aged 6-8 weeks, and the mice were raised in a specific pathogen free (SPF) environment. For the primary immunization, 5-10 $\times$ 10$^6$ 293T/Siglec-15 cells were injected into the abdominal cavity of each mouse. To boost the immune response, two weeks after the primary immunization, 5-10 $\times$ 10$^6$ 293T/Siglec-15 cells were injected into the abdominal cavity of each mouse, followed by the injection of 5-10 $\times$ 10$^6$ 293T/Siglec-15 cells every three weeks, for a total of 6 times including the primary immunization. One week after each booster immunization, mouse blood was collected and centrifuged to obtain the plasma, and the plasma was diluted to 6 concentrations (1:100, 1:1000, 1:3000, 1:9000, 1:27000, and 1:81000). The titer of the anti-human Siglec-15 antibody in the mouse blood was determined by ELISA on an ELISA plate coated with human Siglec-15 ECD-his protein (Acrobiosystems, Cat#: SG5-H52H3), and the specific reactivity of the mouse plasma at 2 concentrations (1:100 and 1:1000) for CHO-K1/human Siglec-15 cells (KYinno, Beijing) and CHO-K1 blast cells with high expression of Siglec-15 was assayed by flow cytometry. Plasma of mice before immunization was used as a blank control group (PB).

(3) Immunization of H2L2 mice with Siglec-15 DNA: cDNAs encoding human Siglec-15 (SEQ ID NO: 1) were obtained by gene synthesis and cloned into the expression vector pVAX1 to construct human Siglec-15 DNA (pVAX1-hSiglec-15). The immunogen, the pVAX1-hSiglec-15, was used to immunize HARBOUR H2L2 transgenic mice aged 6-8 weeks, and the mice were raised in a specific pathogen free (SPF) environment. For the primary immunization, 4 $\mu$g pVAX1-hSiglec-15 was injected into the abdominal cavity of each mouse by using a gene gun. To boost the immune response, two weeks after the primary immunization, 4 $\mu$g of pVAX1-hSiglec-15 was injected into the abdominal cavity of each mouse, followed by the injection of 4 $\mu$g of pVAX1-hSiglec-15 every three weeks, for a total of 6 times including the primary immunization. One week after each booster immunization, mouse blood was collected and centrifuged to obtain the plasma, and the plasma was diluted to 6 concentrations (1:100, 1:1000, 1:3000, 1:9000, 1:27000, and 1:81000). The titer of the anti-human Siglec-15 antibody in the mouse blood was determined by ELISA on an ELISA plate coated with human Siglec-15 ECD-his protein (Acrobiosystems, Cat#: SG5-H52H3), and the specific reactivity of the mouse plasma at 2 concentrations (1:100 and 1:1000) for CHO-K1/human Siglec-15 cells (KYinno, Beijing) and CHO-K1 blast cells with high expression of Siglec-15 was assayed by flow cytometry. Plasma of mice before immunization was used as a blank control group (PB).

After completion of the above steps, mice with high antibody titer and a specific immune response to human Siglec-15 were selected for fusion. Before the fusion, 25 $\mu$g of purified human Siglec-15 ECD-hFc protein or 5-10 $\times$ 10$^6$ 293T/Siglec-15 cells or 4 $\mu$g of pVAX1-hSiglec-15 was injected for booster immunization. Three days later, the mice were sacrificed, and their spleen cells and lymph node cells were collected.

1.2 Screening of fully human-derived antibodies

(1) Single B-cell screening based on Beacon® Optofluidic system

Beacon® Optofluidic system uses opto-electronic positioning (OEP™) technology for the movement of single cells. The Beacon optoelectronic system is an automatic biological instrument that allows various operations such as biological function tests, experimental analysis, positive clone selection, and the like to be simultaneously performed under the cell culture conditions. The Beacon platform can perform these tasks in a massively parallel and automatic manner for thousands of cells.

In the present application, a plasma cell discovery workflow was used. Up to 14,000 single plasma cells can be screened per experiment for selecting antigen-specific plasma cells secreting anti-Siglec-15 antibodies. Those plasma cells secreting specific antibodies were then individually introduced into a 96-well plate containing a cell lysis buffer for subsequent single B-cell sequencing to identify the heavy and light chain sequences of the antibodies produced by the single B-cells (monoclones).

(2) Screening based on hybridoma technology

5-10 mL of red blood cell lysis buffer was added to the spleen cell and lymph node samples, and the lysis was performed at 4°C for 5 min and then stopped using 1-10 volumes of 37°C 10% FBS DMEM. The supernatant was discarded, and the cells were resuspended in an appropriate amount of DMEM medium, and the survival rate and number of cells were determined. Mouse myeloma cells sp2/0 (ATCC, Cat #: CRL-1581) were counted and kept at 37°C for later use. The spleen cells were then fused with the mouse myeloma cells sp2/0 (ATCC, Cat #: CRL-15 81) at a ratio of 4:1 to 10:1 in cell number by efficient electrofusion.

The fused cells were resuspended in a medium (DMEM) containing 20% FBS (Biological Industries, Cat#: 04-002-1A) supplemented with 1$\times$ hypoxanthine, aminopterin, and thymidine (50$\times$ HAT supplement, Cat#: 21060017, life technologies), and the cell concentration was adjusted to 10$^5$ cells/200 $\mu$L. The fused cells were added to a 96-well

plate at 200 μL/well and cultured at 37°C with 5% $CO_2$. On day 10 after cell fusion, the hybridoma supernatant was assayed for its ability to bind to human Siglec-15 ECD-his protein (Acrobiosystems, Cat#: SG5-H52H3) by enzyme-linked immunosorbent assay (ELISA), and

the positive clones selected ($OD_{450}$ > 1.0) were then screened by flow cytometry for clones specifically binding to CHO-K1/human Siglec-15 cells (KYinno, Beijing). After fluorescence intensity scoring, 30 hybridoma parent clones with the strongest fluorescence intensity were selected and subcloned by the limiting dilution method. The grown submonoclones were screened by ELISA and flow cytometry to find the submonoclones exhibiting the strongest binding to both human Siglec-15 ECD-his protein (Acrobiosystems, Cat#: SG5-H52H3) and CHO-K1/human Siglec-15 cell (KYinno, Beijing). The submonoclones identified as IgG subtype by the rat Ig isotyping kit (Invitrogen, Cat#: 88-50640-88) were to be subjected to sequencing analysis.

The fully human-derived antibodies and sequences thereof obtained by the above method of the present disclosure are shown in Table 2.

Table 2. Corresponding sequence numbers of antibodies (SEQ ID NO: X)

| Antibody | H | VH | HCDR1 | HCDR2 | HCDR3 | L | VL | LCDR1 | LCDR2 | LCDR3 |
|---|---|---|---|---|---|---|---|---|---|---|
| PR300526 | 2 | 3 | 5 | 7 | 9 | 11 | 12 | 14 | 16 | 18 |
| PR300659 | 20 | 21 | 23 | 25 | 27 | 29 | 30 | 32 | 34 | 36 |
| PR300670 | 38 | 39 | 41 | 43 | 45 | 47 | 48 | 50 | 52 | 54 |
| PR300679 | 56 | 57 | 59 | 61 | 63 | 65 | 66 | 68 | 70 | 72 |
| PR301285 | 74 | 75 | 77 | 79 | 81 | 83 | 84 | 86 | 88 | 90 |
| PR301286 | 92 | 93 | 95 | 97 | 99 | 101 | 102 | 104 | 106 | 108 |
| PR301289 | 128 | 129 | 131 | 133 | 135 | 137 | 138 | 140 | 142 | 144 |

Example 2. Expression and Purification of Anti-Siglec-15 Antibodies

The heavy chain variable region sequence of the anti-Siglec-15 antibody was subcloned into a pTT5 expression vector containing a signal peptide and the human heavy chain IgG1 constant region. The light chain variable region sequence of the anti-Siglec-15 antibody was subcloned into an expression vector containing a signal peptide and the human antibody light chain kappa constant region. The recombinant plasmids were confirmed by sequencing and extracted with a Maxi kit (Macherey-Nagel, NucleoBond® Xtra Midi) to improve the purity and quality of the recombinant plasmids, which were then filtered through a 0.22 μm filter (millpore). The purified plasmids were used for transfection.

Transfection was performed after the density of Freestyle 293F cells (Thermo/Gibco, R79007) was adjusted to 1-1.5 × $10^6$/mL. The heavy and light chain plasmids were co-transfected into the Freestyle 293F cells via PEI (polyethylenimine, Polysciences, Cat#: 24885), and the transfected cells were cultured under conditions of 37°C, 125 rpm, and 8% carbon dioxide. After about 6-7 days of culturing, the cell viability decreased to no more than 70%, AmMag™ Protein A Magnetic Beads (GenScript, Cat#: L00695-4) were added to the cell culture, and the mixture was incubated at 37°C for 1-4 h. Magnetic Beads were collected with AmMag™ MR magnetic rack (GenScript, Cat#: L00722) and the cell culture medium was removed. The Magnetic Beads were washed with PBS that was 10-20 times the volume of the Magnetic Beads, and the procedure was repeated 3-5 times. The Magnetic Beads were collected and eluted with 0.1 M Glycine-HCl (pH 2.8) that was 2-3 times the volume of the Magnetic Beads and the eluate containing the anti-Siglec-15 antibody was collected, and the procedure was repeated 3-5 times. Then, 1 M Tris-HCl (pH 8.0) was added for neutralization, and the neutralized eluate was added to a dialysis bag (Thermo, Cat#: 68100) and dialyzed in 1× PBS for 8-16 h. After dialysis, the concentration of the anti-Siglec-15 antibody was determined using a NanoPhotometer (IMPLEN, Cat#: NP80), the purity of the antibody was determined using SDS-PAGE or high performance liquid chromatography-mass spectrometry, and the endotoxin content was determined using an endotoxin assay kit (GenScript, Cat#: L00350).

Example 3. Sequence Analysis and Variant Acquisition of Antibodies

The sequence of the heavy chain variable domain of the antibody is derived from events such as gene rearrangements of germline gene V, D, and J segments of heavy chain gene clusters and somatic hypermutations on chromosomes; the sequence of the light chain variable domain is derived from the events such as gene rearrangements of germline gene V, D, and J segments of light chain gene clusters and somatic hypermutations. Gene rearrangement and somatic hypermutation are major factors in increasing antibody diversity. Antibodies derived from the same germline V gene segment may also produce different sequences, but with relatively high similarity overall. The germline gene segments that are likely to undergo gene rearrangement can be deduced from the antibody variable region sequences using algorithms such as IMGT/DomainGapAlign (http://imgt.org/3Dstructure-DB/cgi/DomainGapAlign.cgi) or NCBI/Ig-BLAST (https://www.ncbi.nlm.nih.gov/igblast/). The antibody sequences in Example 1 and Table 2 were analyzed and germline gene V segments for the heavy chain variable domain (VH) and light chain variable domain (VL) are listed in Table 3.

Chemical modifications sometimes introduced after translation and synthesis of protein or polypeptide amino acid chains in cells are known as post-translational modifications (PTMs). For antibodies, some PTM sites are very conservative. For example, the conservative amino acid asparagine (Asn) at position 297 (EU numbering) of the constant domain of the human IgG1 antibody is often glycosylated to form a saccharide chain whose structure is critical for antibody structure and associated effector functions. However, PTMs may have great effect on antigen binding or result in changes in the physicochemical properties of an antibody if they are present in the variable regions, particularly in the antigen-binding regions (e.g., CDRs) of the antibody. For example, glycosylation, deamidation, isomerization, oxidation, and the like may increase the instability or heterogeneity of antibody molecules, thereby increasing the difficulty and risk of antibody development. Thus, it is very important to avoid some potential PTMs in the development of therapeutic antibodies. As experience has accumulated, it has been found that some PTMs are highly correlated with the composition of amino acid sequences, especially the "pattern" of the composition of adjacent amino acids, which makes it possible to predict potential PTMs from the primary amino acid sequences of a protein. For example, an N-linked glycosylation site can be predicted from the sequence pattern N-x-S/T (asparagine at the first position, any amino acid except for non-proline at the second position, and serine or threonine at the third position). The amino acid sequence patterns leading to PTMs may be derived from germline gene sequences, e.g., the human germline gene fragment IGHV3-33 naturally having a glycosylation pattern NST in the FR3 region, or they may also be derived from somatic hypermutations. The predicted PTMs for the variable domains VH and VL of the antibodies of Example 1 are listed in Table 3. Specifically, NSS or NLT may be a glycosylation site, and NS, NT, or NN may be a deamidation site.

The amino acid sequence patterns of PTMs may be disrupted by amino acid mutations, thereby reducing or eliminating the formation of specific PTMs. There are different methods for designing mutations depending on the antibody sequences and PTM sequence patterns. One method is to replace a "hot spot" amino acid (e.g., N or S in the NS pattern) with an amino acid with similar physicochemical properties (e.g., to mutate N into Q). If the PTM sequence pattern is derived from somatic hypermutations and is not present in the germline gene sequence, the other method can be to replace the sequence pattern with the corresponding germline gene sequence. In practice, a variety of methods for designing mutations may be used for the same PTM sequence pattern.

New antibody molecules (referred to as PTM variants) resulting from amino acid mutations to the sequences of two antibodies with potential PTM sites from Example 1 are listed in Table 4. The amino acid sequences of the light and heavy chain variable domains, the full-length amino acid sequence of the light chain, the full-length amino acid sequence of the heavy chain (human IgG1), and the amino acid sequences of the CDRs defined according to the Combined definition scheme of those PTM variants in this example are listed in Table 5. All designed PTM variants were purified by the method described in Example 2 to obtain the purified recombinant antibodies, which were subjected to functional verification.

Table 3. Germline gene analysis and post-translational modification site (PTM) analysis of sequences of anti-siglec-15 antibodies

| Antibody | VH germline V gene | VL germline V gene | VH PTM | VL PTM |
|---|---|---|---|---|
| PR301286 | VH3_30*01 | Vk2_28*01 | DG (HCDR2) | NG (LCDR1) |
| PR301289 | VH1_8*01 | Vk3_20*01 | NxS/T (HFR3) | / |

Table 4. Mutation site design for anti-siglec-15 antibody sequences

| Initial antibody | PTM variant | Mutation site | VH PTMs of variant molecule | VL PTMs of variant molecule |
|---|---|---|---|---|
| PR301286 | PR301286-1 | VH(HCDR2):G/A | / | NG (LCDR1) |
| | PR301286-3 | VH(HCDR2):G/V | / | NG (LCDR1) |
| | PR301286-5 | VH(HCDR2):G/A; VL(LCDR1):N/S | / | / |
| | PR301286-6 | VH(HCDR2):G/A; VL(LCDR1):G/A | / | / |
| | PR301286-7 | VH(HCDR2):G/A; VL(LCDR1):G/T | / | / |
| PR301289 | PR301289-1 | HFR3:N/Q | / | / |
| | PR301289-2 | HFR3:N/Q; CDRH3: YYY/YYH | / | / |

Table 5. Sequence numbers corresponding to antibodies obtained after mutation of PTMs (SEQ ID NO: X)

| PTM variant | H | VH | HCDR1 | HCDR2 | HCDR3 | L | VL | LCDR1 | LCDR2 | LCDR3 |
|---|---|---|---|---|---|---|---|---|---|---|
| PR301286-1 | 110 | 111 | 95 | 112 | 99 | 101 | 102 | 104 | 106 | 108 |
| PR301286-3 | 113 | 114 | 95 | 115 | 99 | 101 | 102 | 104 | 106 | 108 |
| PR301286-5 | 116 | 117 | 95 | 118 | 99 | 119 | 120 | 121 | 106 | 108 |
| PR301286-6 | 116 | 117 | 95 | 118 | 99 | 122 | 123 | 124 | 106 | 108 |
| PR301286-7 | 116 | 117 | 95 | 118 | 99 | 125 | 126 | 127 | 106 | 108 |
| PR301289-1 | 146 | 147 | 131 | 133 | 135 | 137 | 138 | 140 | 142 | 144 |
| PR301289-2 | 148 | 149 | 131 | 133 | 151 | 137 | 138 | 140 | 142 | 144 |

Example 4. Binding of Recombinant Antibodies to Human Siglec-15 Protein

1 $\mu$g/mL human Siglec-15 ECD-his protein (Acrobiosystems, Cat#: SG5-H52H3) dissolved in PBS was added to an ELISA plate at 100 $\mu$L/well for coating and the plate was incubated overnight at 4°C. The ELISA plate was then blocked with PBST (PBS + 0.05% Tween 20) containing 2% BSA at 200 $\mu$L/well and incubated at 37°C for 1 h. The ELISA plate was washed 4 times with PBST. The anti-Siglec-15 antibody diluted in a gradient (5-fold dilution, starting concentration: 6.67 nM) was added to the ELISA plate at 100 $\mu$L/well and the plate was incubated at 37°C for 1 h. The ELISA plate was washed 4 times with PBST. The goat anti-human IgG (H+L)-HRP (1:5000, Jackson, Cat#: 109-035-088) was added at 100 $\mu$L/well and the plate was incubated at 37°C for 1 h. The ELISA plate was washed 4 times with PBST. The TMB substrate (Biopanda, Cat#: TMB-S-003) was added at 100 $\mu$L/well, and the plate was incubated at room temperature for 5 min, and then the stop buffer (Solarbio, Cat#: C1058) was added at 100 $\mu$L/well to stop the reaction. $OD_{450\ nm}$ readings were recorded using a microplate reader (Molecular Devices, Spectra max 384 plus).

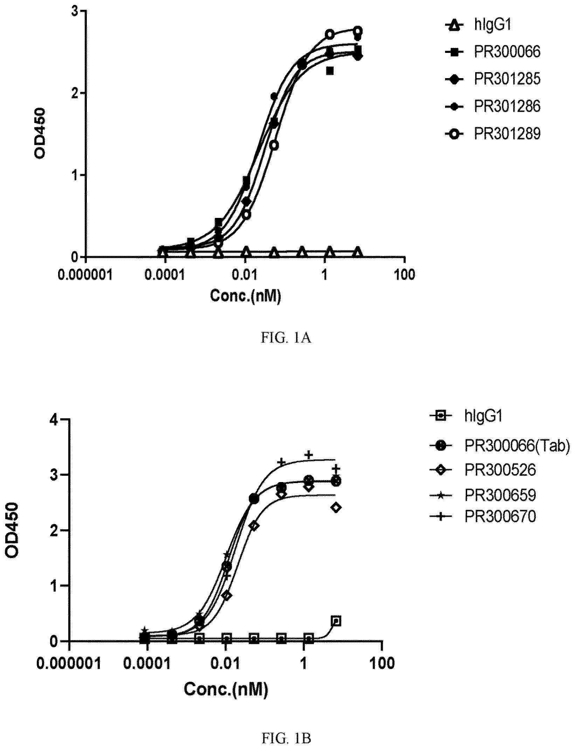

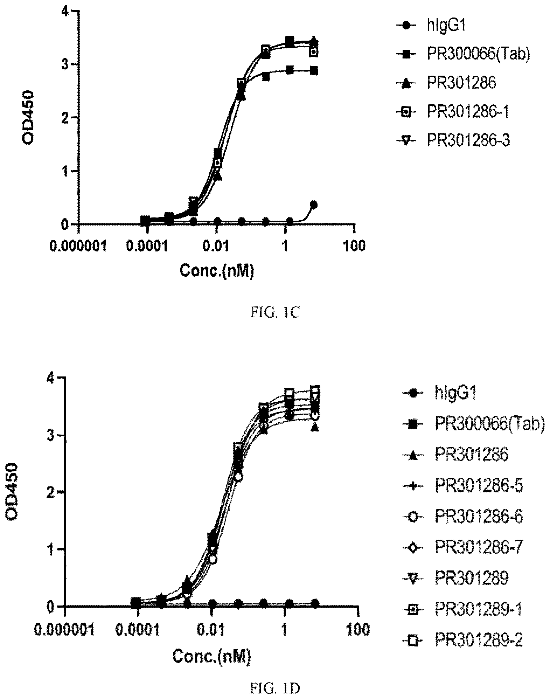

As shown in FIGs. 1A-1D and Table 6 (Tables 6A-6D), the initial antibodies and variants thereof obtained by the present disclosure were all able to specifically bind to human Siglec-15 protein, and the detected binding ability of the antibodies increased in a positive correlation with the antibody concentration. Compared with the reference antibody PR300066 Tab (the reference antibody variable region was from clone 5G12 of WO 2018/057735A1), the 6 anti-Siglec-15 antibodies disclosed herein (PR301285, PR301286, PR301289, PR300526, PR300659, and PR300670) showed binding activity comparable to that of the reference antibody (PR300066). Engineered mutants of PR301286 and PR301289 showed comparable or stronger binding activity compared with the parent antibodies.

Table 6. Binding of recombinant antibodies to human Siglec-15 protein

Table 6A

| Antibody | EC50 (nM) |
|---|---|
| hIgG1 | - |
| PR300066(Tab) | 0.022 |
| PR301285 | 0.031 |
| PR301286 | 0.022 |
| PR301289 | 0.058 |

Table 6B

| Antibody | EC50 (nM) |
|---|---|
| hIgG1 | - |
| PR300066(Tab) | 0.012 |
| PR300526 | 0.020 |
| PR300659 | 0.010 |
| PR300670 | 0.018 |

Table 6C

| Antibody | EC50 (nM) |
|---|---|
| hIgG1 | - |
| PR300066(Tab) | 0.012 |
| PR301286 | 0.027 |
| PR301286-1 | 0.019 |
| PR301286-3 | 0.019 |

Table 6D

| Antibody | EC50 (nM) |
|---|---|
| hIgG1 | - |
| PR300066(Tab) | 0.021 |
| PR301286 | 0.018 |
| PR301286-5 | 0.020 |
| PR301286-6 | 0.029 |
| PR301286-7 | 0.025 |
| PR301289 | 0.027 |
| PR301289-1 | 0.020 |
| PR301289-2 | 0.029 |

Example 5. Binding of Recombinant Antibodies to Cynomolgus Monkey Siglec-15 Protein

1 μg/mL cynomolgus monkey Siglec-15-his protein (Acrobiosystems, Cat#: SG5-C52H6) dissolved in PBS was added to an ELISA plate at 100 μL/well for coating and the plate was incubated overnight at 4°C. The ELISA plate was then blocked with PBST (PBS + 0.05% Tween 20) containing 2% BSA at 200 μL/well and incubated at 37°C for 1 h. The ELISA plate was washed 4 times with PBST. The anti-Siglec-15 antibody diluted in a gradient (5-fold dilution, starting concentration: 6.67 nM) was added to the ELISA plate at 100 μL/well and the plate was incubated at 37°C for 1 h. The ELISA plate was washed 4 times with PBST. The goat anti-human IgG (H+L)-HRP (1:5000, Jackson, Cat#: 109-035-088) was added at 100 μL/well and the plate was incubated at 37°C for 1 h. The ELISA plate was washed 4 times with PBST. The TMB substrate (Biopanda, Cat#: TMB-S-003) was added at 100 μL/well, and the plate was incubated at room temperature for 5 min, and then the stop buffer (Solarbio, Cat#: C1058) was added at 100 μL/well to stop the reaction. $OD_{450\,nm}$ readings were recorded using a microplate reader (Molecular Devices, Spectra max 384 plus).

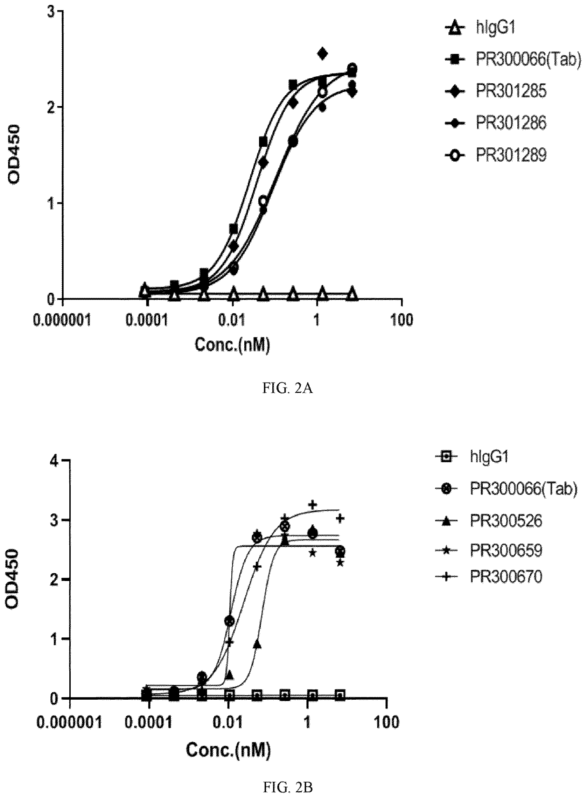

As shown in FIGs. 2A-2B and Table 7 (Tables 7A-7B), the PR301285, PR301286, PR301289, PR300526, PR300659, and PR300670 disclosed herein were all able to cross-bind to cynomolgus monkey Siglec-15 protein, and compared with the reference antibody PR300066 (Tab), i.e., 5G12, the 6 anti-Siglec-15 antibodies disclosed herein showed binding activity comparable to that of the reference antibody.

Table 7. Binding of recombinant antibodies to cynomolgus monkey Siglec-15 protein

Table 7A

| Antibody | EC50 (nM) |
|---|---|
| hIgG1 | - |
| PR300066(Tab) | 0.025 |
| PR301285 | 0.038 |
| PR301286 | 0.090 |
| PR301289 | 0.104 |

Table 7B

| Antibody | EC50 (nM) |
|---|---|
| hIgG1 | - |
| PR300066(Tab) | 0.012 |
| PR300526 | 0.071 |
| PR300659 | ~0.011 |
| PR300670 | 0.025 |

Example 6. Binding of Recombinant Antibodies to Mouse Siglec-15

1 μg/mL mouse Siglec-15-his protein (Acrobiosystems, Cat#: SG5-M52H7) dissolved in PBS was added to an ELISA plate at 100 μL/well for coating and the plate was incubated overnight at 4°C. The ELISA plate was then blocked with PBST (PBS + 0.05% Tween 20) containing 2% BSA at 200 μL/well and incubated at 37°C for 1 h. The ELISA plate was washed 4 times with PBST. The anti-Siglec-15 antibody diluted in a gradient (5-fold dilution, starting concentration: 6.67 nM) was added to the ELISA plate at 100 μL/well and the plate was incubated at 37°C for 1 h. The ELISA plate was washed 4 times with PBST. The goat anti-human IgG (H+L)-HRP (1:5000, Jackson, Cat: 109-035-088) was added at 100 μL/well and the plate was incubated at 37°C for 1 h. The ELISA plate was washed 4 times with PBST. The TMB substrate (Biopanda, Cat: TMB-S-003) was added at 100 μL/well, and the plate was incubated at room temperature for 5 min, and then the stop buffer (Solarbio, Cat#: C1058) was added at 100 μL/well to stop the reaction. $OD_{450\,nm}$ readings were recorded using a microplate reader (Molecular Devices, Spectra max 384 plus).

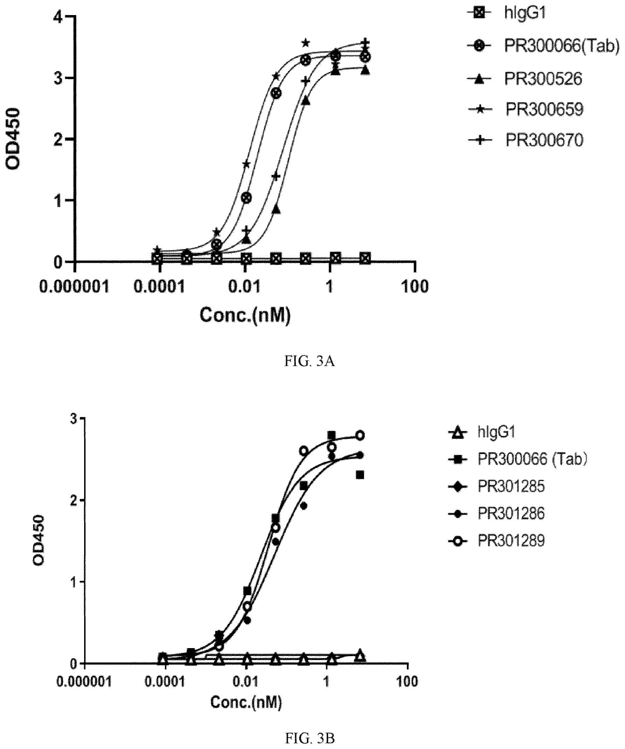

As shown in FIGs. 3A-3B and Table 8 (Tables 8A-8B), the 5 antibodies PR300526, PR300659, PR300670, PR301286, and PR301289 disclosed herein were all able to cross-bind to mouse Siglec-15 protein, and compared with the reference antibody PR300066 (Tab), i.e., 5G12, the 5 anti-Siglec-15 antibodies disclosed herein showed binding activity comparable to that of the reference antibody. However, PR301285 was not able to cross-bind to mouse Siglec-15 protein.

Table 8. Binding of recombinant antibodies to mouse Siglec-15 protein

EP 4 438 055 A1

Table 8A

| Antibody | EC50 (nM) |
|---|---|
| hIgG1 | - |
| PR300066(Tab) | 0.019 |
| PR300526 | 0.104 |
| PR300659 | 0.013 |
| PR300670 | 0.079 |

Table 8B

| Antibody | EC50 (nM) |
|---|---|
| hIgG1 | - |
| PR300066(Tab) | 0.023 |
| PR301285 | - |
| PR301286 | 0.051 |
| PR301289 | 0.036 |

Example 7. Binding Ability of Recombinant Anti-Siglec-15 Antibodies for CHO-K1/Human Siglec-15

CHO-K1/human Siglec-15 cells (KYinno, Beijing) were adjusted to a density of $1 \times 10^6$/mL with PBS, and the cell suspension was added to a 96-well V-bottom plate at 100 $\mu$L/well. The 96-well V-bottom plate was centrifuged at 1000 rpm for 5 min and washed once with PBS + 2% FBS. The cells were resuspended in 100 $\mu$L of antibody diluted in a gradient (5-fold dilution, starting concentration: 500 nM) and incubated at 4°C for 1 h, and PR300066 and hIgGI were used as positive control and negative control, respectively. After 1 h, the cells were washed twice with PBS + 2% FBS. The cells were incubated with 100 $\mu$L of Alexa Fluor 488 goat anti-human IgG (Jackson, Cat#: 109-545-098) at 4°C for 1 h in the dark and then washed twice with PBS + 2% FBS. The cells were then resuspended in 200 $\mu$L of PBS + 2% FBS. The fluorescence intensity of the cells was analyzed using a BD FACSCanto™ II flow cytometer.

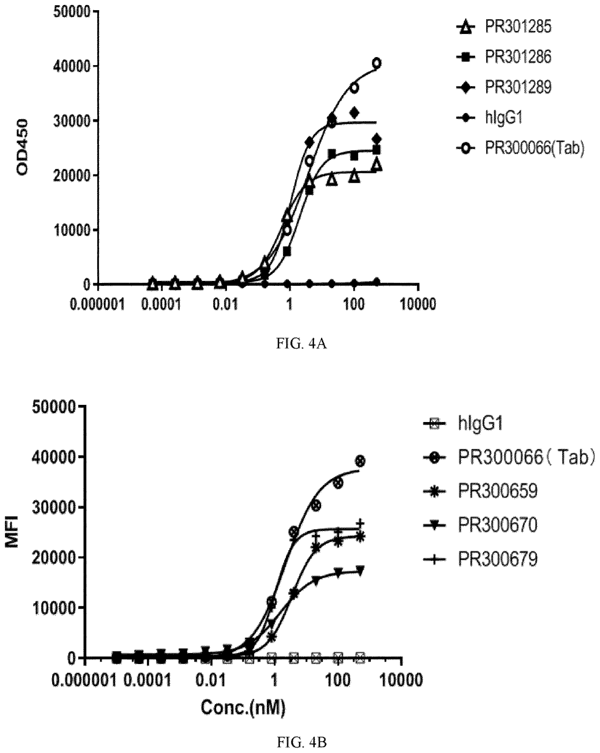

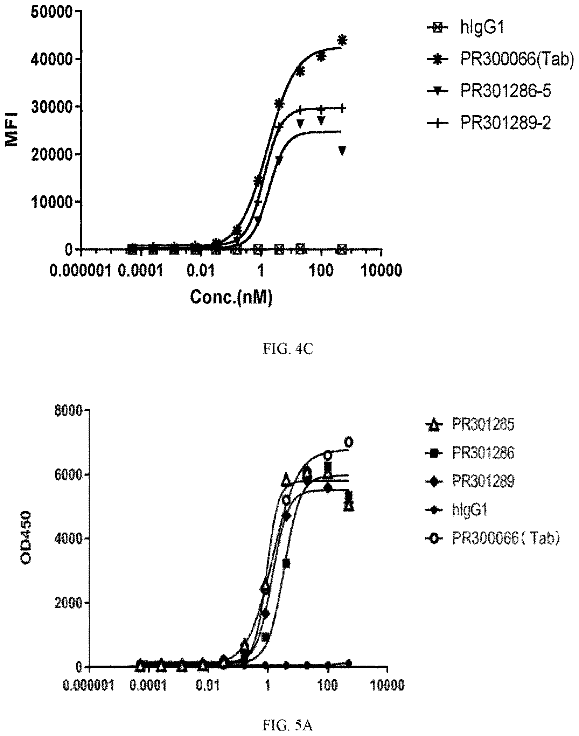

As shown in FIGs. 4A-4C and Table 9 (Tables 9A-9C), at the cellular level, the antibodies PR301285, PR301286, PR301289, PR300659, PR300670 and PR300679 and the engineered mutants PR301286-5 and PR301289-2 of PR301286 and PR301289 disclosed herein were able to bind to human Siglec-15, wherein except for PR300659 and PR301286-5, the $EC_{50}$ of the other 6 antibodies for human Siglec-15 was less than the $EC_{50}$ of the reference antibody PR300066 (Tab), i.e., 5G12, indicating that these antibodies were able to bind to Siglec-15 more sensitively at a lower concentration.

Table 9. Binding of recombinant antibodies to CHO-K1/human Siglec-15

Table 9A

| Antibody | EC50 (nM) | Maximum (MFI) |
|---|---|---|
| hIgG1 | - | - |
| PR300066(Tab) | 3.657 | 40910 |
| PR301285 | 0.5528 | 20327 |
| PR301286 | 1.984 | 24204 |
| PR301289 | 1.039 | 29231 |

Table 9B

| Antibody | EC50 (nM) | Maximum (MFI) |
|---|---|---|
| hIgG1 | - | - |

(continued)

| Antibody | EC50 (nM) | Maximum (MFI) |
|---|---|---|
| PR300066(Tab) | 2.197 | 37867 |
| PR300659 | 3.447 | 23970 |
| PR300670 | 1.487 | 16631 |
| PR300679 | 1.033 | 25330 |

Table 9C

| Antibody | EC50 (nM) | Maximum (MFI) |
|---|---|---|
| hIgG1 | - | - |
| PR300066(Tab) | 1.638 | 42520 |
| PR301286-5 | 1.815 | 24396 |
| PR301289-2 | 1.264 | 28759 |

Example 8. Binding of Recombinant Antibodies to CHO-K1/Cynomolgus Monkey Siglec-15

cDNAs encoding cynomolgus monkey Siglec-15 (the amino acid sequence of which is set forth in SEQ ID NO: 155) were obtained by gene synthesis and cloned into the expression vector pcDNA3.1, and then the expression vector was transfected into CHO-K1 cells to construct CHO-K1/cynomolgus monkey Siglec-15 cells expressing cynomolgus monkey Siglec-15. CHO-K1/cynomolgus monkey Siglec-15 cells were adjusted to a density of $1 \times 10^6$/mL with PBS, and the cell suspension was added to a 96-well V-bottom plate at 100 μL/well. The 96-well V-bottom plate was centrifuged at 1000 rpm for 5 min and washed once with PBS + 2% FBS. The cells were resuspended in 100 μL of antibody diluted in a gradient (5-fold dilution, starting concentration: 500 nM) and incubated at 4°C for 1 h, and PR300066 and hIgG1 were used as positive control and negative control, respectively. After 1 h, the cells were washed twice with PBS + 2% FBS. The cells were incubated with 100 μL of Alexa Fluor 488 goat anti-human IgG (Jackson, Cat#: 109-545-098) at 4°C for 1 h in the dark and then washed twice with PBS + 2% FBS. The cells were then resuspended in 200 μL of PBS + 2% FBS. The fluorescence intensity of the cells was analyzed using a BD FACS Canto™ II flow cytometer.

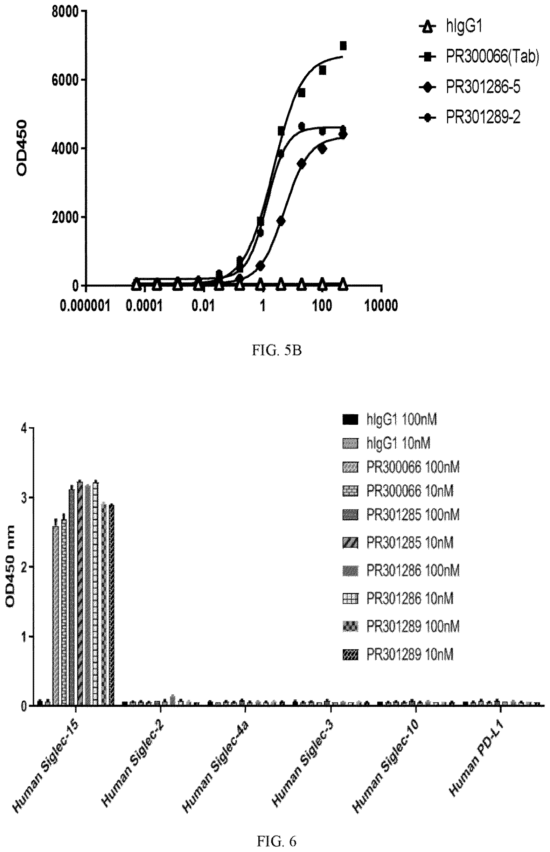

As shown in FIGs. 5A-5B and Table 10 (Tables 10A-10B), at the cellular level, the antibodies PR301285, PR301286, and PR301289 and the engineered mutants PR301286-5 and PR301289-2 of PR301286 and PR301289 disclosed herein were able to bind to cynomolgus monkey Siglec-15, wherein the $EC_{50}$ of PR301285 and PR301289-2 was less than the $EC_{50}$ of the reference antibody PR300066 (Tab), i.e., 5G12, indicating that PR301285 and PR301289-2 were able to bind to cynomolgus monkey Siglec-15 more sensitively at a lower concentration. However, PR001286 and PR301286-5 showed a slightly weaker binding for the cynomolgus monkey Siglec-15.

Table 10. Binding of recombinant antibodies to CHO-K1/cynomolgus monkey Siglec-15

Table 10A

| Antibody | EC50 (nM) | Maximum (MFI) |
|---|---|---|
| hIgG1 | - | - |
| PR300066(Tab) | 1.495 | 6596 |
| PR301285 | 0.889 | 5656 |
| PR301286 | 3.386 | 5862 |
| PR301289 | 1.363 | 5419 |

Table 10B

| Antibody | EC50 (nM) | Maximum (MFI) |
|---|---|---|
| hIgG1 | - | - |

(continued)

| Antibody | EC50 (nM) | Maximum (MFI) |
|---|---|---|
| PR300066(Tab) | 2.192 | 6678 |
| PR301286-5 | 5.258 | 4280 |
| PR301289-2 | 1.342 | 4420 |

Example 9. Determination of Binding Affinity and Dissociation Constant of Anti-Siglec-15 Antibodies for Recombinant Human Siglec-15 Protein

Affinity was determined using an Octet RED96 instrument (ForteBio) and an anti-human IgG Fc capture sensor (AHC biosensor, ForteBio, Cat#: 18-5060) according to the detailed procedures and methods provided by the manufacturer. Specifically, candidate antibodies were each diluted to a final concentration of 6 μg/mL and directly immobilized on AHC biosensors for kinetic measurements. The antigenic protein Siglec-15 ECD-his protein (Acrobiosystems, Cat#: SG5-H52H3) was diluted with 10× Kinetics buffer to 3 concentrations of 60 nM, 30 nM, and 15 nM separately and injected as the analyte for 120 s with a dissociation time of 400 s and then subjected to regeneration with 10 mM glycine HCl (pH 1.5) for 15 s. The association rate (kon) and dissociation rate (kdis) were calculated using a simple one-to-one Languir binding model (Octet Red96 data analysis software). The equilibrium dissociation constant (kD) was calculated as the ratio of kdis/kon.

As shown in Table 11 (Tables 11A-11B), PR300659, PR300670, PR301285, PR301286, and PR301286-1 of the antibodies tested had a lower KD value for human Siglec-15 protein than the reference antibody PR300066 (Tab), i.e., 5G12, indicating that they had stronger binding affinity for human Siglec-15. PR300679, PR301289, PR301286-3, PR301286-5, PR301286-6, PR301286-7, PR301289-1, and PR301289-2 have binding affinity for human Siglec-15 protein comparable to that of the reference antibody PR300066 (Tab), i.e., 5G12.

Table 11. Determination of binding affinity and dissociation constant of anti-Siglec-15 antibodies for recombinant human Siglec-15 protein

Table 11A

| Antibody | AHC sensor | | | |
|---|---|---|---|---|
| | $K_D$ (M) | Kon (1/Ms) | Kdis (1/s) | Full R^2 |
| PR300066 (Tab) | 1.30E-10 | 7.30E+05 | 9.50E-05 | 0.9943 |
| PR300659 | 3.05E-12 | 1.07E+06 | 3.25E-06 | 0.9798 |
| PR300670 | 8.58E-11 | 3.61E+05 | 3.09E-05 | 0.9988 |
| PR300679 | 1.06E-10 | 9.68E+05 | 1.02E-04 | 0.9951 |
| PR301285 | <1.0E-12 | 5.29E+05 | <1.0E-07 | 0.9688 |
| PR301286 | <1.0E-12 | 3.68E+05 | <1.0E-07 | 0.9933 |
| PR301289 | 1.73E-10 | 3.73E+05 | 6.47E-05 | 0.9940 |

Table 11B

| Antibody | AHC sensor | | | |
|---|---|---|---|---|
| | $K_D$ (M) | Kon (1/Ms) | Kdis (1/s) | Full R^2 |
| PR300066(Tab) | 2.93E-10 | 6.21E+05 | 1.82E-04 | 0.9991 |
| PR301286 | <1.0E-12 | 2.92E+05 | <1.0E-07 | 0.9989 |
| PR301286-1 | 1.62E-11 | 2.38E+05 | 3.85E-06 | 0.9990 |
| PR301286-3 | 1.95E-10 | 3.30E+05 | 6.44E-05 | 0.9985 |
| PR301286-5 | 1.37E-10 | 3.60E+05 | 4.93E-05 | 0.9989 |
| PR301286-6 | 2.79E-10 | 3.84E+05 | 1.07E-04 | 0.9971 |

(continued)

| Antibody | AHC sensor | | | |
|---|---|---|---|---|
| | $K_D$ (M) | Kon (1/Ms) | Kdis (1/s) | Full R^2 |
| PR301286-7 | 3.61E-10 | 3.79E+05 | 1.37E-04 | 0.9975 |
| PR301289-1 | 1.16E-10 | 3.37E+05 | 3.89E-05 | 0.9986 |
| PR301289-2 | 1.90E-10 | 3.14E+05 | 5.96E-05 | 0.9980 |

Example 10. Cross-Reaction of Recombinant Antibodies with Other Proteins of the Same Class

Human Siglec-15 ECD-his protein (Acrobiosystems, Cat#: SG5-H52H3), human Siglec-2-his protein (Acrobiosystems, Cat#: CD2-H52H8), human Siglec-4a-his protein (Acrobiosystems, Cat#: MAG-H52H8), human Siglec-3-his protein (Acrobiosystems, Cat#: CD3-H5226), human Siglec-10-his protein (Acrobiosystems, Cat#: SI0-H52H9), and human PD-L1B7-H1-his protein (Acrobiosystems, Cat#: PD1-H5229), each at 1 $\mu$g/mL and dissolved in PBS, were each added to an ELISA plate at 100 $\mu$L/well for coating and the plate was incubated overnight at 4°C. The ELISA plate was then blocked with PBST (PBS + 0.05% Tween 20) containing 2% BSA at 200 $\mu$L/well and incubated at 37°C for 1 h. The ELISA plate was washed 4 times with PBST. The anti-Siglec-15 antibody diluted in a gradient (concentrations: 100 nM and 10 nM) was added to the ELISA plate at 100 $\mu$L/well and the plate was incubated at 37°C for 1 h. The ELISA plate was washed 4 times with PBST. The goat anti-human IgG (H+L)-HRP (1:5000, Jackson, Cat#: 109-035-088) was added at 100 $\mu$L/well and the plate was incubated at 37°C for 1 h. The ELISA plate was washed 4 times with PBST. The TMB substrate (Biopanda, Cat#: TMB-S-003) was added at 100 $\mu$L/well, and the plate was incubated at room temperature for 5 min, and then the stop buffer (Solarbio, Cat#: C1058) was added at 100 $\mu$L/well to stop the reaction. $OD_{450\,nm}$ readings were recorded using a microplate reader (Molecular Devices, Spectra max 384 plus).

The results are shown in FIG. 6. The anti-Siglec-15 antibodies (PR301285, PR301286, and PR301289) of the present application did not cross-react with other proteins of the same class, i.e., Siglec-2, Siglec-4a, Siglec-3, Siglec-10, and PD-L1.

Example 11. T Cell Function Assay of Recombinant Antibodies-Secretion of Cytokine IFN-$\gamma$

cDNAs encoding human Siglec-15 (the amino acid sequence of which is set forth in SEQ ID NO: 1) and OS8 (being OKT3 scFv, the amino acid sequence of which is set forth in SEQ ID NO: 156) were obtained by gene synthesis and cloned into the expression vector pcDNA3.1, and then the expression vector was transfected into CHO cells to construct CHO-OS8-hSiglec-15 cells expressing both OS8 and human Siglec-15. CHO-OS8-hSiglec-15 cells were adjusted to a density of $2.5 \times 10^4$/mL with 1640 complete medium, and the cell suspension was added to a 96-well plate at 100 $\mu$L/well. The cells were incubated in an incubator at 37°C with $CO_2$ overnight. Pan T cells were isolated from peripheral blood mononuclear cells (PBMCs) with a human T cell kit (Miltenyi, Cat#: 130-096-535) and adjusted to a cell density of $2 \times 10^6$/mL with 1640 complete medium. The cell suspension was added to the 96-well plate at 50 $\mu$L/well, and the antibody diluted in a gradient (20-fold dilution; initial final concentration: 10 nM) was added to the 96-well plate at 50 $\mu$L/well. After incubation in an incubator at 37°C with $CO_2$ for 72 h, the supernatant was taken and tested for INF-$\gamma$ using the IFN gamma Human Uncoated ELISA Kit (Ebioscience, Cat#: 88-7316-88). PR300066 and hIgG1 were used as references, i.e., as positive control and negative control, respectively.

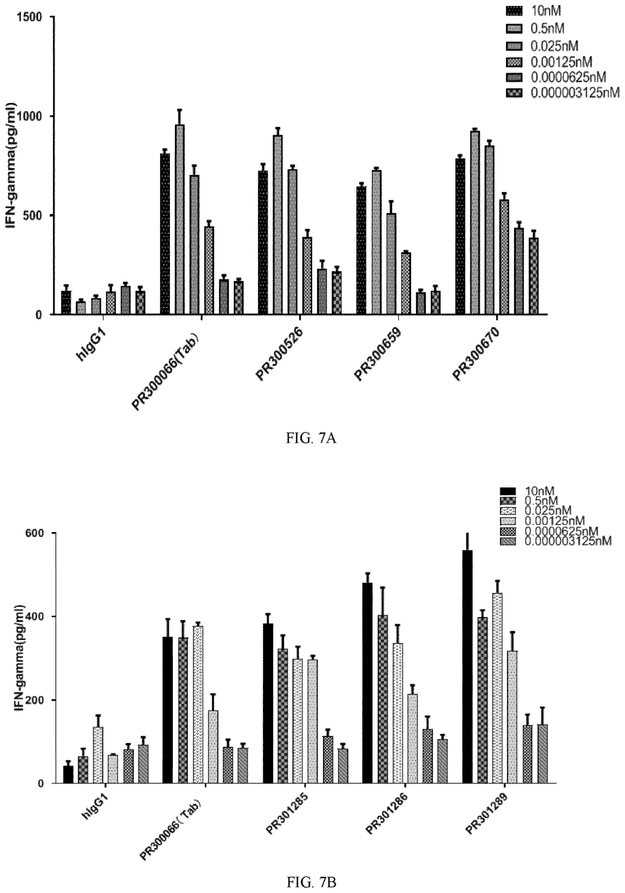

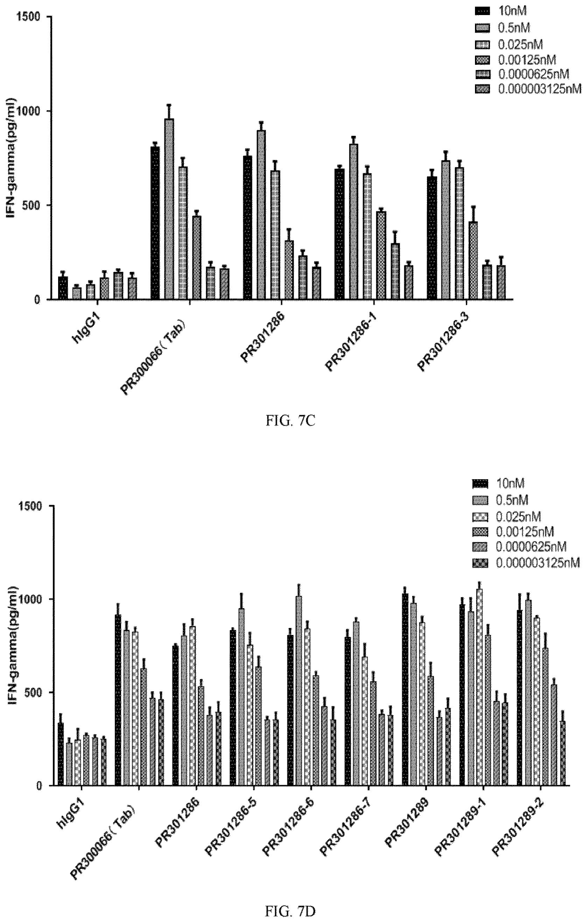

The results are shown in FIGs. 7A-7D. The tested antibodies PR300526, PR300659, PR300670, PR301285, PR3001286, and PR3001289 all dose-dependently enhanced the secretion of cytokine IFN-$\gamma$ from the activated T lymphocytes and had comparable or even stronger activation compared with the positive control. Besides, the engineered mutants of PR301286 and PR301289 also showed activity comparable to that of the parent antibodies.

Example 12. Myeloid Cell Function Assay of Recombinant Antibodies-Cell Proliferation

Monocyte cells were isolated from PBMCs using a human monocyte cell kit (Miltenyi, Cat#: 130-050-201) and adjusted to a cell density of $1 \times 10^6$/mL with 1640 complete medium. The cell suspension was added to a 96-well plate at 100 $\mu$L/well, and the antibody diluted in a gradient (2-fold dilution; final concentration starting at 40 nM) was added to the 96-well plate at 50 $\mu$L/well. Human Siglec-15 ECD-hFc protein (SEQ ID NO: 154) was diluted to 16 $\mu$g/mL and added at 50 $\mu$L/well (final concentration: 4 $\mu$g/mL). After 5 days of incubation in an incubator at 37°C with $CO_2$, the cell proliferation was tested. PR300066 and hIgG1 were used as positive control and negative control, respectively. The number of viable cells was determined by using the CellTiter-Glo® Luminescent Cell Viability Assay (Promega, Cat#:

G7572), and the experimental procedures were performed strictly according to the instructions of the kit. The specific experiment is briefly described as follows: a 96-well plate was equilibrated at room temperature for 30 min before the assay, then the CellTiter-Glo® reagent with the same volume as the cell culture medium was added, and the plate was incubated in a shaker for 10 min to induce the cell lysis and then left to stand at room temperature for 10 min to stabilize the luminescent signals, and finally, the values were read with a plate reader (EnVision, PerkinElmer).

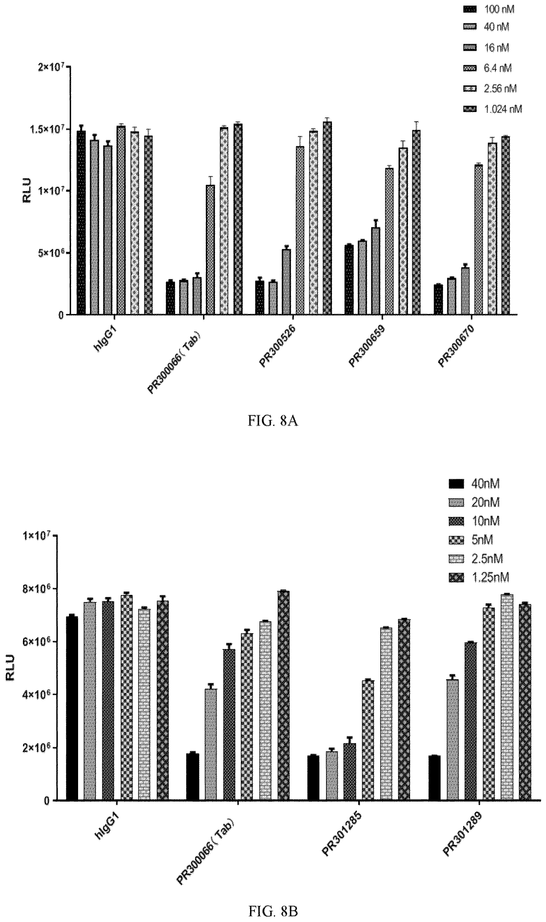

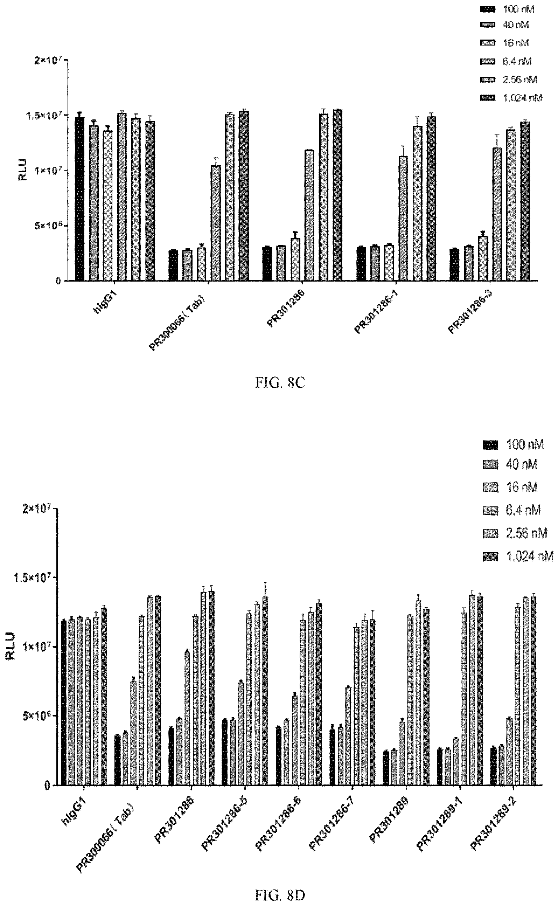

The results are shown in FIGs. 8A-8D. The tested antibodies PR300526, PR300659, PR300670, PR201285, PR3001286, and PR3001289 all dose-dependently inhibited proliferation of myeloid cells and had comparable or even stronger inhibitory effect compared with the positive control. Besides, the engineered mutants of PR301286 and PR301289 also showed activity comparable to that of the parent antibodies.

Example 13. Myeloid Cell Function Assay of Recombinant Antibodies-Secretion of Cytokine TNF-α

Monocyte cells were isolated from PBMCs using a human monocyte cell kit (Miltenyi, Cat#: 130-050-201) and adjusted to a cell density of $1 \times 10^6$/mL with 1640 complete medium. The cell suspension was added to a 96-well plate at 100 μL/well, and the antibody diluted in a gradient (5-fold dilution; final concentration starting at 100 nM) was added to the 96-well plate at 50 μL/well. Human Siglec-15 ECD-hFc protein (SEQ ID NO: 154) was diluted to 16 μg/mL and added at 50 μL/well (final concentration: 4 μg/mL). After incubation for 24 h, the supernatant was taken and tested for TNF-α using the TNF alpha Human Uncoated ELISA Kit (Ebioscience, Cat#: 88-7346-88). PR300066 and hIgG1 were used as positive control and negative control, respectively.

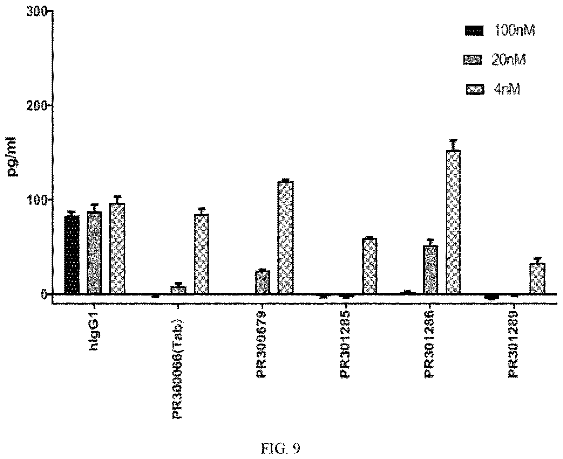

The results are shown in FIG. 9. The 4 antibodies tested were all able to dose-dependently inhibit the secretion of inflammatory factor TNF-α from myeloid cells; PR301285 and PR301289 had stronger inhibitory effect compared with the positive control, and PR300679 had comparable inhibitory effect compared with the positive control; however, PR301286 had slightly weaker inhibitory effect than the positive control.

Example 14. Inhibitory Effect of Anti-SigLec-15 Antibodies on hPBMC-A375 Graft Tumor

cDNAs encoding human Siglec-15 (SEQ ID NO: 1) were obtained by gene synthesis and cloned into the expression vector plenti6.3, and then the expression vector was transfected into A375 cells to construct A375-hSiglec-15 cells expressing human Siglec-15.

SPF-grade B-NDG mice were purchased from Biocytogen; each mouse was inoculated subcutaneously in the right armpit with $5 \times 10^6$ A375-hSiglec-15 cells. After tumor appeared, each mouse was injected with $1 \times 10^7$ hPBMC cells via the tail vein. When the mean tumor volume reached 100-300 mm³, the animals were randomly divided into 4 groups of 6. The day of grouping was d0 and the administration regimen is shown in Table 12. The tumor volume was measured 2-3 times a week, meanwhile the mouse was weighed, and the data were recorded; general performance of the mice was observed and recorded daily; after the experiment was completed, the tumors were taken out and weighed. The calculation formulas are as follows:

$$\text{Tumor volume TV (mm}^3) = 1/2 \times (a \times b^2),$$

wherein a is the long diameter and b is the short diameter.

Relative tumor volume RTV = TVt/TVo, wherein TVo is the tumor volume at d0, and TVt is the tumor volume at each measurement:

$$\text{Relative tumor proliferation rate T/C (\%)} = \frac{T_{RTV}}{C_{RTV}} \times 100\%,$$

wherein $T_{RTV}$ is the RTV of the treatment group, and $C_{RTV}$ is the RTV of the solvent group; the tumor volume inhibition rate = 1 - T/C.

Tumor weight inhibition rate TGI (%) = (1 - tumor weight of treatment group/tumor weight of solvent group) ×100%.

Weight change rate WCR (%) = $(Wt_t - Wt_0)/Wt_0 \times 100\%$, wherein Wto is the body weight of mice at d0, and Wtt is the body weight of mice at each measurement.

Table 12. Administration regimen

| Groups | Drug | Dose of administration | Administration volume | Time of administration | Route of administration |
|---|---|---|---|---|---|
| i | Solvent (PBS) | -- | 10 mL/kg | d0, d3, d7, d10, d14, d17, and d20 | i.p. |
| ii | PR300066 | 10 mg/kg | 10 mL/kg | d0, d3, d7, d10, d14, d17, and d20 | i.p. |
| iii | PR301286-5 | 10 mg/kg | 10 mL/kg | d0, d3, d7, d10, d14, d17, and d20 | i.p. |
| iv | PR301289-2 | 10 mg/kg | 10 mL/kg | d0, d3, d7, d10, d14, d17, and d20 | i.p. |
| Note: i.p. represents intraperitoneal injection | | | | | |

The results are shown in Table 13. PR300066, PR301286-5, and PR301289-2 had inhibitory effect on the growth of A375-hS15 human melanoma, the tumor volume inhibition rates at d21 were 16.8%, 35.3%, and 42.7%, respectively, the tumor weight inhibition rates at d21 were 27.7%, 36.1%, and 45.9%, respectively, and the inhibitory effect of PR301286-5 and PR301289-2 was significantly better than that of PR300066. In addition, the tumor-bearing mice were able to well tolerate the above drugs, and the weight change rates of mice in all the treatment groups were close to that of the solvent group, indicating that PR300066, PR301286-5, and PR301289-2 showed low probability of inducing a toxic response.

Table 13. Inhibitory effect of anti-SigLec15 antibodies on hPBMC-A375 graft tumor

| Groups | TV($mm^3$) (mean±SD) | | RTV (mean±SD) | T/C | 1-T/C | Tumor weight (mean±SD) | TGI (%) |
|---|---|---|---|---|---|---|---|
| | d0 | d21 | d21 | d21 | | d21 | d21 |
| i | 241±29 | 1831±475 | 7.64±2.05 | - | - | 1.157±0.221 | |
| ii | 241±29 | 1517±331 | 6.36±1.47 | 83.2% | 16.8% | 0.837±0.250* | 27.7% |
| iii | 241±26 | 1182±121 | 4.94±0.73 | 64.7% | 35.3% | 0.740±0.088** | 36.1% |
| iv | 241±26 | 1031±521 | 4.38±2.59 | 57.3% | 42.7% | 0.626±0.314** | 45.9% |
| Note: i.p. represents intraperitoneal injection; * p < 0.05 and ** p < 0.01 in comparison with the solvent group as determined by student's t test | | | | | | | |

All technical features disclosed in the specification may be combined in any way. Each feature disclosed in the specification may also be replaced by other features that have the same, equivalent, or similar effects. Thus, unless otherwise stated, each feature disclosed is only an example of a series of equivalent or similar features. Furthermore, those skilled in the art can readily understand the key features of the present disclosure according to the above description. Many modifications can be made to the present invention without departing from the spirit and scope of the present disclosure so the present invention is suitable for a variety of purposes and conditions of use, and therefore such modifications are also intended to fall within the scope of the appended claims.

**Claims**

1. An isolated anti-Siglec-15 antibody or an antigen-binding fragment thereof, wherein the antibody or the antigen-binding fragment thereof comprises any one of (i)-(iii):

   (i) a heavy chain CDR1, a heavy chain CDR2, and a heavy chain CDR3, wherein

(1) the heavy chain CDR1 comprises the amino acid sequence set forth in SEQ ID NO: 131 or an amino acid sequence having at least 80% identity to SEQ ID NO: 131, the heavy chain CDR2 comprises the amino acid sequence set forth in SEQ ID NO: 133 or an amino acid sequence having at least 80% identity to SEQ ID NO: 133, and the heavy chain CDR3 comprises the amino acid sequence set forth in SEQ ID NO: 135 or an amino acid sequence having at least 80% identity to SEQ ID NO: 135;

(2) the heavy chain CDR1 comprises the amino acid sequence set forth in SEQ ID NO: 131 or an amino acid sequence having at least 80% identity to SEQ ID NO: 131, the heavy chain CDR2 comprises the amino acid sequence set forth in SEQ ID NO: 133 or an amino acid sequence having at least 80% identity to SEQ ID NO: 133, and the heavy chain CDR3 comprises the amino acid sequence set forth in SEQ ID NO: 151 or an amino acid sequence having at least 80% identity to SEQ ID NO: 151;

(3) the heavy chain CDR1 comprises the amino acid sequence set forth in SEQ ID NO: 5 or an amino acid sequence having at least 80% identity to SEQ ID NO: 5, the heavy chain CDR2 comprises the amino acid sequence set forth in SEQ ID NO: 7 or an amino acid sequence having at least 80% identity to SEQ ID NO: 7, and the heavy chain CDR3 comprises the amino acid sequence set forth in SEQ ID NO: 9 or an amino acid sequence having at least 80% identity to SEQ ID NO: 9;

(4) the heavy chain CDR1 comprises the amino acid sequence set forth in SEQ ID NO: 23 or an amino acid sequence having at least 80% identity to SEQ ID NO: 23, the heavy chain CDR2 comprises the amino acid sequence set forth in SEQ ID NO: 25 or an amino acid sequence having at least 80% identity to SEQ ID NO: 25, and the heavy chain CDR3 comprises the amino acid sequence set forth in SEQ ID NO: 27 or an amino acid sequence having at least 80% identity to SEQ ID NO: 27;

(5) the heavy chain CDR1 comprises the amino acid sequence set forth in SEQ ID NO: 41 or an amino acid sequence having at least 80% identity to SEQ ID NO: 41, the heavy chain CDR2 comprises the amino acid sequence set forth in SEQ ID NO: 43 or an amino acid sequence having at least 80% identity to SEQ ID NO: 43, and the heavy chain CDR3 comprises the amino acid sequence set forth in SEQ ID NO: 45 or an amino acid sequence having at least 80% identity to SEQ ID NO: 45;

(6) the heavy chain CDR1 comprises the amino acid sequence set forth in SEQ ID NO: 59 or an amino acid sequence having at least 80% identity to SEQ ID NO: 59, the heavy chain CDR2 comprises the amino acid sequence set forth in SEQ ID NO: 61 or an amino acid sequence having at least 80% identity to SEQ ID NO: 61, and the heavy chain CDR3 comprises the amino acid sequence set forth in SEQ ID NO: 63 or an amino acid sequence having at least 80% identity to SEQ ID NO: 63;

(7) the heavy chain CDR1 comprises the amino acid sequence set forth in SEQ ID NO: 77 or an amino acid sequence having at least 80% identity to SEQ ID NO: 77, the heavy chain CDR2 comprises the amino acid sequence set forth in SEQ ID NO: 79 or an amino acid sequence having at least 80% identity to SEQ ID NO: 79, and the heavy chain CDR3 comprises the amino acid sequence set forth in SEQ ID NO: 81 or an amino acid sequence having at least 80% identity to SEQ ID NO: 81;

(8) the heavy chain CDR1 comprises the amino acid sequence set forth in SEQ ID NO: 95 or an amino acid sequence having at least 80% identity to SEQ ID NO: 95, the heavy chain CDR2 comprises the amino acid sequence set forth in SEQ ID NO: 97 or an amino acid sequence having at least 80% identity to SEQ ID NO: 97, and the heavy chain CDR3 comprises the amino acid sequence set forth in SEQ ID NO: 99 or an amino acid sequence having at least 80% identity to SEQ ID NO: 99;

(9) the heavy chain CDR1 comprises the amino acid sequence set forth in SEQ ID NO: 95 or an amino acid sequence having at least 80% identity to SEQ ID NO: 95, the heavy chain CDR2 comprises the amino acid sequence set forth in SEQ ID NO: 112 or an amino acid sequence having at least 80% identity to SEQ ID NO: 112, and the heavy chain CDR3 comprises the amino acid sequence set forth in SEQ ID NO: 99 or an amino acid sequence having at least 80% identity to SEQ ID NO: 99;

(10) the heavy chain CDR1 comprises the amino acid sequence set forth in SEQ ID NO: 95 or an amino acid sequence having at least 80% identity to SEQ ID NO: 95, the heavy chain CDR2 comprises the amino acid sequence set forth in SEQ ID NO: 115 or an amino acid sequence having at least 80% identity to SEQ ID NO: 115, and the heavy chain CDR3 comprises the amino acid sequence set forth in SEQ ID NO: 99 or an amino acid sequence having at least 80% identity to SEQ ID NO: 99; or

(11) the heavy chain CDR1 comprises the amino acid sequence set forth in SEQ ID NO: 95 or an amino acid sequence having at least 80% identity to SEQ ID NO: 95, the heavy chain CDR2 comprises the amino acid sequence set forth in SEQ ID NO: 118 or an amino acid sequence having at least 80% identity to SEQ ID NO: 118, and the heavy chain CDR3 comprises the amino acid sequence set forth in SEQ ID NO: 99 or an amino acid sequence having at least 80% identity to SEQ ID NO: 99;

(ii) a light chain CDR1, a light chain CDR2, and a light chain CDR3, wherein

(1) the light chain CDR1 comprises the amino acid sequence set forth in SEQ ID NO: 140 or an amino acid sequence having at least 80% identity to SEQ ID NO: 140, the light chain CDR2 comprises the amino acid sequence set forth in SEQ ID NO: 142 or an amino acid sequence having at least 80% identity to SEQ ID NO: 142, and the light chain CDR3 comprises the sequence set forth in SEQ ID NO: 144 or an amino acid sequence having at least 80% identity to SEQ ID NO: 144;

(2) the light chain CDR1 comprises the amino acid sequence set forth in SEQ ID NO: 14 or an amino acid sequence having at least 80% identity to SEQ ID NO: 14, the light chain CDR2 comprises the amino acid sequence set forth in SEQ ID NO: 16 or an amino acid sequence having at least 80% identity to SEQ ID NO: 16, and the light chain CDR3 comprises the amino acid sequence set forth in SEQ ID NO: 18 or an amino acid sequence having at least 80% identity to SEQ ID NO: 18;

(3) the light chain CDR1 comprises the amino acid sequence set forth in SEQ ID NO: 32 or an amino acid sequence having at least 80% identity to SEQ ID NO: 32, the light chain CDR2 comprises the amino acid sequence set forth in SEQ ID NO: 34 or an amino acid sequence having at least 80% identity to SEQ ID NO: 34, and the light chain CDR3 comprises the amino acid sequence set forth in SEQ ID NO: 36 or an amino acid sequence having at least 80% identity to SEQ ID NO: 36;

(4) the light chain CDR1 comprises the amino acid sequence set forth in SEQ ID NO: 50 or an amino acid sequence having at least 80% identity to SEQ ID NO: 50, the light chain CDR2 comprises the amino acid sequence set forth in SEQ ID NO: 52 or an amino acid sequence having at least 80% identity to SEQ ID NO: 52, and the light chain CDR3 comprises the amino acid sequence set forth in SEQ ID NO: 54 or an amino acid sequence having at least 80% identity to SEQ ID NO: 54;

(5) the light chain CDR1 comprises the amino acid sequence set forth in SEQ ID NO: 68 or an amino acid sequence having at least 80% identity to SEQ ID NO: 68, the light chain CDR2 comprises the amino acid sequence set forth in SEQ ID NO: 70 or an amino acid sequence having at least 80% identity to SEQ ID NO: 70, and the light chain CDR3 comprises the amino acid sequence set forth in SEQ ID NO: 72 or an amino acid sequence having at least 80% identity to SEQ ID NO: 72;

(6) the light chain CDR1 comprises the amino acid sequence set forth in SEQ ID NO: 86 or an amino acid sequence having at least 80% identity to SEQ ID NO: 86, the light chain CDR2 comprises the amino acid sequence set forth in SEQ ID NO: 88 or an amino acid sequence having at least 80% identity to SEQ ID NO: 88, and the light chain CDR3 comprises the amino acid sequence set forth in SEQ ID NO: 90 or an amino acid sequence having at least 80% identity to SEQ ID NO: 90;

(7) the light chain CDR1 comprises the amino acid sequence set forth in SEQ ID NO: 104 or an amino acid sequence having at least 80% identity to SEQ ID NO: 104, the light chain CDR2 comprises the amino acid sequence set forth in SEQ ID NO: 106 or an amino acid sequence having at least 80% identity to SEQ ID NO: 106, and the light chain CDR3 comprises the amino acid sequence set forth in SEQ ID NO: 108 or an amino acid sequence having at least 80% identity to SEQ ID NO: 108;

(8) the light chain CDR1 comprises the amino acid sequence set forth in SEQ ID NO: 121 or an amino acid sequence having at least 80% identity to SEQ ID NO: 121, the light chain CDR2 comprises the amino acid sequence set forth in SEQ ID NO: 106 or an amino acid sequence having at least 80% identity to SEQ ID NO: 106, and the light chain CDR3 comprises the amino acid sequence set forth in SEQ ID NO: 108 or an amino acid sequence having at least 80% identity to SEQ ID NO: 108;

(9) the light chain CDR1 comprises the amino acid sequence set forth in SEQ ID NO: 124 or an amino acid sequence having at least 80% identity to SEQ ID NO: 124, the light chain CDR2 comprises the amino acid sequence set forth in SEQ ID NO: 106 or an amino acid sequence having at least 80% identity to SEQ ID NO: 106, and the light chain CDR3 comprises the amino acid sequence set forth in SEQ ID NO: 108 or an amino acid sequence having at least 80% identity to SEQ ID NO: 108; or

(10) the light chain CDR1 comprises the amino acid sequence set forth in SEQ ID NO: 127 or an amino acid sequence having at least 80% identity to SEQ ID NO: 127, the light chain CDR2 comprises the amino acid sequence set forth in SEQ ID NO: 106 or an amino acid sequence having at least 80% identity to SEQ ID NO: 106, and the light chain CDR3 comprises the amino acid sequence set forth in SEQ ID NO: 108 or an amino acid sequence having at least 80% identity to SEQ ID NO: 108;

(iii) a heavy chain CDR1, a heavy chain CDR2, and a heavy chain CDR3 set forth in (i), and a light chain CDR1, a light chain CDR2, and a light chain CDR3 set forth in (ii).

2. The antibody or the antigen-binding fragment thereof according to claim 1, wherein the antibody or the antigen-binding fragment thereof comprises a heavy chain CDR1, a heavy chain CDR2, a heavy chain CDR3, a light chain CDR1, a light chain CDR2, and a light chain CDR3, wherein

(1) the heavy chain CDR1 comprises the amino acid sequence set forth in SEQ ID NO: 131 or an amino acid sequence having at least 80% identity to SEQ ID NO: 131, the heavy chain CDR2 comprises the amino acid sequence set forth in SEQ ID NO: 133 or an amino acid sequence having at least 80% identity to SEQ ID NO: 133, the heavy chain CDR3 comprises the amino acid sequence set forth in SEQ ID NO: 135 or an amino acid sequence having at least 80% identity to SEQ ID NO: 135, the light chain CDR1 comprises the amino acid sequence set forth in SEQ ID NO: 140 or an amino acid sequence having at least 80% identity to SEQ ID NO: 140, the light chain CDR2 comprises the amino acid sequence set forth in SEQ ID NO: 142 or an amino acid sequence having at least 80% identity to SEQ ID NO: 142, and the light chain CDR3 comprises the amino acid sequence set forth in SEQ ID NO: 144 or an amino acid sequence having at least 80% identity to SEQ ID NO: 144;

(2) the heavy chain CDR1 comprises the amino acid sequence set forth in SEQ ID NO: 131 or an amino acid sequence having at least 80% identity to SEQ ID NO: 131, the heavy chain CDR2 comprises the amino acid sequence set forth in SEQ ID NO: 133 or an amino acid sequence having at least 80% identity to SEQ ID NO: 133, the heavy chain CDR3 comprises the amino acid sequence set forth in SEQ ID NO: 151 or an amino acid sequence having at least 80% identity to SEQ ID NO: 151, the light chain CDR1 comprises the amino acid sequence set forth in SEQ ID NO: 140 or an amino acid sequence having at least 80% identity to SEQ ID NO: 140, the light chain CDR2 comprises the amino acid sequence set forth in SEQ ID NO: 142 or an amino acid sequence having at least 80% identity to SEQ ID NO: 142, and the light chain CDR3 comprises the amino acid sequence set forth in SEQ ID NO: 144 or an amino acid sequence having at least 80% identity to SEQ ID NO: 144;

(3) the heavy chain CDR1 comprises the amino acid sequence set forth in SEQ ID NO: 5 or an amino acid sequence having at least 80% identity to SEQ ID NO: 5, the heavy chain CDR2 comprises the amino acid sequence set forth in SEQ ID NO: 7 or an amino acid sequence having at least 80% identity to SEQ ID NO: 7, the heavy chain CDR3 comprises the amino acid sequence set forth in SEQ ID NO: 9 or an amino acid sequence having at least 80% identity to SEQ ID NO: 9, the light chain CDR1 comprises the amino acid sequence set forth in SEQ ID NO: 14 or an amino acid sequence having at least 80% identity to SEQ ID NO: 14, the light chain CDR2 comprises the amino acid sequence set forth in SEQ ID NO: 16 or an amino acid sequence having at least 80% identity to SEQ ID NO: 16, and the light chain CDR3 comprises the amino acid sequence set forth in SEQ ID NO: 18 or an amino acid sequence having at least 80% identity to SEQ ID NO: 18;

(4) the heavy chain CDR1 comprises the amino acid sequence set forth in SEQ ID NO: 23 or an amino acid sequence having at least 80% identity to SEQ ID NO: 23, the heavy chain CDR2 comprises the amino acid sequence set forth in SEQ ID NO: 25 or an amino acid sequence having at least 80% identity to SEQ ID NO: 25, the heavy chain CDR3 comprises the amino acid sequence set forth in SEQ ID NO: 27 or an amino acid sequence having at least 80% identity to SEQ ID NO: 27, the light chain CDR1 comprises the amino acid sequence set forth in SEQ ID NO: 32 or an amino acid sequence having at least 80% identity to SEQ ID NO: 32, the light chain CDR2 comprises the amino acid sequence set forth in SEQ ID NO: 34 or an amino acid sequence having at least 80% identity to SEQ ID NO: 34, and the light chain CDR3 comprises the amino acid sequence set forth in SEQ ID NO: 36 or an amino acid sequence having at least 80% identity to SEQ ID NO: 36;

(5) the heavy chain CDR1 comprises the amino acid sequence set forth in SEQ ID NO: 41 or an amino acid sequence having at least 80% identity to SEQ ID NO: 41, the heavy chain CDR2 comprises the amino acid sequence set forth in SEQ ID NO: 43 or an amino acid sequence having at least 80% identity to SEQ ID NO: 43, the heavy chain CDR3 comprises the amino acid sequence set forth in SEQ ID NO: 45 or an amino acid sequence having at least 80% identity to SEQ ID NO: 45, the light chain CDR1 comprises the amino acid sequence set forth in SEQ ID NO: 50 or an amino acid sequence having at least 80% identity to SEQ ID NO: 50, the light chain CDR2 comprises the amino acid sequence set forth in SEQ ID NO: 52 or an amino acid sequence having at least 80% identity to SEQ ID NO: 52, and the light chain CDR3 comprises the amino acid sequence set forth in SEQ ID NO: 54 or an amino acid sequence having at least 80% identity to SEQ ID NO: 54;

(6) the heavy chain CDR1 comprises the amino acid sequence set forth in SEQ ID NO: 59 or an amino acid sequence having at least 80% identity to SEQ ID NO: 59, the heavy chain CDR2 comprises the amino acid sequence set forth in SEQ ID NO: 61 or an amino acid sequence having at least 80% identity to SEQ ID NO: 61, the heavy chain CDR3 comprises the amino acid sequence set forth in SEQ ID NO: 63 or an amino acid sequence having at least 80% identity to SEQ ID NO: 63, the light chain CDR1 comprises the amino acid sequence set forth in SEQ ID NO: 68 or an amino acid sequence having at least 80% identity to SEQ ID NO: 68, the light chain CDR2 comprises the amino acid sequence set forth in SEQ ID NO: 70 or an amino acid sequence having at least 80% identity to SEQ ID NO: 70, and the light chain CDR3 comprises the amino acid sequence set forth in SEQ ID NO: 72 or an amino acid sequence having at least 80% identity to SEQ ID NO: 72;

(7) the heavy chain CDR1 comprises the amino acid sequence set forth in SEQ ID NO: 77 or an amino acid sequence having at least 80% identity to SEQ ID NO: 77, the heavy chain CDR2 comprises the amino acid sequence set forth in SEQ ID NO: 79 or an amino acid sequence having at least 80% identity to SEQ ID NO: 79, the heavy chain CDR3 comprises the amino acid sequence set forth in SEQ ID NO: 81 or an amino acid sequence having at least 80% identity to SEQ ID NO: 81, the light chain CDR1 comprises the amino acid sequence set forth in SEQ ID NO: 86 or an amino acid sequence having at least 80% identity to SEQ ID NO: 86, the light chain CDR2 comprises the amino acid sequence set forth in SEQ ID NO: 88 or an amino acid sequence having at least 80% identity to SEQ ID NO: 88, and the light chain CDR3 comprises the amino acid sequence set forth in SEQ ID NO: 90 or an amino acid sequence having at least 80% identity to SEQ ID NO: 90;

(8) the heavy chain CDR1 comprises the amino acid sequence set forth in SEQ ID NO: 95 or an amino acid sequence having at least 80% identity to SEQ ID NO: 95, the heavy chain CDR2 comprises the amino acid sequence set forth in SEQ ID NO: 97 or an amino acid sequence having at least 80% identity to SEQ ID NO: 97, the heavy chain CDR3 comprises the amino acid sequence set forth in SEQ ID NO: 99 or an amino acid sequence having at least 80% identity to SEQ ID NO: 99, the light chain CDR1 comprises the amino acid sequence set forth in SEQ ID NO: 104 or an amino acid sequence having at least 80% identity to SEQ ID NO: 104, the light chain CDR2 comprises the amino acid sequence set forth in SEQ ID NO: 106 or an amino acid sequence having at least 80% identity to SEQ ID NO: 106, and the light chain CDR3 comprises the amino acid sequence set forth in SEQ ID NO: 108 or an amino acid sequence having at least 80% identity to SEQ ID NO: 108;

(9) the heavy chain CDR1 comprises the amino acid sequence set forth in SEQ ID NO: 95 or an amino acid sequence having at least 80% identity to SEQ ID NO: 95, the heavy chain CDR2 comprises the amino acid sequence set forth in SEQ ID NO: 112 or an amino acid sequence having at least 80% identity to SEQ ID NO: 112, the heavy chain CDR3 comprises the amino acid sequence set forth in SEQ ID NO: 99 or an amino acid sequence having at least 80% identity to SEQ ID NO: 99, the light chain CDR1 comprises the amino acid sequence set forth in SEQ ID NO: 104 or an amino acid sequence having at least 80% identity to SEQ ID NO: 104, the light chain CDR2 comprises the amino acid sequence set forth in SEQ ID NO: 106 or an amino acid sequence having at least 80% identity to SEQ ID NO: 106, and the light chain CDR3 comprises the amino acid sequence set forth in SEQ ID NO: 108 or an amino acid sequence having at least 80% identity to SEQ ID NO: 108;

(10) the heavy chain CDR1 comprises the amino acid sequence set forth in SEQ ID NO: 95 or an amino acid sequence having at least 80% identity to SEQ ID NO: 95, the heavy chain CDR2 comprises the amino acid sequence set forth in SEQ ID NO: 115 or an amino acid sequence having at least 80% identity to SEQ ID NO: 115, the heavy chain CDR3 comprises the amino acid sequence set forth in SEQ ID NO: 99 or an amino acid sequence having at least 80% identity to SEQ ID NO: 99, the light chain CDR1 comprises the amino acid sequence set forth in SEQ ID NO: 104 or an amino acid sequence having at least 80% identity to SEQ ID NO: 104, the light chain CDR2 comprises the amino acid sequence set forth in SEQ ID NO: 106 or an amino acid sequence having at least 80% identity to SEQ ID NO: 106, and the light chain CDR3 comprises the amino acid sequence set forth in SEQ ID NO: 108 or an amino acid sequence having at least 80% identity to SEQ ID NO: 108;

(11) the heavy chain CDR1 comprises the amino acid sequence set forth in SEQ ID NO: 95 or an amino acid sequence having at least 80% identity to SEQ ID NO: 95, the heavy chain CDR2 comprises the amino acid sequence set forth in SEQ ID NO: 118 or an amino acid sequence having at least 80% identity to SEQ ID NO: 118, the heavy chain CDR3 comprises the amino acid sequence set forth in SEQ ID NO: 99 or an amino acid sequence having at least 80% identity to SEQ ID NO: 99, the light chain CDR1 comprises the amino acid sequence set forth in SEQ ID NO: 121 or an amino acid sequence having at least 80% identity to SEQ ID NO: 121, the light chain CDR2 comprises the amino acid sequence set forth in SEQ ID NO: 106 or an amino acid sequence having at least 80% identity to SEQ ID NO: 106, and the light chain CDR3 comprises the amino acid sequence set forth in SEQ ID NO: 108 or an amino acid sequence having at least 80% identity to SEQ ID NO: 108;

(12) the heavy chain CDR1 comprises the amino acid sequence set forth in SEQ ID NO: 95 or an amino acid sequence having at least 80% identity to SEQ ID NO: 95, the heavy chain CDR2 comprises the amino acid sequence set forth in SEQ ID NO: 118 or an amino acid sequence having at least 80% identity to SEQ ID NO: 118, the heavy chain CDR3 comprises the amino acid sequence set forth in SEQ ID NO: 99 or an amino acid sequence having at least 80% identity to SEQ ID NO: 99, the light chain CDR1 comprises the amino acid sequence set forth in SEQ ID NO: 124 or an amino acid sequence having at least 80% identity to SEQ ID NO: 124, the light chain CDR2 comprises the amino acid sequence set forth in SEQ ID NO: 106 or an amino acid sequence having at least 80% identity to SEQ ID NO: 106, and the light chain CDR3 comprises the amino acid sequence set forth in SEQ ID NO: 108 or an amino acid sequence having at least 80% identity to SEQ ID NO: 108; or

(13) the heavy chain CDR1 comprises the amino acid sequence set forth in SEQ ID NO: 95 or an amino acid sequence having at least 80% identity to SEQ ID NO: 95, the heavy chain CDR2 comprises the amino acid sequence set forth in SEQ ID NO: 118 or an amino acid sequence having at least 80% identity to SEQ ID NO: 118, the heavy chain CDR3 comprises the amino acid sequence set forth in SEQ ID NO: 99 or an amino acid sequence having at least 80% identity to SEQ ID NO: 99, the light chain CDR1 comprises the amino acid sequence set forth in SEQ ID NO: 127 or an amino acid sequence having at least 80% identity to SEQ ID NO: 127, the light chain CDR2 comprises the amino acid sequence set forth in SEQ ID NO: 106 or an amino acid sequence having at least 80% identity to SEQ ID NO: 106, and the light chain CDR3 comprises the amino acid sequence set forth in SEQ ID NO: 108 or an amino acid sequence having at least 80% identity to SEQ ID NO: 108.

3. The antibody or the antigen-binding fragment thereof according to claim 1 or 2, wherein the antibody or the antigen-binding fragment thereof is chimeric, humanized, or fully human.

4. The antibody or the antigen-binding fragment thereof according to any one of claims 1-3, wherein the antibody or the antigen-binding fragment thereof comprises any one of (i)-(iii):

(i) a heavy chain variable region comprising the amino acid sequence set forth in SEQ ID NO: 129, 147, 149, 3, 21, 39, 57, 75, 93, 111, 114 or 117 or an amino acid sequence having at least 80% identity to SEQ ID NO: 129, 147, 149, 3, 21, 39, 57, 75, 93, 111, 114 or 117;
(ii) a light chain variable region comprising the amino acid sequence set forth in SEQ ID NO: 138, 12, 30, 48, 66, 84, 102, 120, 123 or 126 or an amino acid sequence having at least 80% identity to SEQ ID NO: 138, 12, 30, 48, 66, 84, 102, 120, 123 or 126; or
(iii) a heavy chain variable region set forth in (i) and a light chain variable region set forth in (ii).

5. The antibody or the antigen-binding fragment thereof according to any one of claims 1-4, wherein the antibody or the antigen-binding fragment thereof comprises a heavy chain variable region and a light chain variable region, wherein

(1) the heavy chain variable region comprises the amino acid sequence set forth in SEQ ID NO: 129 or an amino acid sequence having at least 80% identity to SEQ ID NO: 129, and the light chain variable region comprises the amino acid sequence set forth in SEQ ID NO: 138 or an amino acid sequence having at least 80% identity to SEQ ID NO: 138;
(2) the heavy chain variable region comprises the amino acid sequence set forth in SEQ ID NO: 147 or an amino acid sequence having at least 80% identity to SEQ ID NO: 147, and the light chain variable region comprises the amino acid sequence set forth in SEQ ID NO: 138 or an amino acid sequence having at least 80% identity to SEQ ID NO: 138;
(3) the heavy chain variable region comprises the amino acid sequence set forth in SEQ ID NO: 149 or an amino acid sequence having at least 80% identity to SEQ ID NO: 149, and the light chain variable region comprises the amino acid sequence set forth in SEQ ID NO: 138 or an amino acid sequence having at least 80% identity to SEQ ID NO: 138;
(4) the heavy chain variable region comprises the amino acid sequence set forth in SEQ ID NO: 3 or an amino acid sequence having at least 80% identity to SEQ ID NO: 3, and the light chain variable region comprises the amino acid sequence set forth in SEQ ID NO: 12 or an amino acid sequence having at least 80% identity to SEQ ID NO: 12;
(5) the heavy chain variable region comprises the amino acid sequence set forth in SEQ ID NO: 21 or an amino acid sequence having at least 80% identity to SEQ ID NO: 21, and the light chain variable region comprises the amino acid sequence set forth in SEQ ID NO: 30 or an amino acid sequence having at least 80% identity to SEQ ID NO: 30;
(6) the heavy chain variable region comprises the amino acid sequence set forth in SEQ ID NO: 39 or an amino acid sequence having at least 80% identity to SEQ ID NO: 39, and the light chain variable region comprises the amino acid sequence set forth in SEQ ID NO: 48 or an amino acid sequence having at least 80% identity to SEQ ID NO: 48;
(7) the heavy chain variable region comprises the amino acid sequence set forth in SEQ ID NO: 57 or an amino acid sequence having at least 80% identity to SEQ ID NO: 57, and the light chain variable region comprises the amino acid sequence set forth in SEQ ID NO: 66 or an amino acid sequence having at least 80% identity to SEQ ID NO: 66;
(8) the heavy chain variable region comprises the amino acid sequence set forth in SEQ ID NO: 75 or an amino acid sequence having at least 80% identity to SEQ ID NO: 75, and the light chain variable region comprises the amino acid sequence set forth in SEQ ID NO: 84 or an amino acid sequence having at least 80% identity to SEQ ID NO: 84;
(9) the heavy chain variable region comprises the amino acid sequence set forth in SEQ ID NO: 93 or an amino acid sequence having at least 80% identity to SEQ ID NO: 93, and the light chain variable region comprises the amino acid sequence set forth in SEQ ID NO: 102 or an amino acid sequence having at least 80% identity to SEQ ID NO: 102;
(10) the heavy chain variable region comprises the amino acid sequence set forth in SEQ ID NO: 111 or an amino acid sequence having at least 80% identity to SEQ ID NO: 111, and the light chain variable region comprises the amino acid sequence set forth in SEQ ID NO: 102 or an amino acid sequence having at least 80% identity to SEQ ID NO: 102;

(11) the heavy chain variable region comprises the amino acid sequence set forth in SEQ ID NO: 114 or an amino acid sequence having at least 80% identity to SEQ ID NO: 114, and the light chain variable region comprises the amino acid sequence set forth in SEQ ID NO: 102 or an amino acid sequence having at least 80% identity to SEQ ID NO: 102;

(12) the heavy chain variable region comprises the amino acid sequence set forth in SEQ ID NO: 117 or an amino acid sequence having at least 80% identity to SEQ ID NO: 117, and the light chain variable region comprises the amino acid sequence set forth in SEQ ID NO: 120 or an amino acid sequence having at least 80% identity to SEQ ID NO: 120;

(13) the heavy chain variable region comprises the amino acid sequence set forth in SEQ ID NO: 117 or an amino acid sequence having at least 80% identity to SEQ ID NO: 117, and the light chain variable region comprises the amino acid sequence set forth in SEQ ID NO: 123 or an amino acid sequence having at least 80% identity to SEQ ID NO: 123; or

(14) the heavy chain variable region comprises the amino acid sequence set forth in SEQ ID NO: 117 or an amino acid sequence having at least 80% identity to SEQ ID NO: 117, and the light chain variable region comprises the amino acid sequence set forth in SEQ ID NO: 126 or an amino acid sequence having at least 80% identity to SEQ ID NO: 126.

6. The antibody or the antigen-binding fragment thereof according to any one of claims 1-5, wherein the antibody or the antigen-binding fragment thereof comprises any one of (i)-(iii):

(i) a heavy chain comprising the amino acid sequence set forth in SEQ ID NO: 128, 146, 148, 2, 20, 38, 56, 74, 92, 110, 113 or 116 or an amino acid sequence having at least 80% identity to SEQ ID NO: 128, 146, 148, 2, 20, 38, 56, 74, 92, 110, 113 or 116;

(ii) a light chain comprising the amino acid sequence set forth in SEQ ID NO: 137, 11, 29, 47, 65, 83, 101, 119, 122 or 125 or an amino acid sequence having at least 80% identity to SEQ ID NO: 137, 11, 29, 47, 65, 83, 101, 119, 122 or 125; or

(iii) a heavy chain set forth in (i) and a light chain set forth in (ii).

7. The antibody or the antigen-binding fragment thereof according to any one of claims 1-6, wherein the antibody or the antigen-binding fragment thereof comprises a heavy chain and a light chain, wherein

(1) the heavy chain comprises the amino acid sequence set forth in SEQ ID NO: 128 or an amino acid sequence having at least 80% identity to SEQ ID NO: 128, and the light chain comprises the amino acid sequence set forth in SEQ ID NO: 137 or an amino acid sequence having at least 80% identity to SEQ ID NO: 137;

(2) the heavy chain comprises the amino acid sequence set forth in SEQ ID NO: 146 or an amino acid sequence having at least 80% identity to SEQ ID NO: 146, and the light chain comprises the amino acid sequence set forth in SEQ ID NO: 137 or an amino acid sequence having at least 80% identity to SEQ ID NO: 137;

(3) the heavy chain comprises the amino acid sequence set forth in SEQ ID NO: 148 or an amino acid sequence having at least 80% identity to SEQ ID NO: 148, and the light chain comprises the amino acid sequence set forth in SEQ ID NO: 137 or an amino acid sequence having at least 80% identity to SEQ ID NO: 137;

(4) the heavy chain comprises the amino acid sequence set forth in SEQ ID NO: 2 or an amino acid sequence having at least 80% identity to SEQ ID NO: 2, and the light chain comprises the amino acid sequence set forth in SEQ ID NO: 11 or an amino acid sequence having at least 80% identity to SEQ ID NO: 11;

(5) the heavy chain comprises the amino acid sequence set forth in SEQ ID NO: 20 or an amino acid sequence having at least 80% identity to SEQ ID NO: 20, and the light chain comprises the amino acid sequence set forth in SEQ ID NO: 29 or an amino acid sequence having at least 80% identity to SEQ ID NO: 29;

(6) the heavy chain comprises the amino acid sequence set forth in SEQ ID NO: 38 or an amino acid sequence having at least 80% identity to SEQ ID NO: 38, and the light chain comprises the amino acid sequence set forth in SEQ ID NO: 47 or an amino acid sequence having at least 80% identity to SEQ ID NO: 47;

(7) the heavy chain comprises the amino acid sequence set forth in SEQ ID NO: 56 or an amino acid sequence having at least 80% identity to SEQ ID NO: 56, and the light chain comprises the amino acid sequence set forth in SEQ ID NO: 65 or an amino acid sequence having at least 80% identity to SEQ ID NO: 65;

(8) the heavy chain comprises the amino acid sequence set forth in SEQ ID NO: 74 or an amino acid sequence having at least 80% identity to SEQ ID NO: 74, and the light chain comprises the amino acid sequence set forth in SEQ ID NO: 83 or an amino acid sequence having at least 80% identity to SEQ ID NO: 83;

(9) the heavy chain comprises the amino acid sequence set forth in SEQ ID NO: 92 or an amino acid sequence having at least 80%, 81%, or 82% identity to SEQ ID NO: 92, and the light chain comprises the amino acid sequence set forth in SEQ ID NO: 101 or an amino acid sequence having at least 80% identity to SEQ ID NO: 101;

(10) the heavy chain comprises the amino acid sequence set forth in SEQ ID NO: 110 or an amino acid sequence having at least 80% identity to SEQ ID NO: 110, and the light chain comprises the amino acid sequence set forth in SEQ ID NO: 101 or an amino acid sequence having at least 80% identity to SEQ ID NO: 101;

(11) the heavy chain comprises the amino acid sequence set forth in SEQ ID NO: 113 or an amino acid sequence having at least 80% identity to SEQ ID NO: 113, and the light chain comprises the amino acid sequence set forth in SEQ ID NO: 101 or an amino acid sequence having at least 80% identity to SEQ ID NO: 101;

(12) the heavy chain comprises the amino acid sequence set forth in SEQ ID NO: 116 or an amino acid sequence having at least 80% identity to SEQ ID NO: 116, and the light chain comprises the amino acid sequence set forth in SEQ ID NO: 119 or an amino acid sequence having at least 80% identity to SEQ ID NO: 119;

(13) the heavy chain comprises the amino acid sequence set forth in SEQ ID NO: 116 or an amino acid sequence having at least 80% identity to SEQ ID NO: 116, and the light chain comprises the amino acid sequence set forth in SEQ ID NO: 122 or an amino acid sequence having at least 80% identity to SEQ ID NO: 122; or

(14) the heavy chain comprises the amino acid sequence set forth in SEQ ID NO: 116 or an amino acid sequence having at least 80% identity to SEQ ID NO: 116, and the light chain comprises the amino acid sequence set forth in SEQ ID NO: 125 or an amino acid sequence having at least 80% identity to SEQ ID NO: 125.

8. The antibody or the antigen-binding fragment thereof according to any one of claims 1-7, wherein the antibody or the antigen-binding fragment thereof substantially does not bind to Siglec-2, Siglec-3, Siglec-4a, Siglec-10, and PD-L1.

9. The antibody or the antigen-binding fragment thereof according to any one of claims 1-8, wherein the antibody or the antigen-binding fragment thereof comprises a monoclonal antibody, a nanobody, a Fab fragment, a F(ab')$_2$ fragment, an Fd fragment, an Fv fragment, a dAb fragment, an isolated CDR region, a scFv, or a combination thereof.

10. An isolated nucleic acid molecule encoding the antibody or the antigen-binding fragment thereof according to any one of claims 1-9.

11. An expression vector, comprising the nucleic acid molecule according to claim 10.

12. A host cell, comprising the nucleic acid molecule according to claim 10 or the expression vector according to claim 11.

13. A polypeptide fusion or a multispecific antibody, comprising the antibody or the antigen-binding fragment thereof according to any one of claims 1-9.

14. A viral vector, comprising a nucleic acid molecule encoding the antibody or the antigen-binding fragment thereof according to any one of claims 1-9 or the nucleic acid molecule according to claim 10.

15. A pharmaceutical composition, comprising the antibody or the antigen-binding fragment thereof according to any one of claims 1-9 and one or more pharmaceutically acceptable excipients, diluents, or carriers.

16. A method for treating or ameliorating Siglec-15-associated diseases or disorders in a subject in need thereof, comprising administering to the subject a therapeutically effective amount of the antibody or the antigen-binding fragment thereof according to any one of claims 1-9, the nucleic acid molecule according to claim 10, or the pharmaceutical composition according to claim 15.

17. The method according to claim 16, wherein the Siglec-15-associated diseases or disorders comprise cancers, infections, autoimmune diseases, abnormal bone metabolism, or a combination thereof.

18. A method for regulating an immune response or reducing T cell suppression in a subject in need thereof, comprising administering to the subject a therapeutically effective amount of the antibody or the antigen-binding fragment thereof according to any one of claims 1-9, the nucleic acid molecule according to claim 10, or the pharmaceutical composition according to claim 15.

19. A method for detecting or diagnosing a disease or disorder, comprising:

(a) determining Siglec-15 expression in a cell or a tissue sample of a subject using the antibody or the antigen-binding fragment thereof according to any one of claims 1-9, and
(b) comparing the level of Siglec-15 with a control level, wherein an increase in the determined level of Siglec-15 as compared with the control level indicates the presence of the disease or disorder.

20. A method for monitoring response to treatment, comprising:

(a) determining Siglec-15 expression in a cell or a tissue sample of a subject before and after the treatment using the antibody or the antigen-binding fragment thereof according to any one of claims 1-9, and
(b) comparing the levels of Siglec-15 over time, wherein a decrease in the level of Siglec-15 determined after the treatment as compared with the level of Siglec-15 before the treatment indicates good response to the treatment.

FIG. 1A

FIG. 1B

FIG. 1C

FIG. 1D

FIG. 2A

FIG. 2B

FIG. 3A

FIG. 3B

FIG. 4A

FIG. 4B

FIG. 4C

FIG. 5A

FIG. 5B

FIG. 6

FIG. 7A

FIG. 7B

FIG. 7C

FIG. 7D

FIG. 8A

FIG. 8B

FIG. 8C

FIG. 8D

FIG. 9

## INTERNATIONAL SEARCH REPORT

| International application No. |
| --- |
| **PCT/CN2022/134161** |

| A. | CLASSIFICATION OF SUBJECT MATTER |
| --- | --- |

A61K39/00(2006.01)i;C07K16/28(2006.01)i

According to International Patent Classification (IPC) or to both national classification and IPC

| B. | FIELDS SEARCHED |
| --- | --- |

Minimum documentation searched (classification system followed by classification symbols)

IPC: A61K, C07K

Documentation searched other than minimum documentation to the extent that such documents are included in the fields searched

Electronic data base consulted during the international search (name of data base and, where practicable, search terms used)

CNTXT, VEN, ENTXT, CNKI, ISI, STN, NCBI, 万方数据库, WANFANG DATABASE: T细胞, T cell? , 单抗, antibody, 免疫应答, SIGLEC15, SIGLEC-15, 发明人, 序列检索

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 110035769 A (NEXTCURE, INC.) 19 July 2019 (2019-07-19)<br>entire document | 1-20 |
| A | CN 112694532 A (BETA PHARMA (SUZHOU) CO., LTD.) 23 April 2021 (2021-04-23)<br>entire document | 1-20 |
| A | US 2020339683 A1 (INNOVATIVE CELLULAR THERAPEUTICS HOLDINGS, LTD.) 29 October 2020 (2020-10-29)<br>entire document | 1-20 |
| A | WO 2021190622 A1 (BIOSION, INC.) 30 September 2021 (2021-09-30)<br>entire document | 1-20 |
| A | 苏敏存 等 (SU, Mincun et al.). "Siglec-15在多种疾病中的研究进展 (Research Progress of Siglec-15 in Various Diseases)"<br>生物化工 (Biological Chemical Engineering), Vol. 7, No. 5, 31 October 2021 (2021-10-31), pages 158-159 and 174 | 1-20 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **07 February 2023** | **14 February 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

## INTERNATIONAL SEARCH REPORT

| International application No. |
| --- |
| **PCT/CN2022/134161** |

**C.** **DOCUMENTS CONSIDERED TO BE RELEVANT**

| Category* | Citation of document, with indication, where appropriate, of the relevant passages | Relevant to claim No. |
| --- | --- | --- |
| A | WANG, J. et al. "Siglec-15 as an immune suppressor and potential target for normalization cancer immunotherapy" *NAT MED.*, Vol. 25, No. 4, 30 April 2019 (2019-04-30), pages 656-666 | 1-20 |
| A | HE, F. et al. "High affinity monoclonal antibody targeting Siglec-15 for cancer immunotherapy" *JOURNAL OF CLINICAL AND TRANSLATIONAL RESEARCH,* Vol. 7, No. 6, 16 November 2021 (2021-11-16), pages 739-749 | 1-20 |

Form PCT/ISA/210 (second sheet) (July 2022)

**INTERNATIONAL SEARCH REPORT**

International application No.

**PCT/CN2022/134161**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

| International application No. |
| --- |
| **PCT/CN2022/134161** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **16-18**
because they relate to subject matter not required to be searched by this Authority, namely:

Claims 16-17 relate to a method for treating or alleviating a disease or condition associated with Siglec-15 in a subject in need. Claim 18 relates to a method for regulating an immune response in a subject in need or reducing T cell inhibition in a subject in need. That is, claims 16-18 relate to the subject matter as defined in PCT Rule 39.1 that does not warrant a search conducted by the international searching authority: (4) methods for the treatment of a human or animal body by surgery or therapy, as well as diagnostic methods implemented on the human or animal body. An international search was made on the basis of a use of a therapeutically effective amount of the antibody or the antigen-binding fragment thereof according to any one of claims 1-9, the nucleic acid molecule according to claim 10 or the pharmaceutical composition according to claim 15 in the preparation of a drug for treating or alleviating Siglec-15-related diseases or disease cases or a drug for regulating the immune response or reducing T cell inhibition in the subject in need.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2022/134161**

| Patent document cited in search report | | | Publication date (day/month/year) | Patent family member(s) | | | Publication date (day/month/year) |
|---|---|---|---|---|---|---|---|
| CN | 110035769 | A | 19 July 2019 | CA | 3033571 | A1 | 29 March 2018 |
| | | | | WO | 2018057735 | A1 | 29 March 2018 |
| | | | | WO | 2018057735 | A8 | 07 June 2018 |
| | | | | RU | 2019111722 | A | 22 October 2020 |
| | | | | RU | 2019111722 | A3 | 21 December 2020 |
| | | | | RU | 2759334 | C2 | 12 November 2021 |
| | | | | JP | 2022126630 | A | 30 August 2022 |
| | | | | JP | 2019536470 | A | 19 December 2019 |
| | | | | JP | 7069177 | B2 | 17 May 2022 |
| | | | | AU | 2017330346 | A1 | 21 February 2019 |
| | | | | KR | 20190050816 | A | 13 May 2019 |
| | | | | EP | 3515478 | A1 | 31 July 2019 |
| | | | | EP | 3515478 | A4 | 15 July 2020 |
| | | | | BR | 112019005292 | A2 | 03 September 2019 |
| | | | | US | 2019202912 | A1 | 04 July 2019 |
| | | | | US | 11390675 | B2 | 19 July 2022 |
| CN | 112694532 | A | 23 April 2021 | None | | | |
| US | 2020339683 | A1 | 29 October 2020 | US | 11104732 | B2 | 31 August 2021 |
| | | | | US | 2021347893 | A1 | 11 November 2021 |
| WO | 2021190622 | A1 | 30 September 2021 | KR | 20220158696 | A | 01 December 2022 |
| | | | | CA | 3173201 | A1 | 30 September 2021 |
| | | | | IL | 296736 | A | 01 November 2022 |
| | | | | AU | 2021240769 | A1 | 20 October 2022 |
| | | | | BR | 112022019129 | A2 | 06 December 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018057735 A1
- US 6951646 B
- US 6914128 B
- US 6090382 A
- US 6818216 B
- US 6156313 A
- US 6827925 B
- US 5833943 A
- US 5762905 A
- US 5760185 A
- US 5225539 A
- US 5530101 A
- US 5585089 A
- US 5693762 A
- US 6180370 B
- US 20030153043 A
- US 5677425 A
- US 6165745 A
- US 5714350 A
- US 6350861 B
- US 20040110704 A
- EP 1176195 A
- WO 03035835 A
- WO 06089231 A
- WO 9954342 A
- EP 0154316 A
- EP 0401384 A
- US 4816567 A
- US 6180370 A
- US 4399216 A
- US 4634665 A
- US 5179017 A
- WO 8704462 A
- WO 8901036 A
- EP 338841 A
- WO 2010070263 A1

### Non-patent literature cited in the description

- **HIRUMA Y.** *Biochemical and Biophysical Research Communications,* 2011, vol. 409 (3), 424-429
- **WANG, J. ; SUN, J. ; LIU, L.N. et al.** Siglec-15 as an immune suppressor and potential target for normalization cancer immunotherapy. *Nat Med,* 2019, vol. 25, 656-666
- **WARD et al.** *Nature,* 1989, vol. 341, 544-546
- **BIRD et al.** *Science,* 1988, vol. 242, 423-426
- **HUSTON et al.** *Proc. Natl. Acad. Sci.,* 1988, vol. 85, 5879-5883
- **KABAT et al.** Sequences of Proteins of Immunological Interest,. National Institutes of Health, 1991
- *J Mol Biol,* 1997, vol. 273, 927-48
- **LEFRANC M.P.** *Immunologist,* 1999, vol. 7, 132-136
- **LEFRANC, M.P. et al.** *Dev. Comp. Immunol.,* 2003, vol. 27, 55-77
- **KLIMKA et al.** *British J. of Cancer.,* 2000, vol. 83 (2), 252-260
- **BEIBOER et al.** *J. Mol. Biol.,* 2000, vol. 296, 833-849
- **RADER et al.** *Proc. Natl. Acad. Sci. U.S.A.,* 1998, vol. 95, 8910-8915
- **BARBAS et al.** *J. Am. Chem. Soc.,* 1994, vol. 116, 2161-2162
- **BARBAS et al.** *Proc. Natl. Acad. Sci. U.S.A.,* 1995, vol. 92, 2529-2533
- **DITZEL et al.** *J. Immunol.,* 1996, vol. 157, 739-749
- **BEREZOV et al.** *BLAjournal 8: Scientific Review,* 2001, vol. 8
- **IGARASHI et al.** *J. Biochem (Tokyo),* 1995, vol. 117, 452-7
- **BOURGEOIS et al.** *J. Virol.,* 1998, vol. 72, 807-10
- **LEVI et al.** *Proc. Natl. Acad. Sci. U.S.A.,* 1993, vol. 90, 4374-8
- **POLYMENIS ; STOLLER.** *J. Immunol.,* 1994, vol. 152, 5218-5329
- **XU ; DAVIS.** *Immunity,* 2000, vol. 13, 37-45
- **BRUMMELL et al.** *Biochem,* 1993, vol. 32, 1180-8
- **DE WILDT et al.** *Prot. Eng.,* 1997, vol. 10, 835-41
- **KOMISSAROV et al.** *J. Biol. Chem.,* 1997, vol. 272, 26864-26870
- **HALL et al.** *J. Immunol.,* 1992, vol. 149, 1605-12
- **KELLEY ; O'CONNELL.** *Biochem.,* 1993, vol. 32, 6862-35
- **ADIB-CONQUY et al.** *Int. Immunol.,* 1998, vol. 10, 341-6

- **BEERS et al.** *Clin. Can. Res.,* 2000, vol. 6, 2835-43
- **RIECHMANN et al.** *Nature,* 1998, vol. 332, 323-327
- **JONES et al.** *Nature,* 1986, vol. 321, 522-525
- **QUEEN et al.** *Proc. Natl. Acad. U.S.A.,* 1989, vol. 86, 10029-10033
- **TOMLINSON et al.** *J. Mol. Biol.,* 1992, vol. 227, 776-798
- **COX et al.** *Eur. J. Immunol.,* 1994, vol. 24, 827-836
- **OHNUKI et al.** *Biotechnol Bioeng.,* 2004, vol. 87, 614-22
- **SHIELDS et al.** *J. Biol. Chem.,* 2002, vol. 277, 26733-26740
- **UMANA et al.** *Nat. Biotech.,* 1999, vol. 17, 176-180
- **TARENTINO et al.** *Biochem.,* 1975, vol. 14, 5516-23
- **HUSTON et al.** *Proc Nat. Acad. Sci. USA,* 1988, vol. 85, 5879-5883
- **MCCAFFERTY et al.** *Nature,* 1990, vol. 348, 552-554
- **KOHLER ; MILSTEIN.** *Nature,* 1975, vol. 256, 495
- **MORRISON, S.** *Science,* 1985, vol. 229, 1202
- Gene Expression Technology. Methods in Enzymology. Academic Press, 1990, vol. 185
- **TAKEBE et al.** *Mol Cell. Biol.,* 1988, vol. 8, 466-472
- **URLAUB ; CHASIN.** *Proc. Natl. Acad. Sci. USA,* 1980, vol. 77, 4216-4220
- **RJ KAUFMAN ; PA SHARP.** *J. Mol. Biol.,* 1982, vol. 159, 601-621
- **CAO ; SURESH.** *Bioconjugate Chemistry,* 1998, vol. 9 (6), 635-644
- **VAN SPRIEL et al.** *Immunology Today,* 2000, vol. 21 (8), 391-397